(19) Europäisches Patentamt
European Patent Office
Office européen des brevets

(11) Veröffentlichungsnummer: **0 292 840 B1**

(12) **EUROPÄISCHE PATENTSCHRIFT**

(45) Veröffentlichungstag der Patentschrift: **09.08.95**

(21) Anmeldenummer: **88107867.9**

(22) Anmeldetag: **24.05.88**

Die Akte enthält technische Angaben, die nach dem Eingang der Anmeldung eingereicht wurden und die nicht in dieser Patentschrift enthalten sind.

(51) Int. Cl.$^6$: **C07D 401/06**, C07D 401/14, C07D 405/14, C07D 409/14, C07D 403/06, C07D 403/14, C07D 223/16, C07D 491/04, A61K 31/44, //(C07D491/04, 317:00,221:00),(C07D491/04, 317:00,209:00),(C07D491/04, 317:00,223:00)

# (54) Neue cyclische Aminderivate, diese Verbindungen enthaltende Arzneimittel und Verfahren zu ihrer Herstellung.

(30) Priorität: **25.05.87 DE 3717561**

(43) Veröffentlichungstag der Anmeldung:
**30.11.88 Patentblatt 88/48**

(45) Bekanntmachung des Hinweises auf die Patenterteilung:
**09.08.95 Patentblatt 95/32**

(84) Benannte Vertragsstaaten:
**AT BE CH DE ES FR GB GR IT LI LU NL SE**

(56) Entgegenhaltungen:
**EP-A- 0 065 229**
**EP-A- 0 161 604**
**EP-A- 0 224 794**
**GB-A- 1 548 844**

(73) Patentinhaber: **Dr. Karl Thomae GmbH**
**D-88397 Biberach (DE)**

(72) Erfinder: **Heider, Joachim, Dr. Dipl.-Chem.**
**Am Hang 3**
**D-7951 Warthausen 1 (DE)**
Erfinder: **Psiorz, Manfred, Dr. Dipl.-Chem.**
**Bergstrasse 5**
**D-7957 Schemmerhofen-Alberweiler (DE)**
Erfinder: **Bomhard, Andreas, Dr. Dipl.-Chem.**
**Dinglingerstrasse 9**
**D-7950 Biberach 1 (DE)**
Erfinder: **Hauel, Norbert, Dr. Dipl.-Chem.**
**Stresemannstrasse 40**
**D-7950 Biberach 1 (DE)**
Erfinder: **Narr, Berthold, Dr. Dipl.-Chem.**
**Obere Au 5**
**D-7950 Biberach 1 (DE)**

**EP 0 292 840 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Noll, Klaus, Dr. Dipl.-Chem.**
**Im Schönblick 3**
**D-7951 Warthausen 1 (DE)**
Erfinder: **Lillie, Christian, Dr.**
**Hansi-Niese-Weg 12**
**A-1130 Wien (AT)**
Erfinder: **Kobinger, Walter, Prof. Dr.**
**Belghofergasse 27**
**A-1121 Wien (AT)**
Erfinder: **Dämmgen, Jürgen, Dr.**
**Eichweg 7**
**D-7951 Sulmingen (DE)**

## Beschreibung

In der britischen Patentschrift 1 548 844 werden 3,4-Dihydro-2-isochinolin-1-one und 1,2,3,4-Tetrahydro-5H-2-benzazepin-1-one sowie in der EP-A-0,065,229 2H-3-Benzazepine beschrieben. So wird in Beispiel 82 der EP-A-0,065,229 die einzige Verbindung, in der E eine verzweigte Alkylengruppe darstellt, nämlich 1-[7,8-Dimethoxy-1,3,4,5-tetrahydro-2H-3-benzazepin-2-on-3-yl]-2-methyl-3-[N-methyl-N-(2-(3,4-dimethoxyphenyl)-ethyl)-amino]propan beschrieben. Die vorstehend erwähnten Verbindungen und deren physiologisch verträglichen Säureadditionssalze weisen wertvolle pharmakologische Eigenschaften auf, nämlich neben einer milden blutdrucksenkenden Wirkung insbesondere eine selektive herzfrequenzsenkende Wirkung.

Überraschenderweise wurde nun gefunden, daß die neuen cyclischen Aminderivate der allgemeinen Formel (I)

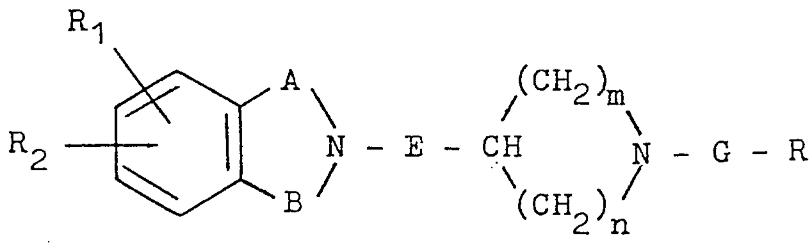 (I)

deren Enantiomeren, deren Diastereomeren, deren N-Oxide und deren Säureadditionssalze, insbesondere deren physiologisch verträgliche Säureadditionssalze mit anorganischen oder organischen Säuren, noch wertvollere pharmakologische Eigenschaften aufweisen, insbesondere eine blutdrucksenkende und eine langanhaltende herzfrequenzsenkende Wirkung und eine herabsetzende Wirkung auf den $0_2$-Bedarf des Herzens.

Gegenstand der vorliegenden Erfindung sind somit die neuen cyclischen Aminderivate der obigen allgemeinen Formel (I), deren Enantiomeren, deren Diastereomeren, deren N-Oxide, deren Säureadditionssalze, insbesondere für die pharmazeutische Verwendung deren physiologisch verträglichen Säureadditionssalze mit anorganischen oder organischen Säuren, Verfahren zu ihrer Herstellung und diese Verbindungen enthaltende Arzneimittel.

In der obigen allgemeinen Formel (I) bedeuten

A eine $-CH_2-$, $-CH_2-CH_2-$ oder $-CH=CH-$Gruppe,

B eine $-CH_2-$, $-CH_2-CH_2-$, -CO- oder $-\overset{x}{C}H_2CO-$Gruppe, wobei das mit x gekennzeichnete Kohlenstoffatom mit dem Phenylkern verknüpft ist,

E eine geradkettige Alkylengruppe mit 1 bis 3 Kohlenstoffatomen,

G eine geradkettige Alkylengruppe mit 1 bis 6 Kohlenstoffatomen, wobei eine mit einem aromatischen oder heteroaromatischen Rest R verbundene Methylengruppe einer geradkettigen Alkylengruppe mit 3 bis 6 Kohlenstoffatomen durch ein Sauerstoffatom, eine Methylimino- oder Ethyliminogruppe ersetzt sein kann,

m die Zahl 1, 2, 3, 4, 5 oder 6 und

n die Zahl 0, 1, 2 oder 3, wobei jedoch n + m die Zahl 3, 4, 5 oder 6 darstellen muß,

$R_1$ eine Methyl- oder Methoxygruppe,

$R_2$ eine Methyl- oder Methoxygruppe oder $R_1$ und $R_2$ zusammen eine Methylendioxygruppe und

R eine gegebenenfalls durch eine Methylgruppe substituierte Furyl-, Thienyl-, Pyridyl-, Benzo[b]furyl- oder Benzo[b]thienylgruppe, eine durch ein Halogenatom, eine Methoxy- oder Methansulfonyloxygruppe substituierte Benzo[b]thienylgruppe, eine gegebenenfalls durch eine Hydroxy-, Methoxy- oder Benzyloxygruppe substituierte Indolyl- oder N-Methylindolylgruppe, eine Dimethyl-thienyl- oder Dimethoxy-isochinolylgruppe, eine gegebenenfalls durch Methyl- oder Methoxygruppen mono- oder disubstituierte Naphthylgruppe, wobei die Substituenten gleich oder verschieden sein können, oder auch, wenn B eine $-CH_2-$ oder CO-Gruppe darstellt, eine gegebenenfalls durch eine Methylendioxygruppe substituierte Phenylgruppe, eine durch Chlor- oder Bromatome, Methyl- oder Methoxygruppen mono- oder disubstituierte Phenylgruppe, wobei die Substituenten gleich oder verschieden sein können, eine durch eine Hydroxy-, Benzyloxy-, Methansulfonyloxy-, Trifluormethansulfonyloxy-, Trifluormethyl-, Trifluormethoxy-, Nitro-, Amino-, Acetamido-, Methansulfonylamino- oder Bis(methansulfonyl)aminogruppe substituierte Phenylgruppe, eine Trimethoxyphenyl-, Tetramethylphenyl- oder Dihalogenaminophenylgruppe.

Beispielsweise seien folgende Verbindungen genannt, die unter den Schutzumfang der vorliegenden Erfindung fallen, aber in den Beispielen nicht explicit beschrieben werden:

2-[(N-(2-(Naphthyl-2)-ethyl)-hexahydro-azepinyl-3)-methyl]-6,7-dimethoxy-1,2,3,4-tetrahydro-isochinolin,
2-[(N-(2-(Naphthyl-2)-ethyl)-azacyclooctyl-3)-methyl]-6,7-dimethoxy-1,2,3,4-tetrahydro-isochinolin,
2-[(N-(2-(Naphthyl-1)-ethyl)-hexahydro-azepinyl-3)-methyl]-6,7-dimethoxy-1-oxo-1,2,3,4-tetrahydro-isochinolin,
2-[(N-(2-(Naphthyl-1)-ethyl)-azacyclooctyl-3)-methyl]-6,7-dimethoxy-1-oxo-1,2,3,4-tetrahydro-isochinolin,
2-[(N-(2-Methyl-naphthyl-1)-methyl)-pyrrolidyl-3)-methyl]-6,7-dimethoxy-1-oxo-1,2,3,4-tetrahydro-isochinolin,
2-[3-(N-(2-Methyl-naphthyl-1)-methyl)-piperidyl-3)-propyl]-6,7-dimethoxy-1-oxo-1,2,3,4-tetrahydro-isochinolin,
2-[(N-(2-(5-Methyl-6-methoxy-naphthyl-2)-ethyl)-pyrrolidyl-3)-methyl]-6,7-dimethoxy-1,2,3,4-tetrahydro-isochinolin,
2-[(N-(2-(5-Methyl-6-methoxy-naphthyl-2)-ethyl)-azacyclooctyl-3)-methyl]-6,7-dimethoxy-1-oxo-1,2,3,4-tetrahydro-isochinolin,
2-[(N-(2-(6-Methoxy-naphthyl-2)-ethyl)-hexahydro-azepinyl-3)-methyl]-6,7-dimethoxy-1-oxo-1,2,3,4-tetrahydro-isochinolin,
2-[(N-(2-(6-Methoxy-naphthyl-2)-ethyl)-azacyclooctyl-3)-methyl]-6,7-dimethoxy-1-oxo-1,2,3,4-tetrahydro-isochinolin,
2-[3-(N-(2-(6-Methoxy-naphthyl-2)-ethyl)-pyridyl-3)-propyl]-6,7-dimethoxy-1-oxo-1,2,3,4-tetrahydro-isochinolin,
2-[(N-(4-(Naphthyl-2-oxy)-butyl)-azacyclooctyl-3)-methyl]-6,7-dimethoxy-1,2,3,4-tetrahydro-isochinolin,
2-[2-(N-(4-(Naphthyl-2-oxy)-butyl)-piperidyl-2)-ethyl]-6,7-dimethoxy-1-oxo-1,2,3,4-tetrahydro-isochinolin,
2-[(N-(3-(Naphthyl-2-oxy)-propyl)-azacyclooctyl-3)-methyl]-6,7-dimethoxy-1-oxo-1,2,3,4-tetrahydro-isochinolin,
2-[(N-(3-(Naphthyl-2)-propyl)-piperidyl-3)-methyl]-6,7-dimethoxy-1,2,3,4-tetrahydro-isochinolin,
2-[(N-(3-(Naphthyl-2)-propyl)-azacyclooctyl-3)-methyl]-6,7-dimethoxy-1-oxo-1,2,3,4-tetrahydro-isochinolin,
2-[(N-(2-(Naphthyl-2)-ethyl)-piperidyl-3)-methyl]-6,7-methylendioxy-1,2,3,4-tetrahydro-isochinolin,
2-[(N-(2-(Naphthyl-2)-ethyl)-azacyclooctyl-3)-methyl]-6,7-methylendioxy-1,2,3,4-tetrahydro-isochinolin,
2-[(N-(2-Methyl-naphthyl-1)-methyl)-pyrrolidyl-3)-methyl]-6,7-methylendioxy-1-oxo-1,2,3,4-tetrahydro-isochinolin,
2-[(N-(2-Methyl-naphthyl-1)-methyl)-piperidyl-3)-methyl]-6,7-methylendioxy-1,2,3,4-tetrahydro-isochinolin,
2-[(N-(2-Methyl-naphthyl-1)-methyl)-azacyclooctyl-3)-methyl]-6,7-methylendioxy-1-oxo-1,2,3,4-tetrahydro-isochinolin,
2-[(N-(2-(5-Methyl-6-methoxy-naphthyl-2)-ethyl)-azacyclooctyl-3)-methyl]-6,7-methylendioxy-1-oxo-1,2,3,4-tetrahydro-isochinolin,
2-[2-(N-(2-(5-Methyl-6-methoxy-naphthyl-2)-ethyl)-piperidyl-3)-methyl]-6,7-methylendioxy-1-oxo-1,2,3,4-tetrahydro-isochinolin,
2-[(N-(2-(6-Methoxy-naphthyl-2)-ethyl)-piperidyl-3)-methyl]-6,7-methylendioxy-1-oxo-1,2,3,4-tetrahydro-isochinolin,
2-[(N-(4-(Naphthyl-2-oxy)-butyl)-pyrrolidyl-3)-methyl]-6,7-methylendioxy-1-oxo-1,2,3,4-tetrahydro-isochinolin,
2-[(N-(4-(Naphthyl-2-oxy)-butyl)-piperidyl-3)-methyl]-6,7-methylendioxy-1-oxo-1,2,3,4-tetrahydro-isochinolin,
2-[(N-(4-(Naphthyl-2-oxy)-butyl)-hexahydro-azepinyl-3)-methyl]-6,7-methylendioxy-1,2,3,4-tetrahydro-isochinolin,
2-[(N-(2-(Naphthyl-2)-ethyl)-pyrrolidyl-3)-methyl]-6,7-dimethyl-1-oxo-1,2,3,4-tetrahydro-isochinolin,
2-[(N-(2-(Naphthyl-2)-ethyl)-piperidyl-3)-methyl]-6,7-dimethyl-1,2,3,4-tetrahydro-isochinolin,
2-[(N-(2-(Naphthyl-2)-ethyl)-azacyclooctyl-3)-methyl]-6,7-dimethyl-1-oxo-1,2,3,4-tetrahydro-isochinolin,
2-[(N-(2-(Naphthyl-2)-ethyl)-azacyclooctyl-3)-methyl]-6,7-dimethyl-1,2,3,4-tetrahydro-isochinolin,
2-[(N-(2-(Naphthyl-1)-ethyl)-pyrrolidyl-3)-methyl]-6,7-dimethyl-1,2,3,4-tetrahydro-isochinolin,
2-[(N-(2-(Naphthyl-1)-ethyl)-hexahydro-azepinyl-3)-methyl]-6,7-dimethyl-1,2,3,4-tetrahydro-isochinolin,
2-[3-(N-(2-(Naphthyl-1)-ethyl)-piperidyl-3)-propyl]-6,7-dimethyl-1-oxo-1,2,3,4-tetrahydro-isochinolin,
2-[(N-(2-Methyl-naphthyl-1)-methyl)-piperidyl-3)-methyl]-6,7-dimethyl-1-oxo-1,2,3,4-tetrahydro-isochinolin,
2-[(N-(2-Methyl-naphthyl-1)-methyl)-piperidyl-3)-methyl]-6,7-dimethyl-1,2,3,4-tetrahydro-isochinolin,
2-[(N-(2-Methyl-naphthyl-1)-methyl)-azacyclooctyl-3)-methyl]-6,7-dimethyl-1-oxo-1,2,3,4-tetrahydro-isochinolin,
2-[2-(N-(2-Methyl-naphthyl-1)-methyl)-piperidyl-2)-ethyl]-6,7-dimethyl-1-oxo-1,2,3,4-tetrahydro-isochinolin,
2-[(N-(2-(5-Methyl-6-methoxy-naphthyl-2)-ethyl)-piperidyl-3)-methyl]-6,7-dimethyl-1-oxo-1,2,3,4-tetrahydro-isochinolin,
2-[(N-(2-(5-Methyl-6-methoxy-naphthyl-2)-ethyl)-hexahydro-azepinyl-3)-methyl]-6,7-dimethyl-1-oxo-1,2,3,4-tetrahydro-isochinolin,
2-[(N-(2-(5-Methyl-6-methoxy-naphthyl-2)-ethyl)-azacyclooctyl-3)-methyl]-6,7-dimethyl-1-oxo-1,2,3,4-tetrahydro-isochinolin,

2-[(N-(2-(5-Methyl-6-methoxy-naphthyl-2)-ethyl)-azacyclooctyl-3)-methyl]-6,7-dimethyl-1,2,3,4-tetrahydro-isochinolin,
2-[(N-(2-(6-Methoxy-naphthyl-2)-ethyl)-pyrrolidyl-3)-methyl]-6,7-dimethyl-1,2,3,4-tetrahydro-isochinolin,
2-[(N-(2-(6-Methoxy-naphthyl-2)-ethyl)-piperidyl-3)-methyl]-6,7-dimethyl-1,2,3,4-tetrahydro-isochinolin,
2-[(N-(2-(6-Methoxy-naphthyl-2)-ethyl)-hexahydro-azepinyl-3)-methyl]-1,2,3,4-tetrahydro-isochinolin,
2-[2-(N-(2-(6-Methoxy-naphthyl-2)-ethyl)-piperidyl-2)-ethyl]-6,7-dimethyl-1-oxo-1,2,3,4-tetrahydro-isochinolin,
2-[(N-(4-(Naphthyl-2-oxy)-butyl)-piperidyl-3)-methyl]-6,7-dimethyl-1-oxo-1,2,3,4-tetrahydro-isochinolin,
2-[(N-(4-(Naphthyl-2-oxy)-butyl)-hexahydro-azepinyl-3)-methyl]-6,7-dimethyl-1-oxo-1,2,3,4-tetrahydro-isochinolin,
2-[(N-(4-(Naphthyl-2-oxy)-butyl)-azacyclooctyl-3)-methyl]-6,7-dimethyl-1,2,3,4-tetrahydro-isochinolin,
2-[(N-(3-(6-Methoxy-naphthyl-2-oxy)-propyl)-hexahydro-azepinyl-3)-methyl]-6,7-dimethyl-1-oxo-1,2,3,4-tetrahydro-isochinolin,
2-[(N-(3-(6-Methoxy-naphthyl-2-oxy)-propyl)-azacyclooctyl-3)-methyl]-6,7-dimethyl-1-oxo-1,2,3,4-tetrahydro-isochinolin,
2-[(N-(3-(5,6-Dimethoxy-naphthyl-2-oxy)-propyl)-azacyclooctyl-3)-methyl]-6,7-dimethyl-1-oxo-1,2,3,4-tetrahydro-isochinolin,
2-[(N-(3-(Naphth-2-yl)-propyl)-azacyclooctyl-3)-methyl]-6,7-dimethyl-1-oxo-1,2,3,4-tetrahydro-isochinolin,
3-[(N-(3-(Pyridyl-3)-propyl)-pyrrolidyl-3)-methyl]-7,8-methylendioxy-2-oxo-1,3,4,5-tetrahydro-2H-3-benzazepin,
3-[(N-(1-(Pyridyl-3)-methyl)-pyrrolidyl-3)-methyl]-7,8-methylendioxy-1,3,4,5-tetrahydro-2H-3-benzazepin,
3-[(N-(3-(Pyridyl-4)-propyl)-pyrrolidyl-3)-methyl]-7,8-dimethyl-1,3,4,5-tetrahydro-2H-3-benzazepin,
2-[(N-(1-(Pyridyl-3)-methyl)-pyrrolidyl-3)-methyl]-6,7-dimethyl-1-oxo-1,2,3,4-tetrahydro-isochinolin,
2-[(N-(3-(Pyridyl-3)-propyl)-pyrrolidyl-3)-methyl]-5,6-methylendioxy-1-oxo-1,3-dihydro-isoindol,
2-[(N-(3-(Pyridyl-4)-propyl)-pyrrolidyl-3)-methyl]-5,6-methylendioxy-1,3-dihydro-isoindol,
2-[(N-(2-(6-Methyl-pyridyl-2)-ethyl)-pyrrolidyl-3)-methyl]-5,6-methylendioxy-1-oxo-1,3-dihydro-isoindol,
2-[(N-(2-(6-Methyl-pyridyl-2)-ethyl)-pyrrolidyl-3)-methyl]-5,6-methylendioxy-1,3-dihydro-isoindol,
2-[(N-(1-(Pyridyl-3)-methyl)-pyrrolidyl-3)-methyl]-5,6-dimethyl-1,3-dihydro-isoindol,
3-[(N-(3-(Pyridyl-3)-propyl)-pyrrolidyl-3)-methyl]-7,8-dimethoxy-1,3,4,5-tetrahydro-2H-3-benzazepin,
2-[(N-(3-(Pyridyl-4)-propyl)-pyrrolidyl-3)-methyl]-5,6-dimethoxy-1,3-dihydro-isoindol,
2-[(N-(1-(Pyridyl-3)-methyl)-pyrrolidyl-3)-methyl]-5,6-dimethoxy-1,3-dihydro-isoindol,
2-[(N-(1-(Pyridyl-3)-methyl)-pyrrolidyl-3)-methyl]-5,6-dimethoxy-1-oxo-1,3-dihydro-isoindol,
2-[(N-(2-(6-Methyl-pyridyl-2)-ethyl)-pyrrolidyl-3)-methyl]-6,7-dimethoxy-1,2,3,4-tetrahydro-isochinolin,
2-[(N-(2-(6-Methyl-pyridyl-2)-ethyl)-pyrrolidyl-3)-methyl]-5,6-dimethoxy-1,3-dihydro-isoindol,
2-[(N-(2-(6-Methyl-pyridyl-2)-ethyl)-pyrrolidyl-3)-methyl]-6,7-dimethoxy-1-oxo-1,2,3,4-tetrahydro-isochinolin,
2-[(N-(2-(6-Methyl-pyridyl-2)-ethyl)-pyrrolidyl-3)-methyl]-5,6-dimethoxy-1-oxo-1,3-dihydro-isoindol,
2-[2-(N-(3-(Pyridyl-4)-propyl)-piperidyl-2)-ethyl]-6,7-dimethoxy-1,2,3,4-tetrahydro-isochinolin,
2-[2-(N-(3-(Pyridyl-4)-propyl)-piperidyl-2)-ethyl]-6,7-dimethoxy-1-oxo-1,2,3,4-tetrahydro-isochinolin,
3-[(N-(2-(6,7-Dimethoxy-isochinolyl-4)-ethyl)-piperidyl-2)-methyl]-7,8-dimethyl-2-oxo-1,3,4,5-tetrahydro-2H-3-benzazepin,
2-[(N-(1-(Pyridyl-4)-methyl)-piperidyl-3)-methyl]-6,7-dimethyl-1,2,3,4-tetrahydro-isochinolin,
2-[(N-(2-(6,7-Dimethoxy-isochinolyl-4)-ethyl)-piperidyl-3)-methyl]-5,6-methylendioxy-1-oxo-1,3-dihydro-isoindol,
2-[2-(N-(3-(Pyridyl-4)-propyl)-piperidyl-2)-ethyl]-5,6-methylendioxy-1,3-dihydro-isoindol,
2-[(N-(3-(Pyridyl-3)-propyl)-hexahydro-azepinyl-3)-methyl]-6,7-dimethyl-1-oxo-1,2,3,4-tetrahydro-isochinolin,
2-[(N-(3-(Pyridyl-3)-propyl)-hexahydro-azepinyl-3)-methyl]-6,7-dimethyl-1,2,3,4-tetrahydro-isochinolin,
2-[(N-(2-(6-Methyl-pyridyl-2)-ethyl)-hexahydro-azepinyl-3)-methyl]-5,6-dimethoxy-1,3-dihydro-isoindol,
2-[(N-(2-(6-Methyl-pyridyl-2)-ethyl)-hexahydro-azepinyl-3)-methyl]-5,6-dimethoxy-1-oxo-1,3-dihydro-isoindol,
3-[(N-(3-(5-Hydroxy-indolyl-3)-propyl)-pyrrolidyl-3)-methyl]-7,8-dimethyl-2-oxo-1,3,4,5-tetrahydro-2H-3-benzazepin,
2-[(N-(3-(5-Hydroxy-indolyl-3)-propyl)-pyrrolidyl-3)-methyl]-6,7-methylendioxy-1,2,3,4-tetrahydro-isochinolin,
3-[(N-(3-(5-Methoxy-indolyl-3)-propyl)-pyrrolidyl-3)-methyl]-7,8-dimethyl-1,3,4,5-tetrahydro-2H-3-benzazepin,
2-[(N-(3-(5-Methoxy-indolyl-3)-propyl)-pyrrolidyl-3)-methyl]-5,6-methylen-dioxy-1,3-dihydro-isoindol,
2-[(N-(3-(5-Benzyloxy-indolyl-3)-propyl)-pyrrolidyl-3)-methyl]-6,7-methylendioxy-1-oxo-1,2,3,4-tetrahydro-isochinolin,
2-[(N-(3-(5-Benzyloxy-indolyl-3)-propyl)-pyrrolidyl-3)-methyl]-5,6-methylendioxy-1-oxo-1,3-dihydro-isoindol,
3-[(N-(3-(N-Methyl-indolyl-3)-propyl)-pyrrolidyl-3)-methyl]-7,8-methylendioxy-1,3,4,5-tetrahydro-2H-3-benzazepin,
2-[(N-(3-(N-Methyl-indolyl-3)-propyl)-pyrrolidyl-3)-methyl]-6,7-dimethyl-1,2,3,4-tetrahydro-isochinolin,

2-[(N-(3-(Indolyl-3)-propyl)-pyrrolidyl-3)-methyl]-5,6-dimethyl-1,3-dihydro-isoindol,
3-[(N-(3-(Indolyl-3)-propyl)-pyrrolidyl-3)-methyl]-7,8-dimethoxy-2-oxo-1,3,4,5-tetrahydro-2H-3-benzazepin,
2-[(N-(3-(5-Hydroxy-indolyl-3)-propyl)-pyrrolidyl-3)-methyl]-5,6-dimethyl-1-oxo-1,3-dihydro-isoindol,
2-[(N-(3-(5-Hydroxy-indolyl-3)-propyl)-pyrrolidyl-3)-methyl]-6,7-dimethoxy-1-oxo-1,2,3,4-tetrahydro-isochinolin,
3-[(N-(3-(5-Methoxy-indolyl-3)-propyl)-pyrrolidyl-3)-methyl]-7,8-dimethoxy-1,3,4,5-tetrahydro-2H-3-benzazepin,
2-[(N-(3-(5-Methoxy-indolyl-3)-propyl)-pyrrolidyl-3)-methyl]-5,6-dimethoxy-1-oxo-1,3-dihydro-isoindol,
2-[(N-(3-(5-Benzyloxy-indolyl-3)-propyl)-pyrrolidyl-3)-methyl]-6,7-dimethoxy-1,2,3,4-tetrahydro-isochinolin,
2-[(N-(3-(5-Benzyloxy-indolyl-3)-propyl)-pyrrolidyl-3)-methyl]-6,7-dimethoxy-1-oxo-1,2,3,4-tetrahydro-isochinolin,
2-[(N-(3-(N-Methyl-indolyl-3)-propyl)-pyrrolidyl-3)-methyl]-5,6-dimethyl-1-oxo-1,3-dihydro-isoindol,
2-[(N-(3-(N-Methyl-indolyl-3)-propyl)-pyrrolidyl-3)-methyl]-5,6-dimethoxy-1,3-dihydro-isoindol,
2-[(N-(2-(3,4-Dimethoxy-phenyl)-ethyl)-hexahydro-azepinyl-3)-methyl]-6,7-methylendioxy-1-oxo-1,2,3,4-tetrahydro-isochinolin,
2-[(N-(3-(3,4-Methylendioxy-phenoxy)-propyl)-hexahydro-azepinyl-3)-methyl]-6,7-methylendioxy-1-oxo-1,2,3,4-tetrahydro-isochinolin,
2-[(N-(2-(4-Trifluormethoxy-phenyl)-ethyl)-piperidyl-3)-methyl]-5,6-dimethoxy-1-oxo-1,3-dihydro-isoindol,
2-[(N-(2-(4-Trifluormethyl-phenyl)-ethyl)-piperidyl-3)-methyl]-5,6-dimethoxy-1-oxo-1,3-dihydro-isoindol,
2-[(N-(2-(3,4-Dichlor-phenyl)-ethyl)-piperidyl-3)-methyl]-5,6-dimethoxy-1-oxo-1,3-dihydro-isoindol,
2-[(N-(2-(3,4,5-Trimethoxy-phenyl)-ethyl)-piperidyl-3)-methyl]-5,6-dimethoxy-1-oxo-1,3-dihydro-isoindol,
2-[(N-(2-(4-Methoxy-phenyl)-ethyl)-piperidyl-3)-methyl]-5,6-dimethoxy-1-oxo-1,3-dihydro-isoindol,
2-[(N-(2-(3-Methyl-phenyl)-ethyl)-piperidyl-3)-methyl]-5,6-dimethoxy-1-oxo-1,3-dihydro-isoindol,
2-[(N-(2-(3,4-Dimethyl-phenyl)-ethyl)-piperidyl-3)-methyl]-5,6-dimethoxy-1-oxo-1,3-dihydro-isoindol,
2-[(N-(2-(2,3,4,5-Tetramethyl-phenyl)-ethyl)-piperidyl-3)-methyl]-5,6-dimethoxy-1-oxo-1,3-dihydro-isoindol,
2-[(N-(2-(4-Benzyloxy-phenyl)-ethyl)-piperidyl-3)-methyl]-5,6-dimethoxy-1-oxo-1,3-dihydro-isoindol,
2-[(N-(2-(4-Hydroxy-phenyl)-ethyl)-piperidyl-3)-methyl]-5,6-dimethoxy-1-oxo-1,3-dihydro-isoindol,
2-[(N-(2-(4-Methansulfonyloxy-phenyl)-ethyl)-piperidyl-3)-methyl]-5,6-dimethoxy-1-oxo-1,3-dihydro-isoindol,
2-[(N-(2-(4-Trifluormethansulfonyloxy-phenyl)-ethyl)-piperidyl-3)-methyl]-5,6-dimethoxy-1-oxo-1,3-dihydro-isoindol,
2-[(N-(2-(4-Methansulfonylamino-phenyl)-ethyl)-piperidyl-3)-methyl]-5,6-dimethoxy-1-oxo-1,3-dihydro-isoindol,
2-[(N-(2-(4-Dimethansulfonylamino-phenyl)-ethyl)-piperidyl-3)-methyl]-5,6-dimethoxy-1-oxo-1,3-dihydro-isoindol,
2-[(N-(3-(4-Brom-phenyl)-propyl)-piperidyl-3)-methyl]-5,6-dimethoxy-1-oxo-1,3-dihydro-isoindol,
2-[(N-(3-(4-Methoxy-phenyl)-propyl)-piperidyl-3)-methyl]-5,6-dimethoxy-1-oxo-1,3-dihydro-isoindol,
2-[(N-(3-(3-Methoxy-phenyl)-propyl)-piperidyl-3)-methyl]-5,6-dimethoxy-1-oxo-1,3-dihydro-isoindol,
2-[(N-(3-(3,4-Dimethoxy-phenyl)-propyl)-piperidyl-3)-methyl]-5,6-dimethoxy-1-oxo-1,3-dihydro-isoindol,
2-[(N-(4-(4-Methoxy-phenyl)-butyl)-piperidyl-3)-methyl]-5,6-dimethoxy-1-oxo-1,3-dihydro-isoindol,
2-[(N-(3-(4-Amino-3,5-dibrom-phenoxy)-propyl)-piperidyl-3)-methyl]-5,6-dimethoxy-1-oxo-1,3-dihydro-isoindol,
3-[(N-(2-(2,5-Dimethyl-thienyl-3)-ethyl)-piperidyl-3)-methyl]-7,8-dimethoxy-2-oxo-1,3,4,5-tetrahydro-2H-3-benzazepin,
3-[(N-(2-(5-Methoxy-benzo[b]thienyl-3)-ethyl)-piperidyl-3)-methyl]-7,8-dimethoxy-2-oxo-1,3,4,5-tetrahydro-2H-3-benzazepin,
3-[(N-(2-(6-Methoxy-benzo[b]thienyl-3)-ethyl)-piperidyl-3)-methyl]-7,8-dimethoxy-2-oxo-1,3,4,5-tetrahydro-2H-3-benzazepin,
3-[(N-(2-(6-Brom-benzo[b]thienyl-3)-ethyl)-piperidyl-3)-methyl]-7,8-dimethoxy-2-oxo-1,3,4,5-tetrahydro-2H-3-benzazepin,
3-[(N-(2-(Benzo[b]thienyl-2)-ethyl)-piperidyl-3)-methyl]-7,8-dimethoxy-2-oxo-1,3,4,5-tetrahydro-2H-3-benzazepin,
3-[(N-(2-(Benzo[b]furyl-3)-ethyl)-piperidyl-3)-methyl]-7,8-dimethoxy-2-oxo-1,3,4,5-tetrahydro-2H-3-benzazepin,
3-[(N-(2-(2,5-Dimethyl-thienyl-3)-ethyl)-piperidyl-3)-methyl]-7,8-dimethyl-2-oxo-1,3,4,5-tetrahydro-2H-3-benzazepin,
3-[(N-(2-(5-Methoxy-benzo[b]thienyl-3)-ethyl)-piperidyl-3)-methyl]-7,8-dimethyl-2-oxo-1,3,4,5-tetrahydro-2H-3-benzazepin,
3-[(N-(2-(6-Methoxy-benzo[b]thienyl-3)-ethyl)-piperidyl-3)-methyl]-7,8-dimethyl-2-oxo-1,3,4,5-tetrahydro-2H-3-benzazepin,
3-[(N-(2-(6-Brom-benzo[b]thienyl-3)-ethyl)-piperidyl-3)-methyl]-7,8-dimethyl-2-oxo-1,3,4,5-tetrahydro-2H-3-benzazepin,
3-[(N-(2-(Benzo[b]thienyl-2)-ethyl)-piperidyl-3)-methyl]-7,8-dimethyl-2-oxo-1,3,4,5-tetrahydro-2H-3-benzazepin,
3-[(N-(2-(Benzo[b]furyl-3)-ethyl)-piperidyl-3)-methyl]-7,8-dimethyl-2-oxo-1,3,4,5-tetrahydro-2H-3-benzazepin,
3-[(N-(2-(2,5-Dimethyl-thienyl-3)-ethyl)-piperidyl-3)-methyl]-7,8-methylendioxy-2-oxo-1,3,4,5-tetrahydro-2H-3-benzazepin,
3-[(N-(2-(5-Methoxy-benzo[b]thienyl-3)-ethyl)-piperidyl-3)-methyl]-7,8-methylendioxy-2-oxo-1,3,4,5-tetrahydro-2H-3-benzazepin,
3-[(N-(2-(6-Methoxy-benzo[b]thienyl-3)-ethyl)-piperidyl-3)-methyl]-7,8-methylendioxy-2-oxo-1,3,4,5-tetrahydro-2H-3-benzazepin,
3-[(N-(2-(6-Brom-benzo[b]thienyl-3)-ethyl)-piperidyl-3)-methyl]-7,8-methylendioxy-2-oxo-1,3,4,5-tetrahydro-2H-3-benzazepin,
3-[(N-(2-(Benzo[b]thienyl-2)-ethyl)-piperidyl-3)-methyl]-7,8-methylendioxy-2-oxo-1,3,4,5-tetrahydro-2H-3-benzazepin,
3-[(N-(2-(Benzo[b]furyl-3)-ethyl)-piperidyl-3)-methyl]-7,8-methylendioxy-2-oxo-1,3,4,5-tetrahydro-2H-3-benzazepin,
3-[(N-(3-(2,5-Dimethyl-thienyl-3)-propyl)-piperidyl-3)-methyl]-7,8-dimethoxy-2-oxo-1,3,4,5-tetrahydro-2H-3-benzazepin,
3-[(N-(3-(5-Methoxy-benzo[b]thienyl-3)-propyl)-piperidyl-3)-methyl]-7,8-dimethoxy-2-oxo-1,3,4,5-tetrahydro-2H-3-benzazepin,
3-[(N-(3-(6-Methoxy-benzo[b]thienyl-3)-propyl)-piperidyl-3)-methyl]-7,8-dimethoxy-2-oxo-1,3,4,5-tetrahydro-2H-3-benzazepin,
3-[(N-(3-(6-Brom-benzo[b]thienyl-3)-propyl)-piperidyl-3)-methyl]-7,8-dimethoxy-2-oxo-1,3,4,5-tetrahydro-2H-3-benzazepin,
3-[(N-(3-(Benzo[b]thienyl-2)-propyl)-piperidyl-3)-methyl]-7,8-dimethoxy-2-oxo-1,3,4,5-tetrahydro-2H-3-benzazepin,
3-[(N-(3-(Benzo[b]furyl-3)-propyl)-piperidyl-3)-methyl]-7,8-dimethoxy-2-oxo-1,3,4,5-tetrahydro-2H-3-benzazepin,
3-[(N-(3-(2,5-Dimethyl-thienyl-3)-propyl)-piperidyl-3)-methyl]-7,8-dimethyl-2-oxo-1,3,4,5-tetrahydro-2H-3-benzazepin,
3-[(N-(3-(6-Methoxy-benzo[b]thienyl-3)-propyl)-piperidyl-3)-methyl]-7,8-dimethyl-2-oxo-1,3,4,5-tetrahydro-2H-3-benzazepin,
3-[(N-(3-(Benzo[b]thienyl-2)-propyl)-piperidyl-3)-methyl]-7,8-dimethyl-2-oxo-1,3,4,5-tetrahydro-2H-3-benzazepin,
3-[(N-(3-(Benzo[b]furyl-3)-propyl)-piperidyl-3)-methyl]-7,8-dimethyl-2-oxo-1,3,4,5-tetrahydro-2H-3-benzazepin,
3-[(N-(3-(2,5-Dimethyl-thienyl-3)-propyl)-piperidyl-3)-methyl]-7,8-methylendioxy-2-oxo-1,3,4,5-tetrahydro-2H-3-benzazepin,
3-[(N-(3-(6-Methoxy-benzo[b]thienyl-3)-propyl)-piperidyl-3)-methyl]-7,8-methylendioxy-2-oxo-1,3,4,5-tetrahydro-2H-3-benzazepin,
3-[(N-(3-(Benzo[b]thienyl-2)-propyl)-piperidyl-3)-methyl]-7,8-methylendioxy-2-oxo-1,3,4,5-tetrahydro-2H-3-benzazepin,
3-[(N-(3-(Benzo[b]furyl-3)-propyl)-piperidyl-3)-methyl]-7,8-methylendioxy-2-oxo-1,3,4,5-tetrahydro-2H-3-benzazepin,
3-[(N-(2-(Thienyl-2)-ethyl)-piperidyl-3)-methyl]-7,8-dimethyl2-oxo-1,3,4,5-tetrahydro-2H-3-benzazepin,
3-[(N-(2-(Benzo[b]thienyl-3)-ethyl)-piperidyl-3)-methyl]-7,8-dimethyl-2-oxo-1,3,4,5-tetrahydro-2H-3-benzazepin,
3-[(N-(2-(Benzo[b]furyl-2)-ethyl)-piperidyl-3)-methyl]-7,8-dimethyl-2-oxo-1,3,4,5-tetrahydro-2H-3-benzazepin,
3-[(N-(2-(Thienyl-2)-ethyl)-piperidyl-3)-methyl]-7,8-methylendioxy-2-oxo-1,3,4,5-tetrahydro-2H-3-benzazepin,
3-[(N-(2-(Benzo[b]thienyl-3)-ethyl)-piperidyl-3)-methyl]-7,8-methylendioxy-2-oxo-1,3,4,5-tetrahydro-2H-3-benzazepin,
3-[(N-(2-(Benzo[b]furyl-2)-ethyl)-piperidyl-3)-methyl]-7,8-methylendioxy-2-oxo-1,3,4,5-tetrahydro-2H-3-benzazepin,
3-[(N-(2-(Thienyl-2)-ethyl)-hexahydro-azepinyl-3)-methyl]-7,8-dimethyl-2-oxo-1,3,4,5-tetrahydro-2H-3-benzazepin,
3-[(N-(2-(Benzo[b]thienyl-3)-ethyl)-hexahydro-azepinyl-3)-methyl]-7,8-dimethyl-2-oxo-1,3,4,5-tetrahydro-2H-3-

benzazepin,
3-[(N-(2-(Benzo[b]furyl-2)-ethyl)-hexahydro-azepinyl-3)-methyl]-7,8-dimethyl-2-oxo-1,3,4,5-tetrahydro-2H-3-benzazepin,
3-[(N-(2-(Thienyl-2)-ethyl)-hexahydro-azepinyl-3)-methyl]-7,8-methylendioxy-2-oxo-1,3,4,5-tetrahydro-2H-3-benzazepin,
3-[(N-(2-(Benzo[b]thienyl-3)-ethyl)-hexahydro-azepinyl-3)-methyl]-7,8-methylendioxy-2-oxo-1,3,4,5-tetrahydro-2H-3-benzazepin,
3-[(N-(2-(Benzo[b]furyl-2)-ethyl)-hexahydro-azepinyl-3)-methyl]-7,8-methylendioxy-2-oxo-1,3,4,5-tetrahydro-2H-3-benzazepin,
3-[(N-(2-(Thienyl-2)-ethyl)-pyrrolidyl-3)-methyl]-7,8-dimethyl-2-oxo-1,3,4,5-tetrahydro-2H-3-benzazepin,
3-[(N-(2-(Benzo[b]thienyl-3)-ethyl)-pyrrolidyl-3)-methyl]-7,8-dimethyl-2-oxo-1,3,4,5-tetrahydro-2H-3-benzazepin,
3-[(N-(2-(Benzo[b]furyl-2)-ethyl)-pyrrolidyl-3)-methyl]-7,8-dimethyl-2-oxo-1,3,4,5-tetrahydro-2H-3-benzazepin,
3-[(N-(2-(Thienyl-2)-ethyl)-pyrrolidyl-3)-methyl]-7,8-methylendioxy-2-oxo-1,3,4,5-tetrahydro-2H-3-benzazepin,
3-[(N-(2-(Benzo[b]thienyl-3)-ethyl)-pyrrolidyl-3)-methyl]-7,8-methylendioxy-2-oxo-1,3,4,5-tetrahydro-2H-3-benzazepin,
3-[(N-(2-(Benzo[b]furyl-2)-ethyl)-pyrrolidyl-3)-methyl]-7,8-methylendioxy-2-oxo-1,3,4,5-tetrahydro-2H-3-benzazepin,
3-[(N-(2-(Thienyl-2)-ethyl)-pyrrolidyl-3)-methyl]-7,8-dimethoxy-2-oxo-1,3,4,5-tetrahydro-2H-3-benzazepin,
3-[(N-(2-(Benzo[b]thienyl-3)-ethyl)-pyrrolidyl-3)-methyl]-7,8-dimethoxy-2-oxo-1,3,4,5-tetrahydro-2H-3-benzazepin,
3-[(N-(2-(Benzo[b]furyl-2)-ethyl)-pyrrolidyl-3)-methyl]-7,8-dimethoxy-2-oxo-1,3,4,5-tetrahydro-2H-3-benzazepin,
3-[(N-(2-(Thienyl-2)-ethyl)-hexahydro-azepinyl-3)-methyl]-7,8-dimethoxy-2-oxo-1,3,4,5-tetrahydro-2H-3-benzazepin,
3-[(N-(2-(Benzo[b]thienyl-3)-ethyl)-hexahydro-azepinyl-3)-methyl]-7,8-dimethoxy-2-oxo-1,3,4,5-tetrahydro-2H-3-benzazepin,
3-[(N-(2-(Benzo[b]furyl-2)-ethyl)-hexahydro-azepinyl-3)-methyl]-7,8-dimethoxy-2-oxo-1,3,4,5-tetrahydro-2H-3-benzazepin,
3-[(N-(4-(Thienyl-2)-butyl)-piperidyl-3)-methyl]-7,8-dimethyl2-oxo-1,3,4,5-tetrahydro-2H-3-benzazepin,
3-[(N-(4-(Benzo[b]thienyl-3)-butyl)-piperidyl-3)-methyl]-7,8-dimethyl-2-oxo-1,3,4,5-tetrahydro-2H-3-benzazepin,
3-[(N-(4-(Benzo[b]furyl-2)-butyl)-piperidyl-3)-methyl]-7,8-dimethyl-2-oxo-1,3,4,5-tetrahydro-2H-3-benzazepin,
3-[(N-(5-(Thienyl-2)-pentyl)-piperidyl-3)-methyl]-7,8-dimethyl-2-oxo-1,3,4,5-tetrahydro-2H-3-benzazepin,
3-[(N-(5-(Benzo[b]thienyl-3)-pentyl)-piperidyl-3)-methyl]-7,8-dimethyl-2-oxo-1,3,4,5-tetrahydro-2H-3-benzazepin,
3-[(N-(5-(Benzo[b]furyl-2)-pentyl)-piperidyl-3)-methyl]-7,8-dimethyl-2-oxo-1,3,4,5-tetrahydro-2H-3-benzazepin,
3-[(N-(4-(Thienyl-2)-butyl)-piperidyl-3)-methyl]-7,8-methylendioxy-2-oxo-1,3,4,5-tetrahydro-2H-3-benzazepin,
3-[(N-(4-(Benzo[b]thienyl-3)-butyl)-piperidyl-3)-methyl]-7,8-methylendioxy-2-oxo-1,3,4,5-tetrahydro-2H-3-benzazepin,
3-[(N-(4-(Benzo[b]furyl-2)-butyl)-piperidyl-3)-methyl]-7,8-methylendioxy-2-oxo-1,3,4,5-tetrahydro-2H-3-benzazepin,
3-[(N-(5-(Thienyl-2)-pentyl)-piperidyl-3)-methyl]-7,8-methylendioxy-2-oxo-1,3,4,5-tetrahydro-2H-3-benzazepin,
3-[(N-(5-(Benzo[b]thienyl-3)-pentyl)-piperidyl-3)-methyl]-7,8-methylendioxy-2-oxo-1,3,4,5-tetrahydro-2H-3-benzazepin,
3-[(N-(5-(Benzo[b]furyl-2)-pentyl)-piperidyl-3)-methyl]-7,8-methylendioxy-2-oxo-1,3,4,5-tetrahydro-2H-3-benzazepin,
3-[(N-(4-(Benzo[b]thienyl-3)-butyl)-piperidyl-3)-methyl]-7,8-dimethoxy-2-oxo-1,3,4,5-tetrahydro-2H-3-benzazepin,
3-[(N-(4-(Benzo[b]furyl-2)-butyl)-piperidyl-3)-methyl]-7,8-dimethoxy-2-oxo-1,3,4,5-tetrahydro-2H-3-benzazepin,
3-[(N-(5-(Benzo[b]thienyl-3)-pentyl)-piperidyl-3)-methyl]-7,8-dimethoxy-2-oxo-1,3,4,5-tetrahydro-2H-3-benzazepin und
3-[(N-(5-(Benzo[b]furyl-2)-pentyl)-piperidyl-3)-methyl]-7,8-dimethoxy-2-oxo-1,3,4,5-tetrahydro-2H-3-benzazepin sowie
deren Enantiomeren, deren Diastereomeren, deren N-Oxide und deren Säureadditionssalzen, insbesondere

für die pharmazeutische Anwendung deren physiologisch verträglichen Säureadditionssalze.

Bevorzugte Verbindungen der obigen allgemeinen Formel (I) sind jedoch diejenigen der allgemeinen Formel (Ia)

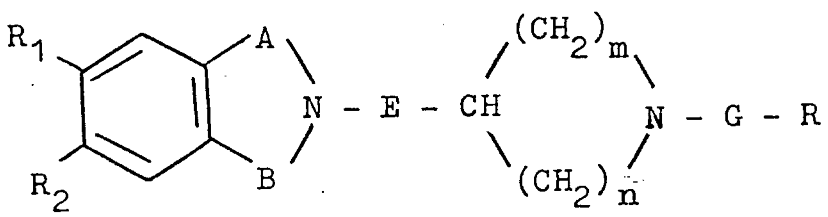

(Ia)

in der

R, $R_1$, $R_2$, A, B, E, G, m und n wie eingangs erwähnt definiert sind,

deren Enantiomeren, deren Diastereomeren, deren N-Oxide und deren Säureadditionssalze.

Besonders bevorzugte Verbindungen der obigen allgemeinen Formel (I) sind jedoch diejenigen, in denen

A eine $-CH_2CH_2$-Gruppe,

B eine $-CH_2-CH_2-$, -CO- oder $-\overset{x}{C}H_2CO$-Gruppe, wobei das mit x gekennzeichnete Kohlenstoffatom mit dem Phenylkern verknüpft ist,

E eine Methylen- oder Ethylengruppe,

G eine geradkettige Alkylengruppe mit 2 bis 4 Kohlenstoffatomen, wobei die mit einem aromatischen oder heteroaromatischen Rest R verbundene Methylengruppe einer geradkettigen Alkylengruppe mit 3 oder 4 Kohlenstoffatomen durch ein Sauerstoff ersetzt sein kann,

$R_1$ eine Methoxygruppe,

$R_2$ eine Methoxygruppe oder $R_1$ und $R_2$ zusammen eine Methylendioxygruppe,

m die Zahl 2, 3 oder 4,

n die Zahl 1 und

R eine Naphthyl-2-, 6-Methoxy-naphthyl-2-, 5-Methyl-6-methoxy-naphthyl-2-, Thienyl-2-, 6-Methyl-pyridyl-2-, Benzo[b]furyl-2- oder Benzo[b]thienyl-3-gruppe oder auch, wenn B die -CO-Gruppe darstellt, eine 4-Methoxyphenyl- oder 3,4-Dimethoxyphenylgruppe bedeuten,

deren Enantiomeren, deren Diastereomeren und deren Säureadditionssalze.

Als besonders bevorzugte Verbindungen seien folgende genannt:

a) 3-[(N-(2-(Naphthyl-2)-ethyl)-piperidyl-3)-methyl]-7,8-dimethoxy-2-oxo-1,3,4,5-tetrahydro-2H-3-benzazepin,

b) 3-[(N-(2-(5-Methyl-6-methoxy-naphthyl-2)-ethyl)-piperidyl-3)-methyl]-7,8-dimethoxy-2-oxo-1,3,4,5-tetrahydro-2H-3-benzazepin,

c) 3-[2-(N-(2-(6-Methoxy-naphthyl-2)-ethyl)-piperidyl-2)-ethyl]-7,8-dimethoxy-2-oxo-1,3,4,5-tetrahydro-2H-3-benzazepin,

d) 2-[(N-(2-(6-Methoxy-naphthyl-2)-ethyl)-hexahydro-azepinyl-3)-methyl]-6,7-methylendioxy-1-oxo-1,2,3,4-tetrahydro-isochinolin,

e) 2-[(N-(2-(Naphthyl-2)-ethyl)-hexahydro-azepinyl-3)-methyl]-6,7-methylendioxy-1-oxo-1,2,3,4-tetrahydro-isochinolin,

f) 2-[(N-(2-(3,4-Dimethoxy-phenyl)-ethyl)-piperidyl-3)-methyl]-6,7-dimethyl-1,2,3,4-tetrahydro-isochinolin,

g) 2-[(N-(2-(3,4-Dimethoxy-phenyl)-ethyl)-piperidyl-3)-methyl]-6,7-dimethyl-1-oxo-1,2,3,4-tetrahydro-isochinolin,

h) 2-[(N-(3-(4-Methoxy-phenoxy)-propyl)-piperidyl-3)-methyl]-6,7-dimethyl-1-oxo-1,2,3,4-tetrahydro-isochinolin,

i) 3-[(N-(4-(Thienyl-2)-butyl)-piperidyl-3)-methyl]-7,8-dimethoxy-2-oxo-1,3,4,5-tetrahydro-2H-3-benzazepin,

k) 3-[(N-(2-(Benzo[b]furyl-2)-ethyl)-piperidyl-3)-methyl]-7,8-dimethoxy-2-oxo-1,3,4,5-tetrahydro-2H-3-benzazepin,

l) 3-[(N-(2-(Benzo[b]thienyl-3)-ethyl)-piperidyl-3)-methyl]-7,8-dimethoxy-2-oxo-1,3,4,5-tetrahydro-2H-3-benzazepin,

m) 2-[(N-(3-(6-Methoxy-naphthyl-2-oxy)-propyl)-pyrrolidyl-3)-methyl]-6,7-dimethoxy-1-oxo-1,2,3,4-tetrahydro-isochinolin und

n) 2-[(N-(2-(6-Methoxy-naphthyl-2)-ethyl)-hexahydro-azepinyl-3)-methyl]-6,7-methylendioxy-1-oxo-1,2,3,4-tetrahydro-isochinolin,

insbesondere jedoch folgende Verbindungen:

a) 3-[(N-(2-(Naphthyl-2)-ethyl)-piperidyl-3)-methyl]-7,8-dimethoxy-2-oxo-1,3,4,5-tetrahydro-2H-3-benzazepin,

b) 2-[(N-(3-(4-Methoxy-phenoxy)-propyl)-piperidyl-3)-methyl]-6,7-dimethyl-1-oxo-1,2,3,4-tetrahydro-isochinolin,

c) 3-[(N-(4-(Thienyl-2)-butyl)-piperidyl-3)-methyl]-7,8-dimethoxy-2-oxo-1,3,4,5-tetrahydro-2H-3-benzazepin,

d) 2-[(N-(3-(6-Methoxy-naphthyl-2-oxy)-propyl)-pyrrolidyl-3)-methyl]-6,7-dimethoxy-1-oxo-1,2,3,4-tetrahydro-isochinolin,

e) 2-[(N-(2-(6-Methoxy-naphthyl-2)-ethyl)-hexahydro-azepin yl-3)-methyl]-6,7-methylendioxy-1-oxo-1,2,3,4-tetrahydro-isochinolin,

f) 3-[(N-(2-(4-Methoxy-phenyl)-ethyl)-hexahydro-azepinyl-3)-methyl]-7,8-dimethoxy-2-oxo-1,3,4,5-tetrahydro-2H-3-benzazepin und

g) 3-[(N-(2-(3,4-Dimethoxy-phenyl)-ethyl)-hexahydro-azepinyl-3)-methyl]-7,8-methylendioxy-2-oxo-1,3,4,5-tetrahydro-2H-3-benzazepin,

deren Enantiomeren, deren Diastereomeren und deren Säureadditionssalze.

Erfindungsgemäß erhält man die neuen Verbindungen der allgemeinen Formel (I) nach folgenden Verfahren:

a) Umsetzung einer Verbindung der allgemeinen Formel (II)

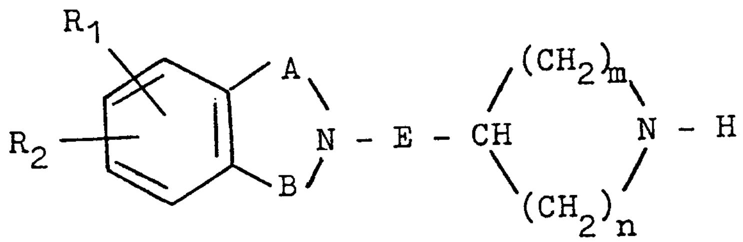

(II)

in der

$R_1$, $R_2$, A, B, E, m und n wie eingangs definiert sind, mit einer Verbindung der allgemeinen Formel (III)

$Z_1$ - G - R (III)

in der

R und G wie eingangs definiert sind und

$Z_1$ eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, oder eine Hydroxygruppe oder $Z_1$ zusammen mit einem Wasserstoffatom der benachbarten Methylengruppe ein Sauerstoffatom darstellt.

Bedeutet $Z_1$ eine nukleophile Austrittsgruppe, so wird die Umsetzung zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindungen der allgemeinen Formeln (II) und/oder (III) und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150 °C, vorzugsweise bei Temperaturen zwischen 50 und 120 °C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel (II) durchgeführt.

Bedeutet $Z_1$ eine Hydroxygruppe, so wird die Umsetzung vorzugsweise in einem geeigneten Lösungsdmittel wie Methanol, Ethanol, Tetrahydrofuran, Dioxan, Essigsäureethylester oder Eisessig mit Wasserstoff in Gegenwart eines Hydrierungskatalysators wie Platin, Palladium/Kohle oder Raney-Nickel

bei einem Wasserstoffdruck von 2 bis 10 bar, vorzugsweise bei 5 bar, und bei Temperaturen zwischen 20 und 120 °C, vorzugsweise bei Temperaturen zwischen 50 und 100 °C, durchgeführt.

Bedeutet $Z_1$ zusammen mit einem Wasserstoffatom der benachbarten Methylengruppe ein Sauerstoffatom, so wird die Umsetzung vorzugsweise in einem geeigneten Lösungsmittel wie Methanol, Ethanol, Tetrahydrofuran, Dioxan, Essigsäureethylester oder Eisessig mit Wasserstoff in Gegenwart eines Hydrierungskatalysators wie Platin, Palladium/Kohle oder Raney-Nickel bei einem Wasserstoffdruck von 2 bis 10 bar, vorzugsweise bei 5 bar, und bei Temperaturen zwischen 20 und 120 °C, vorzugsweise bei Temperaturen zwischen 50 und 100 °C, oder in Gegenwart eines geeigneten komplexen Metallhydrids wie Natriumcyanborhydrid in einem geeigneten Lösungsmittel wie Methanol, Ethanol, Tetrahydrofuran, Dioxan oder Acetonitril bei Temperaturen zwischen 0 und 50 °C, vorzugsweise jedoch bei Raumtemperatur, durchgeführt.

b) Umsetzung einer Verbindung der allgemeinen Formel (IV)

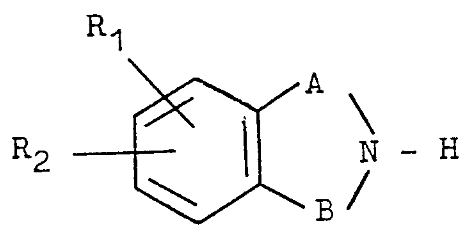

(IV)

in der

$R_1$, $R_2$, A und B wie eingangs definiert sind, mit einer Verbindung der allgemeinen Formel (V)

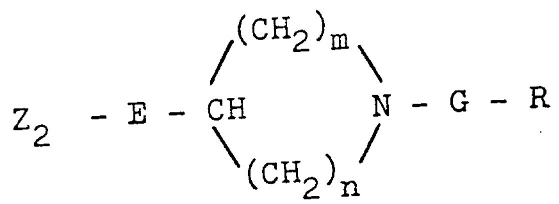

(V)

in der

R, E, G, m und n wie eingangs definiert sind und

$Z_2$ eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, darstellt.

Die Umsetzung wird zweckmäßigerweise in einem Lösungsmittel oder Lösungsmittelgemisch wie Methylformamid, Dimethylformamid, Dimethylsulfoxid, Benzol, Chlorbenzol, Tetrahydrofuran, Benzol/Tetrahydrofuran oder Dioxan in Gegenwart eines säurebindenden Mittels, z.B. eines Alkoholats wie Kaliumtert.butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid zweckmäßigerweise bei Temperaturen zwischen 0 und 150 °C, vorzugsweise bei Temperaturen zwischen 0 und 50 °C, durchgeführt.

c) Zur Herstellung von Verbindungen der allgemeinen Formel (I), in der B eine $-CH_2-$ oder $-CH_2CH_2-$ Gruppe darstellt:

Reduktion einer Verbindung der allgemeinen Formel (VI)

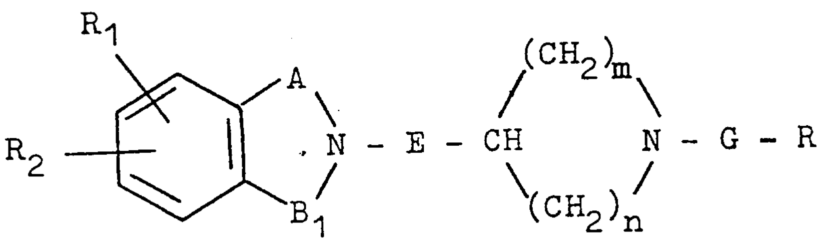

(VI)

in der
R, $R_1$, $R_2$, A, E, G, m und n wie eingangs definiert sind und
$B_1$ eine -CO- oder -$\overset{x}{C}H_2$CO-Gruppe darstellt, wobei das mit x gekennzeichnete Kohlenstoffatom mit dem Phenylkern verknüpft ist.

Die Reduktion wird vorzugsweise mit einem Metallhydrid wie Lithiumaluminiumhydrid oder Diboran oder mit einem Komplex aus Boran und einem Thioäther, z.B. mit Boran-Dimethylsulfid-Komplex, in einem geeigneten Lösungsmittel wie Diäthyläther oder Tetrahydrofuran bei Temperaturen zwischen 0 und 80 °C, vorzugsweise jedoch bei Temperaturen zwischen 10 und 45 °C, durchgeführt.

d) Zur Herstellung von Verbindungen der allgemeinen Formel (I), in der A eine -$CH_2$-Gruppe und B eine -CO- oder -$CH_2$CO-Gruppe darstellen:
Reduktion einer Verbindung der allgemeinen Formel (VII)

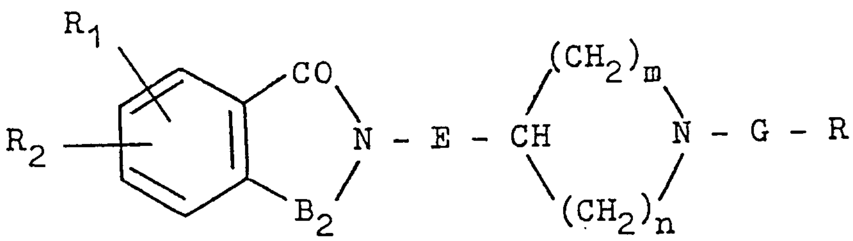

(VII)

in der
R, $R_1$, $R_2$, E, G, m und n wie eingangs definiert sind und $B_2$ eine -CO- oder -$\overset{x}{C}H_2$CO-Gruppe darstellt, wobei das mit
x gekennzeichnete Kohlenstoffatom mit dem Phenylkern verknüpft ist, mit nascierendem Wasserstoff.

Die Reduktion wird in einem geeigneten Lösungsmittel wie Eisessig, Eisessig/Wasser oder Eisessig/Ethanol mit nascierendem Wasserstoff, z.B. in Gegenwart von Zink/Eisessig, Zinn/Salzsäure oder Zinn-dichlorid/Salzsäure bei Temperaturen zwischen 20 und 150 °C, vorzugsweise jedoch bei der Siedetemperatur des Reaktionsgemisches, z.B. bei Temperaturen zwischen 80 und 100 °C, durchgeführt.

e) Zur Herstellung von Verbindungen der allgemeinen Formel I, in der G mit Ausnahme der ein Schwefelatom, eine Sulfinyl- oder Sulfonylgruppe enthaltenden Reste die für G eingangs erwähnten Bedeutungen besitzt und A die -$CH_2$-$CH_2$-Gruppe und B die Methylen- oder Carbonylgruppe darstellt:
Hydrierung einer Verbindung der allgemeinen Formel (VIII)

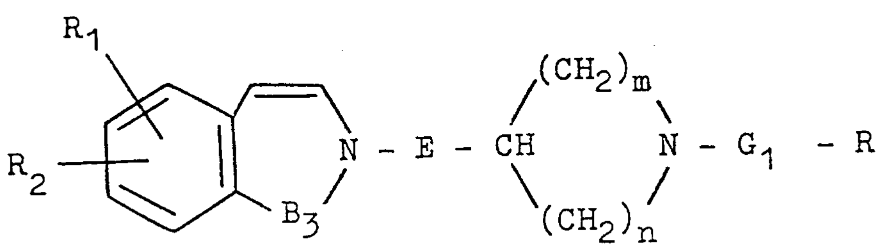

(VIII)

in der
R, $R_1$, $R_2$, B, E, m und n wie eingangs definiert sind,
$G_1$ mit Ausnahme der ein Schwefelatom, eine Sulfinyl- oder Sulfonylgruppe enthaltenden Reste die für G

eingangs erwähnten Bedeutungen besitzt und

$B_3$ eine $-CH_2-$ oder -CO-Gruppe darstellt.

Die Hydrierung wird in einem Lösungsmittel oder Lösungsmittelgemisch wie Methanol, Ethanol, Essigsäureethylester oder Eisessig mit katalytisch angeregtem Wasserstoff, z.B. mit Wasserstoff in Gegenwart von Platin oder Palladium/Kohle, bei einem Wasserstoffdruck von 1 bis 7 bar, vorzugsweise jedoch von 3 bis 5 bar, und bei Temperaturen zwischen 0 und 75 ° C, vorzugsweise jedoch bei Temperaturen zwischen 20 und 50 ° C, durchgeführt.

Bei den vorstehend beschriebenen Umsetzung können gegebenenfalls vorhandene reaktive Gruppen wie Hydroxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 ° C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 ° C, vorzugsweise jedoch bei Raumtemperatur, und einem Wasserstoffdruck von 1 bis 7 bar, vorzugsweise jedoch von 3 bis 5 bar.

Erhält man erfindungsgemäß eine Verbindung der allgemeinen Formel (I), in der R eine Nitrogruppe enthält, so kann diese mittels Reduktion in eine entsprechende Aminoverbindung der allgemeinen Formel I übergeführt werden oder

eine Verbindung der allgemeinen Formel I, in der R eine Aminogruppe enthält, so kann diese mittels Acylierung in eine entsprechende Alkanoylaminoverbindung der allgemeinen Formel (I) übergeführt werden.

Die nachträgliche Reduktion der Nitroverbindung wird vorzugsweise in einem Lösungsmittel wie Wasser, Wasser/Äthanol, Methanol, Eisessig, Essigsäureäthylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)chlorid oder Natriumdithionit oder mit Hydrazin in Gegenwart von Raney-Nickel bei Temperaturen zwischen 0 und 50 ° C, vorzugsweise jedoch bei Raumtemperatur, durchgeführt.

Die nachträgliche Acylierung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid vorzugsweise mit einem reaktiven Derivat der Säure, beispielsweise mit Acetylchlorid oder Acetanhydrid, und gegebenenfalls in Gegenwart einer anorganischen Base wie Natriumkarbonat oder einer tertiären organischen Base wie Triäthylamin oder Pyridin, welche gleichzeitig als Lösungsmittel dienen können, bei Temperaturen zwischen -25 ° C und 100 ° C, vorzugsweise jedoch bei Temperaturen zwischen -10 ° C und der Siedetemperatur des verwendeten Lösungsmittels, durchgeführt.

Die erhaltenen Verbindungen der allgemeinen Formel (I) lassen sich, da diese mindestens ein chirales Zentrum besitzen, mittels üblichen Methoden in ihre Diastereomeren, beispielsweise durch Säulenchromatographie, und in ihre Enantiomeren auftrennen, beispielsweise durch Säulenchromatographie an einer chiralen Phase oder durch Kristallisation mit optisch aktiven Säuren, z.B. mit D- oder L-Monomethylweinsäure, D- oder L-Diacetylweinsäure, D- oder L-Weinsäure, D- oder L-Milchsäure oder D- oder L-Camphersäure.

Die erhaltenen Verbindungen der allgemeinen Formel (I) lassen sich ferner in ihre Säureadditionssalze, insbesondere für ihre pharmazeutische Anwendung in ihre physiologisch verträglichen Säureadditionssalze mit anorganischen oder organischen Säuren überführen. Als Säuren kommen hierbei beispielsweise Salzsäure, Bromwasserstoffsäure, Schwefelsäure, Phosphorsäure, Essigsäure, Milchsäure, Zitronensäure, Weinsäure, Bernsteinsäure, Maleinsäure oder Fumarsäure in Betracht.

Die als Ausgangsstoffe verwendeten Verbindungen der allgemeinen Formeln (II) bis (VIII) sind teilweise literaturbekannt bzw. man erhält sie nach an sich bekannten Verfahren.

So erhält man beispielsweise eine Ausgangsverbindung der allgemeinen Formel (II) durch Alkylierung einer entsprechenden Iminoverbindung der allgemeinen Formel (IV) mit einem am N-Atom durch einen üblichen Schutzrest geschützten cyclischen Amin, das im Kohlenstoffgerüst durch einen Alkylrest substituiert ist, welcher seinerseits endständig durch eine nukleophile Austrittsgruppe substituiert ist, und anschließende Abspaltung des verwendeten Schutzrestes. Das hierfür erforderliche cyclische Amin erhält man durch Überführung eines entsprechenden durch einen Hydroxyalkylrest substituierten cyclischen Amins in dessen geeigneten Halogen- oder Sulfonsäureester und die hierfür erforderliche Iminoverbindung der allgemeinen Formel (IV) durch Cyclisierung einer entsprechenden Verbindung, z.B. durch Cyclisierung einer Verbindung der allgemeinen Formel (IX)

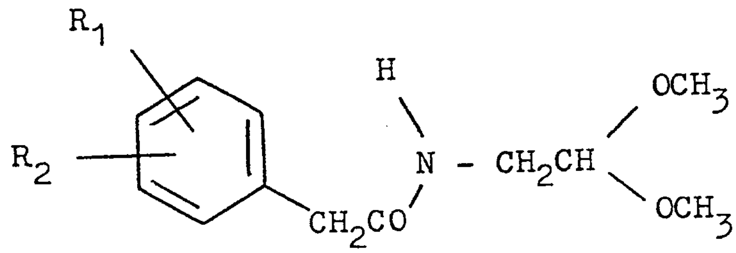

(IX)

oder auch der allgemeinen Formel (X)

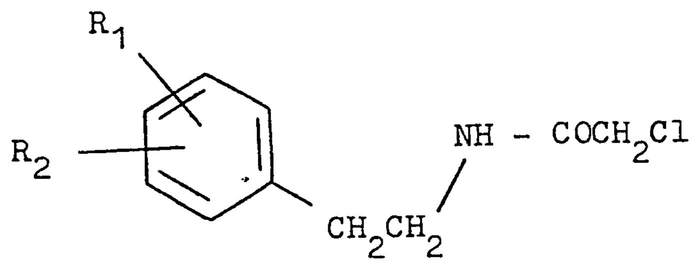

(X)

gegebenenfalls anschließender katalytischer Hydrierung und/oder Reduktion der Carbonylgruppe beispielsweise mit Natriumborhydrid/Eisessig (siehe EP-A1 0.007.070, EP-A1 0.065.229 und EP-A1 0.109.639).

Eine als Ausgangsstoff verwendete Verbindung der allgemeinen Formel (V) erhält man durch N-Alkylierung eines im Kohlenstoffgerüst durch einen Hydroxyalkylrest substituierten entsprechenden cyclischen Amins mit einer entsprechenden Verbindung oder mit einem entsprechenden $\alpha,\omega$ -Dihalogenalkan und anschließende Umsetzung mit einer entsprechenden HO- oder HN-Verbindung und erforderlichenfalls anschließende Oxidation, wobei dann eine so erhaltene Hydroxyalkylverbindung in ihren reaktionsfähigen Halogen- oder Sulfonsäureester übergeführt wird.

Eine als Ausgangsstoff verwendete Verbindung der allgemeinen Formeln (VI), (VII) und (VIII) erhält man vorzugsweise durch Umsetzung einer entsprechenden Halogenverbindung mit einem entsprechenden Amin und gegebenenfalls anschließende Abspaltung von Schutzresten, die zum Schutz von Aminogruppen verwendet werden.

Wie bereits eingangs erwähnt, weisen die neuen Verbindungen der allgemeinen Formel (I) und deren physiologisch verträgliche Säureadditionssalze mit anorganischen oder organischen Säuren wertvolle pharmakologische Eigenschaften auf, insbesondere bei geringen zentralen Nebenwirkungen eine blutdrucksenkende und eine besonders lang anhaltende herzfrequenzsenkende Wirkung sowie eine Herabsetzung des $0_2$-Bedarfs des Herzens.

Beispielsweise wurden die Verbindungen

A = 3-[(N-(2-(Naphthyl-2)-ethyl)-piperidyl-3)-methyl]-7,8-dimethoxy-2-oxo-1,3,4,5-tetrahydro-2H-3-benzazepin-hydrochlorid,

B = 3-[(N-(2-(5-Methyl-6-methoxy-naphthyl-2)-ethyl)-piperidyl-3)-methyl]-7,8-dimethoxy-2-oxo-1,3,4,5-tetrahydro-2H-3-benzazepin-hydrochlorid,

C = 3-[2-(N-(2-(6-Methoxy-naphthyl-2)-ethyl)-piperidyl-2)-ethyl]-7,8-dimethoxy-2-oxo-1,3,4,5-tetrahydro-2H-3-benzazepin-hydrochlorid,

D = 2-[(N-(2-(6-Methoxy-naphthyl-2)-ethyl)-hexahydro-azepinyl-3)-methyl]-6,7-methylendioxy-1-oxo-1,2,3,4-tetrahydro-isochinolin-hydrochlorid,

E = 2-[(N-(2-(Naphthyl-2)-ethyl)-hexahydro-azepinyl-3)-methyl]-6,7-methylendioxy-1-oxo-1,2,3,4-tetrahydro-isochinolin-hydrobromid,

F = 2-[(N-(2-(3,4-Dimethoxy-phenyl)-ethyl)-piperidyl-3)-methyl]-6,7-dimethyl-1,2,3,4-tetrahydro-isochinolin-dihydrochlorid,

G = 2-[(N-(2-(3,4-Dimethoxy-phenyl)-ethyl)-piperidyl-3)-methyl]-6,7-dimethyl-1-oxo-1,2,3,4-tetrahydro-isochinolin-hydrochlorid,

H = 2-[(N-(3-(4-Methoxy-phenoxy)-propyl)-piperidyl-3)-methyl]-6,7-dimethyl-1-oxo-1,2,3,4-tetrahydro-isochinolin-hydrochlorid,

I = 3-[(N-(4-(Thienyl-2)-butyl)-piperidyl-3)-methyl]-7,8-dimethoxy-2-oxo-1,3,4,5-tetrahydro-2H-3-benzazepin-hydrochlorid,

K = 3-[(N-(2-(Benzo[b]furyl-2)-ethyl)-piperidyl-3)-methyl]-7,8-dimethoxy-2-oxo-1,3,4,5-tetrahydro-2H-3-benzazepin-hydrochlorid und

L = 3-[(N-(2-(Benzo[b]thienyl-3)-ethyl)-piperidyl-3)-methyl]-7,8-dimethoxy-2-oxo-1,3,4,5-tetrahydro-2H-3-benzazepin-hydrochlorid

auf ihre biologischen Eigenschaften wie folgt untersucht:

Wirkung auf die Herzfrequenz an Ratten:

Die Wirkung der zu untersuchenden Substanzen auf die Herzfrequenz wurde pro Dosis an 2 Ratten mit einem durchschnittlichen Gewicht von 250-300 g untersucht. Hierzu wurden die Ratten mit Pentobarbital (50 mg/kg i.p. und 20 mg/kg s.c.) narkotisiert. Die zu untersuchenden Substanzen wurden in wäßriger Lösung in die Vena jugularis injiziert (0,1 ml/100 g).

Der Blutdruck wurde über in eine A. carotis eingebundene Kanüle gemessen und die Herzfrequenz wurde aus einem mit Nadelelektroden abgeleiteten EKG (II. oder III. Ableitung) registriert. Die Herzfrequenz der Tiere in der Kontrollperiode lagen zwischen 350 und 400 Schlägen/Minuten (S/min).

Die nachfolgende Tabelle enthält die gefundenen Werte:

| Substanz | Dosis [mg/kg] | Herzfrequenzsenkung in S/Min. nach | | Blutdrucksenkung in mmHg nach | |
|---|---|---|---|---|---|
| | | 5 Min. | 20 Min. | 5 Min. | 20 Min. |
| A | 5,0 | - 218 | - 259 | - 46 | - 32 |
| B | 5,0 | - 188 | - 218 | - 22 | - 15 |
| C | 5,0 | - 236 | - 194 | - 40 | - 31 |
| D | 5,0 | - 173 | - 123 | - 21 | - 16 |
| E | 5,0 | - 169 | - 150 | - 30 | - 16 |
| F | 5,0 | - 150 | - 120 | - 49 | - 27 |
| G | 5,0 | - 207 | - 101 | - 57 | - 8 |
| H | 5,0 | - 190 | - 180 | - 57 | - 33 |
| I | 5,0 | - 320 | - 253 | - 65 | - 32 |
| K | 2,5 | - 138 | - 156 | - 36 | |
| L | 2,5 | - 110 | - 163 | - 40 | - 24 |

Die erfindungsgemäß hergestellten Verbindungen weisen in therapeutischen Dosen keinerlei toxische Nebenwirkungen auf. So konnten beipielsweise bei einer intravenösen Applikation der Substanz A und L auch in einer hohen Dosis von 20 mg/kg an Mäusen, außer einer geringen Sedation keine toxischen Nebenwirkungen beobachtet werden.

Aufgrund ihrer pharmakologischen Eigenschaften eignen sich die erfindungsgemäß hergestellen Verbindungen zur Behandlung von Sinustachykardien verschiedener Genese und zur Prophylaxe und Therapie ischämischer Herzerkrankungen.

Die zur Erzielung einer entsprechenden Wirkung erforderlichen Dosierung beträgt zweckmäßigerweise ein- bis zweimal täglich 0,01 bis 0,2 mg/kg Körpergewicht, vorzugsweise 0,03 bis 0,15 mg/kg Körpergewicht. Hierzu lassen sich die erfindungsgemäß hergestellten Verbindungen der allgemeinen Formel I sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Carboxymethylcellulose oder fetthaltige Substanzen wie Hartfett oder deren geeignete Gemische, in übliche galenische Zubereitungen wie Tabletten, Dragees, Kapseln, Pulver, Suspensionen, Tropfen, Ampullen, Säfte oder Zäpfchen einarbeiten.

Die nachfolgenden Beispiele sollen die Erfindung näher erläutern:
Die Beispiele A - K betreffen Vorprodukte

Beispiel A

2-[(Piperidyl-3)-methyl]-6,7-dimethoxy-1-oxo-1,2,3,4-tetrahydro-isochinolin

a) N-Benzyl-3-(hydroxymethyl)-piperidin

Ein Gemisch von 40,3 g (0,35 Mol) 3-(Hydroxymethyl)-piperidin, 97,4 ml (0,70 Mol) Triethylamin und 40,3 ml (0,35 Mol) Benzylchlorid wird innerhalb von 30 Minuten auf 95°C erhitzt und 2 Stunden bei dieser Temperatur belassen. Das abgekühlte Reaktionsgemisch wird in einer Mischung aus 2 molarer Natronlauge und Essigester gelöst. Die organische Phase wird mit Wasser gewaschen, abgetrennt, über Magnesiumsulfat getrocknet und im Vakuum eingedampft.
Ausbeute: 57,2 g (79,6 % der Theorie),
Rf-Wert: 0,45 (Aluminiumoxid neutral, Laufmittel: 3 % Ethanol in Methylenchlorid).

b) N-Benzyl-3-(benzolsulfonyloxymethyl)-piperidin

Ein Gemisch von 6,8 g (0,033 Mol) N-Benzyl-3-(hydroxymethyl)-piperidin, 6,7 ml (0,052 Mol) Benzolsulfonsäurechlorid, 50 ml 20%ige wässrige Natronlauge, 100 ml Toluol und 1 Spatelspitze Tetrabutylammoniumbromid wird 3 Stunden bei Raumtemperatur gerührt. Anschließend verdünnt man mit 250 ml Essigester und wäscht die organische Phase mit Wasser. Die organische Phase wird abgetrennt, über Magnesiumsulfat getrocknet und im Vakuum zur Trockene eingedampft. Die Reinigung des Rückstandes erfolgt über 200 g Kieselgel (0.063 - 0.2 mm) mit Methylenchlorid und anschließend steigenden Anteilen von Ethanol (bis 5 %).
Ausbeute: 9,4 g (92 % der Theorie),
Rf-Wert: 0,5 (Kieselgel, Laufmittel: 5 % Ethanol in Methylenchlorid).

c) 2-[(N-Benzyl-piperidyl-3)-methyl]-6,7-dimethoxy-1-oxo-1,2,3,4-tetrahydro-isochinolin

5,2 g (0,025 Mol) 6,7-Dimethoxy-1-oxo-1,2,3,4-tetrahydro-isochinolin werden in 70 ml Dimethylsulfoxid gelöst und unter Rühren mit 3,1 g (0,025 Mol) Kalium-tert.butylat versetzt. Nach 1/2 Stunde wird zur erhaltenen Kaliumsalz-Suspension 9,3 g (0,0275 Mol) N-Benzyl-3-(benzolsulfonyloxy-methyl)-piperidin in 20 ml Dimethylsulfoyid gegeben und 2 Stunden bei 40°C gerührt. Man gießt auf Eiswasser und extrahiert 3 x mit je 120 ml Essigester. Die vereinigten organischen Phasen werden mit Wasser gewaschen, über Magnesiumsulfat getrocknet und im Vakuum eingedampft. Der erhaltene Rückstand wird über 200 g Kieselgel (0,063 - 0,2 mm) mit Methylenchlorid und anschließend steigenden Anteilen von Ethanol (bis 2 %) gereinigt.
Ausbeute: 7,7 g (78,5 % der Theorie),
Rf-Wert: 0,5 (Kieselgel, Laufmittel: 5 % Ethanol in Methylenchlorid und 1 Tropfen Ammoniak).

d) 2-[(Piperidyl-3)-methyl]-6,7-dimethoxy-1-oxo-1,2,3,4-tetrahydro-isochinolin

9,4 g (0,0238 Mol) 2-[(N-Benzyl-piperidyl-3)-methyl]-6,7-dimethoxy-1-oxo-1,2,3,4-tetrahydro-isochinolin werden in 200 ml Methanol in Gegenwart von 2 g 20%igem Palladiumhydroxid/Kohle 3 Stunden bei Raumtemperatur und 5 bar Wasserstoff hydriert. Anschließend wird der Katalysator abgesaugt und das Filtrat im Vakuum zur Trockene eingedampft.
Ausbeute: 7,2 g (99 % der Theorie),
Rf-Wert: 0,15 (Kieselgel, Laufmittel: 10 % Ethanol in Methylenchlorid und 1 Tropfen Ammoniak).

Beispiel B

3-Chlormethyl-N-[3-(naphthyl-2-oxy)-propyl]-piperidin

a) 3-(Hydroxymethyl)-N-[3-(naphthyl-2-oxy)-propyl]-piperidin

Ein Gemisch von 11,5 g (0,2 Mol) 3-Hydroxy-methyl-piperidin und 11 g 2-(3-Chlorpropoxy)-naphthalin wird 1 Stunde auf 120°C erhitzt. Der Rückstand wird über Kieselgel (0,063 - 0,2 mm) mit Essigester/Ethanol/Ammoniak = 90:10:1 gereinigt.
Ausbeute: 11,5 g (76,6 % der Theorie),
Schmelzpunkt: 99-101°C.

b) 3-Chlormethyl-N-[3-(naphthyl-2-oxy)-propyl]-piperidin

Eine Lösung von 1,5 g (5 mMol) 3-(Hydroxymethyl)-N-[3-(naphthyl-2-oxy)-propyl]-piperidin in 25 ml Chloroform wird mit 1,5 ml Thionylchlorid versetzt und 1 1/2 Stunden am Rückfluß gekocht. Es wird im Vakuum zur Trockene eingedampft. Der Rückstand wird in Methylenchlorid aufgenommen, mit Wasser, 2 molarer Natronlauge und erneut mit Wasser gewaschen. Nach dem Trocknen der Methylenchloridphase über Magnesiumsulfat wird im Vakuum eingedampft.
Ausbeute: 1,4 g (87,5 % der Theorie),
Rf-Wert: 0,8 (Kieselgel, Laufmittel:
Essigester/Ethanol/Ammoniak = 90:40:2).

Beispiel C

2-[(Hexahydro-azepinyl-3)-methyl]-6,7-methylendioxy-1-oxo-1,2,3,4-tetrahydro-isochinolin

a) N-Benzyl-caprolactam

33,9 g (0,3 Mol) Caprolactam werden in 250 ml Dimethylsulfoxid gelöst und unter Rühren 37 g (0,33 Mol) Kalium-tert.-butylat zugefügt. Hierbei steigt die Reaktionstemperatur auf 60°C an. Es wird 1/2 Stunde bei 60°C gerührt und anschließend 35 ml (0,3 Mol) Benzylbromid zugetropft. Nach weiteren 2 1/2 Stunden bei 60°C wird auf 1 l Eiswasser gegossen und 3 mal mit Essigester ausgeschüttelt. Die organischen Phasen werden vereinigt, mit Wasser gewaschen, über Magnesiumsulfat getrocknet und im Vakuum eingedampft.
Ausbeute: 60,3 g (99 % der Theorie),
Rf-Wert: 0,6 (Kieselgel, Laufmittel: 5 % Ethanol in Methylenchlorid).

b) 1-Benzyl-caprolactam-3-carbonsäure

Zu 33,9 g = 47,1 ml (0,33 Mol) Diisopropylamin in 450 ml absolutem Ether werden unter Rühren und Stickstoff bei -60°C 180 ml 2,6 molare Butyllithium-Lösung in n-Hexan zugegeben. Anschließend tropft man unter weiterer Kühlung 48,8 g (0,24 Mol) N-Benzyl-caprolactam, gelöst in 150 ml absolutem Ether, zu. Nach 10-minütigem Rühren entfernt man das Kältebad und leitet 15 Minuten lang Kohlendioxid ein. Das Reaktionsgemisch wird auf Eis gegossen, die ätherische Phase abgetrennt und 2 mal mit 2 molarer Natronlauge ausgeschüttelt. Die wässrig-alkoholischen Phasen werden vereinigt, mit Ether ausgeschüttelt, mit konzentrierter Salzsäure angesäuert und 2 mal mit Methylenchlorid ausgeschüttelt. Die vereinigten Methylenchlorid-Phasen werden über Magnesiumsulfat getrocknet und das Lösungsmittel im Vakuum abdestilliert.
Ausbeute: 15,7 g (26,5 % der Theorie),
IR-Spektrum (Methylenchlorid): 1735 und 1600 $cm^{-1}$ (CO).

c) 1-Benzyl-3-hydroxymethyl-hexahydro-azepin

Zu 6,84 g (0,28 Mol) Lithiumaluminiumhydrid in 300 ml absolutem Tetrahydrofuran werden 14,8 g (0,06 Mol) 1-Benzyl-caprolactam-3-carbonsäure,gelöst in 300 ml absolutem Tetrahydrofuran, getropft. Danach wird 6 Stunden unter Rückfluß erhitzt, anschließend unter Eiswasserkühlung mit 6,8 ml Wasser, 6,8 ml 2-molarer Natronlauge und 21 ml Wasser versetzt. Der Niederschlag wird abgesaugt, mit Tetrahydrofuran

nachgewaschen und das Filtrat im Vakuum eingeengt. Der Rückstand wird säulenchromatographisch über Aluminiumoxid N (Aktivität II, Elutionsmittel: Methylenchlorid) gereinigt.
Ausbeute: 8,4 g (63,8 % der Theorie),
IR-Spektrum (Methylenchlorid): 3620 $cm^{-1}$ (OH).

d) 1-Benzyl-3-(4-toluolsulfonyloxymethyl)-hexahydro-azepin

15 g (0,0684 Mol) 1-Benzyl-3-hydroxymethyl-hexahydro-azepin werden in 150 ml Pyridin gelöst und unter Rühren mit 14,3 g (0,075 Mol) p-Toluolsulfonsäurechlorid versetzt und 1 Stunde bei Raumtemperatur gerührt. Es wird im Vakuum eingedampft, in Methylenchlorid aufgenommen, mit 2 molarer Natronlauge und Wasser gewaschen. Nach dem Trocknen über Magnesiumsulfat wird die organische Phase im Vakuum eingedampft.
Ausbeute: 23,3 g (91,3 % der Theorie),
Rf-Wert: 0,45 (Kieselgel, Laufmittel: 5 % Ethanol in Methylenchlorid).

e) 2-[(N-Benzyl-hexahydro-azepinyl-3)-methyl]-6,7-methylendioxy-1-oxo-1,2,3,4-tetrahydro-isochinolin

7,4 g (0,0387 Mol) 6,7-Methylendioxy-1-oxo-1,2,3,4-tetrahydro-isochinolinwerden in 100 ml Dimethylsulfoxid gelöst, mit 4,5 g (0,04 Mol) Kalium-tert.butylat versetzt und 1/2 Stunde bei Raumtemperatur gerührt. Anschließend werden 16,8 g (0,044 Mol) 1-Benzyl-3-(4-toluolsulfonyloxymethyl)-hexahydro-azepin zugegeben und 3 Stunden bei Raumtemperatur gerührt. Das Reaktionsgemisch wird in Essigester gelöst und 2 mal mit Wasser ausgeschüttelt. Die organische Phase wird über Magnesiumsulfat getrocknet und im Vakuum eingeengt. Der Rückstand wird über Aluminiumoxid N (Aktivität II, Elutionsmittel: Methylenchlorid, Methylenchlorid + 2 % Ethanol) gereinigt.
Ausbeute: 3,0 g (19,6 % der Theorie),
Rf-Wert: 0,6 (Kieselgel, Laufmittel: 5 % Ethanol in Methylenchlorid).

f) 2-[(Hexahydro-azepinyl-3)-methyl]-6,7-methylendioxy-1-oxo-1,2,3,4-tetrahydro-isochinolin

6,7 g (0,017 Mol) 2-[(N-Benzyl-hexahydro-azepinyl-3)-methyl]-6,7⁻methylendioxy-1-oxo-1,2,3,4-tetrahydro-isochinolinwerden in 250 ml Methanol in Gegenwart von 2 g 20%igem Palladiumhydroxid/Kohle 4 Stunden bei Raumtemperatur und 5 bar Wasserstoff hydriert. Anschließend wird der Katalysator abgesaugt und das Filtrat im Vakuum zur Trockene eingedampft. Der Rückstand kristallisiert aus Aceton.
Ausbeute: 4,9 g (95 % der Theorie),
Schmelzpunkt: 267-269 ° C.

Beispiel D

3-Chlormethyl-N-[3-(naphthyl-2-oxy)-propyl]-hexahydro-azepin

a) 3-Hydroxymethyl-hexahydro-azepin

16,6 g (0,0757 Mol) 1-Benzyl-3-hydroxymethyl-hexahydro-azepin werden in 500 ml Methanol in Gegenwart von 16,6 g 20%-igem Palladiumhydroxid/Kohle 2 Stunden bei Raumtemperatur und 5 bar Wasserstoff hydriert. Anschließend wird der Katalysator abgesaugt und das Filtrat im Vakuum eingedampft.
Ausbeute: 8 g (81,8 % der Theorie),
Rf-Wert: 0,5 (Kieselgel, Laufmittel: Methylenchlorid/Ethanol/Ammoniak = 5:4:1).

b) 3-Hydroxymethyl-N-[3-(naphthyl-2-oxy)-propyl]-hexahydro-azepin-hydrochlorid

Ein Gemisch von 7,8 g (0,06 Mol) 3-Hydroxymethyl-hexahydro-azepin und 6,7 g (0,03 Mol) 2-(3-Chlorpropoxy)-naphthalin werden 1 Stunde auf 120 ° C erhitzt. Das Reaktionsgemisch wird über Kieselgel (0,063 - 0,2 mm) mit Essigester/Ethanol/Ammoniak = 90:10:1 gereinigt.
Ausbeute: 2,3 g (24,3 % der Theorie),
Schmelzpunkt: 127-129 ° C.

c) 3-Chlormethyl-N-[3-(naphthyl-2-oxy)-propyl]-hexahydro-azepin

2,4 g (6,86 mMol) 3-Hydroxymethyl-N-[3-(naphthyl-2-oxy)-propyl]-hexahydro-azepin-hydrochlorid, gelöst in 30 ml Chloroform, werden mit 5 ml Thionylchlorid versetzt und 1 Stunde am Rückfluß gekocht. Es wird im Vakuum zur Trockne eingedampft. Der Rückstand wird in Methylenchlorid gelöst, mit Wasser, 2 molarer Natronlauge und erneut mit Wasser ausgeschüttelt. Die Methylenchlorid-Phase wird über Magnesiumsulfat getrocknet und im Vakuum eingedampft.
Ausbeute: 1,1 g (48,5 % der Theorie),
Rf-Wert: 0,55 (Kieselgel, Laufmittel: 5 % Ethanol in Methylenchlorid).

Beispiel E

2-[(Pyrrolidyl-3)-methyl]-6,7-dimethoxy-1-oxo-1,2,3,4-tetrahydro-isochinolin

a) N-Benzyl-2-pyrrolidon

Zu 25,5 g (0,3 Mol) 2-Pyrrolidon in 300 ml absolutem Dimethylsulfoxid werden portionsweise 14,4 g (0,33 Mol) 50%ige Natriumhydrid-Dispersion in Öl eingetragen. Anschließend wird 5 Stunden bei 40 bis 50 °C gerührt und bei 25-30 °C 56,4 g = 39,2 ml (0,33 Mol) Benzylbromid zugetropft. Nach 10-stündigem Rühren bei Raumtemperatur wird das Reaktionsgemisch in 500 ml Essigester gelöst und mehrmals mit Wasser ausgeschüttelt. Die organische Phase wird abgetrennt über Magnesiumsulfat getrocknet und das Lösungsmittel im Vakuum entfernt. Der erhaltene Rückstand wird über 900 g Aluminiumoxid (neutral, Aktivität II) mit Methylenchlorid und 0,1 % Ethanol gereinigt.
Ausbeute: 35,6 g (67,7 % der Theorie),
Rf-Wert: 0,77 (Aluminiumoxid, neutral, Laufmittel: 5 % Ethanol in Methylenchlorid).

b) N-Benzyl-2-pyrrolidon-3-carbonsäure

Zu 28,3 g = 39,3 ml (0,28 Mol) Diisopropylamin in 400 ml absolutem Ether werden unter Rühren und unter Stickstoff bei -60 °C 150 ml 1,6 molare Butyllithium-Lösung in n-Hexan gegeben. Hierzu tropft man 35,1 g (0,2 Mol) N-Benzyl-2-pyrrolidon, gelöst in 150 ml absolutem Ether, bei -60 °C. Man entfernt das Kältebad und leitet 15 Minuten trockenes Kohlendioxid ein. Nach 10-minütigem Rühren wird auf Eis gegossen, die organische Phase abgetrennt und 2 mal mit 2 molarer Natronlauge ausgeschüttelt. Die vereinigten wässrigen Phasen werden einmal mit Ether ausgeschüttelt und anschließend unter Kühlung mit konzentrierter Salzsäure angesäuert. Die wässrige Phase wird 2 mal mit Methylenchlorid ausgeschüttelt und nach dem Trocknen der organischen Phase über Magnesiumsulfat im Vakuum eingeengt.
Ausbeute: 35 g (79,8 % der Theorie),
Rf-Wert: 0,42 (Kieselgel, Laufmittel: 5 % Ethanol in Methylenchlorid).

c) N-Benzyl-3-hydroxymethyl-pyrrolidin

Zu 12,2 g (0,32 Mol) Lithiumaluminiumhydrid in 350 ml absolutem Tetrahydrofuran tropft man unter Rühren 35 g (0,16 Mol) N-Benzyl-2-pyrrolidon-3-carbonsäure, gelöst in 250 ml absolutem Tetrahydrofuran. Nach 6-stündigem Erhitzen unter Rückfluß versetzt man unter Eiswasserkühlung mit 18,2 ml Wasser, 12,2 ml 15%ige wässrige Natronlauge und 36,6 ml Wasser. Der entstandene Niederschlag wird abgesaugt und mit Tetrahydrofuran gewaschen. Die vereinigten Filtrate werden im Vakuum eingeengt und der erhaltene Rückstand über 900 g Aluminiumoxid (neutral, Aktivität II) mit Methylenchlorid und anschließend mit steigenden Anteilen von Ethanol (bis 2 %) gereinigt.
Ausbeute: 16 g (52,3 % der Theorie),
Rf-Wert: 0,42 (Aluminiumoxid, neutral, Laufmittel: 5 % Ethanol in Methylenchlorid).

d) 3-(Benzolsulfonyloxymethyl)-1-benzyl-pyrrolidin

Zu einem Gemisch von 3,8 g (20 mMol) N-Benzyl-3-hydroxymethyl-pyrrolidin und 3,7 ml (24 mMol) Benzolsulfonsäurechlorid werden 40 ml 20%ige Natronlauge während einer Stunde zugetropft. Man versetzt mit 150 ml Toluol, wäscht die organische Phase mit Wasser, trocknet über Magnesiumsulfat und dampft im Vakuum zur Trockene ein.
Ausbeute: 5,9 g (89,4 % der Theorie),

Rf-Wert: 0,4 (Kieselgel, Laufmittel: 5 % Ethanol in Methylenchlorid).

e) 2-[N-(Benzyl-pyrrolidyl-3)-methyl]-6,7-dimethoxy-1-oxo-1,2,3,4-tetrahydro-isochinolin

3,3 g (15,9 mMol) 6,7-Dimethoxy-1-oxo-1,2,3,4-tetrahydro-isochinolin werden in 50 ml Dimethylsulfoxid gelöst und unter Rühren bei Raumtemperatur mit 2 g (17,5 mMol) Kalium-tert.butylat versetzt. Nach 1/2 Stunde wird zur entstandenen Kaliumsalz-Suspension 5,8 g (17,5 mMol) 3-(Benzolsulfonyloxymethyl)-1-benzyl-pyrrolidin in 10 ml Dimethylsulfoxid gegeben und 3 Stunden bei 60 °C gerührt. Man gießt auf Eiswasser und extrahiert mit Essigester. Die vereinigten organischen Phasen werden mit Wasser gewaschen und über Magnesiumsulfat getrocknet. Die organische Phase wird im Vakuum eingeengt und der erhaltene Rückstand über 350 g Aluminiumoxid N (Aktivität II) mit Methylenchlorid und anschließend steigenden Anteilen Ethanol (bis 1 %) gereinigt.
Ausbeute: 3,3 g (54,4 % der Theorie),
Rf-Wert: 0,74 (Aluminiumoxid N, Laufmittel: 5 % Ethanol in Methylenchlorid).

f) 2-[(Pyrrolid-3-yl)-methyl]-6,7-dimethoxy-1-oxo-1,2,3,4-tetrahydro-isochinolin

3,2 g (8,4 mMol) 2-[(N-Benzyl-pyrrolid-3-yl)-methyl]-6,7-dimethoxy-1-oxo-1,2,3,4-tetrahydro-isochinolin werden in 250 ml Methanol gelöst und in Gegenwart von 1 g 20%igem Palladiumhydroxid/Kohle 3 Stunden bei Raumtemperatur und 5 bar Wasserstoff hydriert. Anschließend wird der Katalysator abgesaugt und das Filtrat im Vakuum zur Trockene eingedampft. Der Rückstand wird über 150 g Kieselgel (0,063 - 0,2 mm) mit Methylenchlorid/Ethanol/Ammoniak = 6:1:0,5 gereinigt.
Ausbeute: 0,9 g (37,5 % der Theorie),
Rf-Wert: 0,6 (Kieselgel, Laufmittel: Methylenchlorid/Ethanol/Ammoniak = 5:4:1).

Beispiel F

3-(p-Toluolsulfonyloxymethyl)-N-[2-(6-methoxy-naphthyl-2)-ethyl]-pyrrolidin

a) 3-Hydroxymethyl-pyrrolidin

14 g (0,073 Mol) N-Benzyl-3-hydroxymethyl-pyrrolidin werden 7 Stunden lang bei 50 °C und 5 bar in 300 ml Methanol und in Gegenwart von 1,5 g 20%igem Palladiumhydroxid/Aktivkohle hydriert. Anschließend wird der Katalysator abgesaugt und das Filtrat im Vakuum eingeengt.
Ausbeute: 7,3 g (99 % der Theorie),
Massenspektrum: Molpeak 101.

b) 3-Hydroxymethyl-N-[2-(6-methoxy-naphthyl-2)-ethyl]-pyrrolidin

Ein Gemisch von 3,6 g (29,4 mMol) 3-Hydroxymethyl-pyrrolidin und 4,7 g (14,7 mMol) 2-(2-Bromethyl)-6-methoxy-naphthalin wird 2 Stunden auf 120 °C erhitzt. Das Reaktionsgemisch wird über 200 g Kieselgel (0,063 - 0,2 mm) mit Methylenchlorid und anschließend steigenden Anteilen Ethanol (bis 5 %) gereinigt.
Ausbeute: 3,48 g (82,5 % der Theorie),
Schmelzpunkt: 121-123 °C.

c) 3-(p-Toluolsulfonyloxymethyl)-N-[2-(6-methoxy-naphthalin-2)-ethyl]-pyrrolidin

0,8 g (2,8 mMol) 3-Hydroxymethyl-N-[2-(6-methoxy-naphth-2-yl)-ethyl]-pyrrolidin werden in 10 ml Pyridin gelöst und unter Rühren 1,2 g (6,3 mMol) p-Toluolsulfonsäurechlorid zugefügt. Nach 2 Stunden bei Raumtemperaturen wird im Vakuum zur Trockne eingedampft. Den Rückstand löst man in Methylenchlorid, wäscht mit 2 molarer Natronlauge und Wasser. Man trocknet anschließend die organische Phase über Magnesiumsulfat und engt im Vakuum ein. Der Rückstand wird über 150 g Kieselgel (0,063 - 0,2 mm) mit Essigester und anschließend steigenden Anteilen Ethanol gereinigt.
Ausbeute: 0,6 g (48,8 % der Theorie),
Rf-Wert: 0,45 (Kieselgel, Laufmittel: 5 % Ethanol in Methylenchlorid).

Beispiel G

2-[(Azacyclooctyl-3)-methyl]-6,7-dimethoxy-1-oxo-1,2,3,4-tetrahydro-isochinolin

a) 1-Benzyl-2-oxo-azacyclooctan

25 g (0,196 Mol) 2-Azacyclooctanon werden in 150 ml Dimethylsulfoxid gelöst und unter Rühren mit 24,2 g (0,216 Mol) Kalium-tert.butylat versetzt und 1/2 Stunde bei 40 °C gerührt. Anschließend werden 24 ml (0,2 Mol) Benzylbromid während 1/4 Stunde zugetropft, dabei steigt die Temperatur auf 80 °C an. Es wird 2 Stunden gerührt, wobei die Reaktionstemperatur wieder auf Raumtemperatur sinkt. Das Reaktionsgemisch wird auf 1 l Eiswasser gegossen und 4 mal mit je 150 ml Essigester ausgeschüttelt. Die vereinigte organische Phase wird mit Wasser gewaschen, über Magnesiumsulfat getrocknet und im Vakuum eingedampft.
Ausbeute: 42,7 g (100 % der Theorie),
Rf-Wert: 0,55 (Kieselgel, Laufmittel: 5 % Ethanol in Methylenchlorid).

b) 1-Benzyl-azacyclooctan-2-oxo-3-carbonsäure

Zu 26,7 g = 38,4 ml (0,26 Mol) Diisopropylamin in 250 ml absolutem Ether werden unter Rühren und Stickstoff bei -60 °C 147 ml 1,6 molare Butyllithium-Lösung in n-Hexan zugetropft. Anschließend tropft man bei -60 °C 42,7 g (0,196 Mol) 1-Benzyl-2-oxo-azacyclooctan in 100 ml absolutem Ether, zu. Nach 10 Minuten werden 20 Minuten lang trockenes Kohlendioxid eingeleitet. Das Reaktionsgemisch wird auf Eis gegossen, die etherische Phase abgetrennt und 2 mal mit 2 molarer Natronlauge ausgeschüttelt. Die vereinigten wässrigen Phasen werden 1 mal mit Ether ausgeschüttelt und anschließend unter Kühlung mit konzentrierter Salzsäure angesäuert. Man schüttelt 3 mal mit Methylenchlorid aus, trocknet die Methylenchlorid-Phase über Magnesiumsulfat und engt im Vakuum ein. Ausbeute: 25,9 g (50,6 % der Theorie),
Rf-Wert: 0,15 (Aluminiumoxid, Laufmittel: 5 % Ethanol in Methylenchlorid).

c) 1-Benzyl-3-hydroxymethyl-azacyclooctan

Zu 22,7 g (0,6 Mol) Lithiumaluminiumhydrid in 800 ml absolutem Ether tropft man unter Rühren 53,6 g (0,205 Mol) 1-Benzyl-2-oxo-azacyclooctan-3-carbonsäure, gelöst in 100 ml absolutem Tetrahydrofuran. Nach 1/2-stündigem Erhitzen unter Rückfluß versetzt man unter Eiswasserkühlung mit 28,4 ml Wasser, 19 ml 15%iger Natronlauge und 57 ml Wasser. Der entstandene Niederschlag wird abgesaugt und mit Tetrahydrofuran gewaschen. Die vereinigten Filtrate werden im Vakuum eingeengt und der erhaltene Rückstand über 700 g Aluminiumoxid (neutral, Aktivität II) mit 1 % Ethanol in Methylenchlorid gereinigt.
Ausbeute: 9,3 g (19,6 % der Theorie),
Rf-Wert: 0,5 (Kieselgel, Laufmittel: 5 % Ethanol in Methylenchlorid).

d) 1-Benzyl-3-chlormethyl-azacyclooctan

9,3 g (39,8 mMol) 1-Benzyl-3-hydroxymethyl-azacyclooctan werden in 30 ml Pyridin mit 10 ml (79,6 mMol) Benzolsulfonsäurechlorid versetzt und 2 1/2 Stunden bei Raumtemperatur gerührt. Das Reaktionsgemisch wird im Vakuum eingedampft. Der verbleibende Rückstand wird in 150 ml Methylenchlorid gelöst, mit 2n Natronlauge und Wasser gewaschen. Man trocknet die organische Phase über Magnesiumsulfat, dampft zur Trockene ein und reinigt über 150 g Kieselgel (0,063-0,2 mm) mit Methylenchlorid.
Ausbeute: 3,5 g (35 % der Theorie)
Rf-Wert: 0,75 (Kieselgel, Laufmittel: 5 % Ethanol in Methylenchlorid).

e) 2-[(N-Benzyl-azacyclooctyl-3)-methyl]-6,7-dimethoxy-1-oxo-1,2,3,4-tetrahydro-isochinolin

2,3 g (11,1 mMol) 6,7-Dimethoxy-1-oxo-1,2,3,4-tetrahydro-isochinolin werden in 40 ml Dimethylsulfoxid gelöst und unter Rühren mit 1,33 g (12,2 mMol) Kalium-tert.butylat versetzt. Nach 1/2 Stunde wird zur erhaltenen Kaliumsalzsuspension 3,5 g (9,4 mMol) 1-Benzyl-3-chlormethyl-azacyclooctan in 40 ml Dimethylsulfoxid gegeben und 2 1/2 Stunden bei 120 °C gerührt. Man gießt auf Eiswasser und extrahiert 3 mal mit je 50 ml Essigester. Die vereinigten organischen Phasen werden mit Wasser gewaschen, über Magnesiumsulfat getrocknet und im Vakuum eingedampft. Der erhaltene Rückstand wird über 150 g Kieselgel (0,063-0,2 mm) mit 1 % Ethanol in Methylenchlorid gereinigt.

Ausbeute: 1 g (25,1 % der Theorie),
Rf-Wert: 0,5 (Kieselgel, Laufmittel: 2 % Ethanol in Methylenchlorid)

f) 2-[(Azacyclooctyl-3)-methyl]-6,7-dimethoxy-1-oxo-1,2,3,4-tetrahydro-isochinolin

0,85 g (2 mMol) 2-[(N-Benzyl-azacyclooctyl-3)-methyl]-6,7-dimethoxy-1-oxo-1,2,3,4-tetrahydro-isochinolin werden in 50 ml Methanol in Gegenwart von 0,85 g 20%igem Palladiumhydroxid/Kohle 4 1/2 Stunden bei Raumtemperatur und 5 bar Wasserstoff hydriert. Anschließend wird der Katalysator abgesaugt und das Filtrat zur Trockene eingedampft.
Ausbeute: 0,5 (74,6 % der Theorie),
Rf-Wert: 0,45 (Kieselgel, Laufmittel: 25 % Ethanol in Methylenchlorid und 1 Tropfen Ammoniak)

Beispiel H

1-Chlor-3-[N-(4-methoxy-phenyl)-methylamino]-propan

10 g (0,073 Mol) N-Methyl-4-methoxy-anilin werden in 50 ml Dimethylsulfoxid gelöst und unter Rühren 9 g (0,08 Mol) Kalium-tert.butylat zugefügt. Nach 1/2 Stunde wird 10 ml 1-Brom-3-chlorpropan zugegeben und 3 Stunden bei Raumtemperatur gerührt. Man gießt auf Eiswasser, schüttelt mit Essigester aus, wäscht die organische Phase mit Wasser, trocknet über Natriumsulfat und engt zur Trockene ein. Der Rückstand wird über Kieselgel (0,063 - 0,2 mm) mit Methylenchlorid gereinigt.
Ausbeute: 9,1 g (58,3 % der Theorie),
Rf-Wert: 0,55 (Kieselgel, Laufmittel: Methylethylketon/Xylol = 1:6)

| | | | | | | | | |
|---|---|---|---|---|---|---|---|---|
| Ber.: | C | 61,82 | H | 7,55 | N | 6,55 | Cl | 16,59 |
| Gef.: | | 61,71 | | 7,88 | | 6,69 | | 16,24. |

Beispiel I

2-[(N-(3-(Pyridyl-3)-propyl)-piperidyl-3)-methyl]-5,6-methylendioxy-phthalimid

2,1 g (0,011 Mol) 5,6-Methylendioxy-phthalimid werden in 100 ml Dimethylsulfoxid gelöst und unter Rühren 1,25 g (0,012 Mol) Kalium-tert.butylat zugesetzt. Das Kaliumsalz fällt aus. Es wird 1/2 Stunde bei Raumtemperatur nachgerührt, eine Lösung von 2,5 g (0,01 Mol) 3-Chlormethyl-N-(3-(pyridyl-3)-propyl)-piperidin in 20 ml Dimethylsulfoxid zugegeben und 8 Stunden auf 120 °C erhitzt. Es wird auf Eiswasser gegossen, 3 mal mit je 150 ml Essigester ausgeschüttelt und die organische Phase nach dem Trocknen über Magnesiumsulfat im Vakuum eingeengt. Der Rückstand wird über 200 g Kieselgel (0,063 - 0,2 mm) mit Essigester/Ethanol/Ammoniak = 80:10:0,5 gereinigt.
Ausbeute: 3 g (74 % der Theorie),

| | | | | | | | | |
|---|---|---|---|---|---|---|---|---|
| Ber. (2 x HCl): | C | 55,42 | H | 5,86 | N | 8,43 | Cl | 14,22 |
| Gef.: | | 55,28 | | 6,06 | | 8,26 | | 14,64 |

Beispiel K

2-[(N-3-Chlorpropyl)-piperidyl-3-methyl]-6,7-dimethoxy-1-oxo-1,2,3,4-tetrahydro-isochinolin

3 g (0,01 Mol) 2-[(Piperidyl-3)-methyl]-6,7-dimethoxy-1-oxo-1,2,3,4-tetrahydro-isochinolin werden in 50 ml Dimethylsulfoxid gelöst und unter Rühren 1,3 g (0,011 Mol) Kalium-tert.-butylat zugegeben. Nach 1/2 Stunde werden 3 ml 1-Brom-3-chlor-propan zugefügt und eine Stunde bei Raumtemperatur gerührt. Man gießt in Eiswasser, extrahiert mit Essigester, wäscht die organische Phase mit Wasser, trocknet über Natriumsulfat und engt im Vakuum zur Trockene ein.
Ausbeute: 2,7 g (71 % der Theorie),
Rf-Wert: 0,65 (Kieselgel, Laufmittel: Essigester/Ethanol/Ammoniak = 50:45:5).

Beispiel 1

2-[(N-(3-(Naphthyl-2)-propyl)-piperidyl-3)-methyl]-6,7-dimethoxy-1-oxo-1,2,3,4-tetrahydro-isochinolin-hydrochlorid

Ein Gemisch von 1 g (3,2 mMol) 2-(Piperidyl-3-methyl)-6,7- dim ethoxy-1-oxo-1,2,3,4-tetrahydro-isochinolin, 5 ml Dime- thylsu lfoxid, 0,5 g (0,36 mMol) Kaliumcarbonat und 0,75 g (3,66 mMol ) 2-(3-Chlorpropyl)-naphthalin wird 3 Stunden auf 120 °C erhitz t. Das Reaktionsgemisch wird auf Eiswasser gegossen und 3 ma 1 mit je 50 ml Essigester ausgeschüttelt. Die vereinigten orga nischen Phasen werden mit 2 molarer Natron- lauge und Wasser g ewaschen, über Magnesiumsulfat getrocknet, im Vakuum eingeengt und der erhaltene Rückstand über Kiesel- gel (0,063 - 0,2 mm) mit 1 % Ethanol in Methylenchlorid gereinigt. Aus einer Lös ung in Aceton wird mit etherischer Salzsäure das Hydrochlorid gefällt und aus Aceton kristalli- siert.
Ausbeute: 0,74 g (44 % der Theorie),
Schmelzpunkt: 179-181 °C

| | | | | | | | | |
|---|---|---|---|---|---|---|---|---|
| Ber.:<br>Gef.: | C | 70,77<br>70,47 | H | 7,37<br>7,40 | N | 5,50<br>5,47 | Cl | 6,96<br>7,06. |

Beispiel 2

2-[(N-(3-(Naphthyl-2-oxy)-propyl)-piperidyl-3)-methyl]-6,7-dimethyl-1-oxo-1,2,3,4-tetrahydro-isochinolin-hydrochloridhydrat

1,58 g (9 mMol) 6,7-Dimethyl-1-oxo-1,2,3,4-tetrahydro-isochinolin werden in 30 ml Dimethylsulfoxid gelöst und unter Rühren mit 1,1 g (9,9 mMol) Kalium-tert.butylat versetzt. Nach einer Stunde wird eine Lösung von 2,9 g (9,1 mMol) 3-Chlormethyl-N-[3-(naphthyl-2-oxy)-propyl]-piperidin in 10 ml Dimethylsulfoxid zugegeben und die Reaktionsmischung 16 Stunden bei 80 °C gerührt. Anschließend gießt man auf Eiswasser, schüttelt 3 mal mit 50 ml Essigester aus, wäscht die organische Phase mit Wasser, trocknet über Magnesiumsulfat und reinigt nach dem Eindampfen über Kieselgel (0,63 - 0,2 mm) mit Essigester/Ethanol/Ammoniak = 95:5:0,5. Aus einer Lösung in Aceton erhält man mit etherischer Salzsäure das Hydrochlorid als Hydrat.
Ausbeute: 2 g (45,1 % der Theorie),
Schmelzpunkt: 152-154 °C

| | | | | | | | | |
|---|---|---|---|---|---|---|---|---|
| Ber.:<br>Gef.: | C | 70,50<br>70,31 | H | 7,69<br>7,52 | N | 5,49<br>5,49 | Cl | 6,93<br>7,10 |

Beispiel 3

2-[(N-(3-(Naphthyl-2)-propyl)-piperidyl-3)-methyl]-6,7-dimethyl-1,2,3,4-tetrahydro-isochinolin-dihydrochlorid

0,8 g (18 mMol) 2-[(N-(3-(Naphthyl-2)-propyl)-piperidyl-3)-methyl]-6,7-dimethyl-1-oxo-1,2,3,4-tetrahydro-isochinolin werden in 10 ml absolutem Tetrahydrofuran und 20 ml absolutem Ether gelöst, 70 mg (18 mMol) Lithiumaluminiumhydrid zugefügt und 1 Stunde am Rückfluß gekocht. Man zersetzt das Reaktionsgemisch durch Zugabe von 5 ml gesättigter wässriger Natriumsulfatlösung, filtriert vom ausgefallenen Natriumsulfat ab und wäscht mit Tetrahydrofuran nach. Das Filtrat wird über Magnesiumsulfat getrocknet und nach dem Einengen über 150 g Kieselgel (0,063 - 0,2 mm) mit Essigester/Ethanol/Ammoniak = 90:10:0,05 gereinigt. Aus einer Lösung in Aceton wird mit etherischer Salzsäure das Hydrochlorid gefällt.
Ausbeute: 0,49 g (54,4 % der Theorie),
Schmelzpunkt: 148-150 °C

| | | | | | | | | |
|---|---|---|---|---|---|---|---|---|
| Ber.: | C | 69,61 | H | 8,18 | N | 5,41 | Cl | 13,70 |
| Gef.: | | 69,46 | | 8,32 | | 5,26 | | 14,17 |

Beispiel 4

2-[(N-(3-(Pyridyl-3)-propyl)-piperidyl-3)-methyl]-5,6-methylendioxy-1-oxo-1,3-dihydro-isoindol-dihydrochlorid

2,4 g (5,9 mMol) 2-[(N-(3-(Pyridyl-3)-propyl)-piperidyl-3)-methyl]-5,6-methylendioxy-phthalimid werden in 50 ml Eisessig gelöst und 5 Stunden am Rückfluß gekocht. Im Abstand von je einer Stunde wird jeweils 1 g Zinkstaub zugegeben. Nach beendeter Reaktionszeit wird abgesaugt, mit Ethanol eingedampft. Der Rückstand wird in Methylenchlorid gelöst, mit konzentriertem Ammoniak ausgeschüttelt, über Magnesiumsulfat getrocknet und nach dem Einengen im Vakuum über 150 g Kieselgel (0,063 - 0,2 mm) Essigester/Ethanol/Ammoniak = 90:10:0,2 gereinigt. Aus einer Lösung in Aceton wird das Hydrochlorid gefällt.
Ausbeute: 2,05 g (75 % der Theorie),
Schmelzpunkt: 165-167 ° C

| | | | | | | | | |
|---|---|---|---|---|---|---|---|---|
| Ber.: | C | 59,22 | H | 6,27 | N | 9,00 | Cl | 15,20 |
| Gef.: | | 59,03 | | 6,45 | | 8,85 | | 15,06 |

Beispiel 5

3-[(N-(3-(Pyridyl-3)-propyl)-pyrrolidyl-3)-methyl]-7,8-dimethoxy-2-oxo-1,3,4,5-tetrahydro-2H-3-benzoazepin-dihydrochlorid

1,1 g (2,6 mMol) 3-[(N-(3-(Pyridyl-3)-propyl)-pyrrolidyl-3)-methyl]-7,8-dimethoxy-2-oxo-1,3-dihydro-2H-3-benzoazepin, gelöst in 50 ml Ethanol, werden in Gegenwart von 1 g 10%igem Palladium auf Aktivkohle bei 80 ° C und 5 bar Wasserstoff 2 Stunden lang hydriert. Anschließend wird der Katalysator abgesaugt und das Filtrat nach dem Einengen im Vakuum über 100 g Kieselgel (0,063 - 0,2 mm) mit Essigester/Ethanol/Ammoniak = 80:40:1 gereinigt. Aus einer Lösung in Aceton wird das Hydrochlorid gefällt.
Ausbeute: 0,37 g (33 % der Theorie),
Schmelzpunkt: 96-98 ° C

| | | | | | | | | |
|---|---|---|---|---|---|---|---|---|
| Ber.: | C | 60,42 | H | 7,11 | N | 8,46 | Cl | 14,28 |
| Gef.: | | 60,35 | | 7,46 | | 8,43 | | 14,58 |

Beispiel 6

3-[(N-(3-(Indolyl-3)-propyl)-hexahydro-azepinyl-3)-methyl]-7,8-dimethoxy-1,3,4,5-tetrahydro-2H-3-benzazepin-dihydrochlorid-monohydrat

Hergestellt aus 3-[(N-(3-(Indolyl-3)-propyl)-hexahydro-azepinyl-3)-methyl]-7,8-dimethoxy-2-oxo-1,3,4,5-tetrahydro-2H-3-benzazepin und Lithiumaluminiumhydrid in Tetrahydrofuran und Diethylether analog Beispiel 3.
Ausbeute: 53 % der Theorie,
Schmelzpunkt: 158-160 ° C

| | | | | | | | | |
|---|---|---|---|---|---|---|---|---|
| Ber.: | C | 63,58 | H | 8,00 | N | 7,41 | Cl | 12,51 |
| Gef.: | | 63,41 | | 8,18 | | 7,36 | | 12,23 |

Beispiel 7

2-[(N-(2-(4-Amino-phenyl)-ethyl)-piperidyl-3)-methyl]-5,6-dimethoxy-1-oxo-1,3-dihydro-isoindol-dihydrochlorid

2,1 g (4,78 mMol) 2-[(N-(2-(4-Nitro-phenyl)-ethyl)-piperidyl-3)-methyl]-5,6-dimethoxy-1-oxo-1,3-dihydro-isoindol werden in 50 ml Eisessig gelöst und unter Rühren 0,4 ml (8,22 mMol) Hydrazinhydrat und 1 Spatelspitze Raney-Nickel zugegeben. Die Zugabe von 0,2 ml Hydrazinhydrat und je 1 Spatelspitze Raney-Nickel wird im Abstand von je einer Stunde 3 mal wiederholt. Es wird vom Katalysator abgesaugt, mit Methanol gewaschen, das Filtrat mit Magnesiumsulfat getrocknet, im Vakuum eingeengt und der erhaltene Rückstand über Aluminiumoxid (neutral, Aktivität II) mit Methylenchlorid und anschließend steigenden Anteilen von Ethanol gereinigt.
Ausbeute: 1,8 g (91,8 % der Theorie),
1 g wird in Aceton gelöst und mit etherischer Salzsäure das Dihydrochlorid gefällt.
Ausbeute: 1,02 g (86,4 % der Theorie bezogen auf Base),
Schmelzpunkt: 232-235 °C

| | | | | | | | | |
|---|---|---|---|---|---|---|---|---|
| Ber.:<br>Gef.: | C | 59,72<br>59,54 | H | 6,89<br>7,08 | N | 8,71<br>8,56 | Cl | 14,69<br>14,45 |

Beispiel 8

2-[(N-(2-(4-Acetamino-phenyl)-ethyl)-piperidyl-3)-methyl]-5,6-dimethoxy-1-oxo-1,3-dihydro-isoindol

819 mg (2 mMol) 2-[(N-(2-(4-Amino-phenyl)-ethyl)-piperidyl-3)-methyl]-5,6-dimethoxy-1-oxo-1,3-dihydro-isoindol werden in 10 ml Methylenchlorid und nach der Zugabe von 0,3 ml (2,2 mMol) Triethylamin tropfenweise mit 0,16 ml (2,2 mMol) Acetylchlorid versetzt. Hierbei steigt die Reaktionstemperatur auf 30 °C an. Man rührt 1/2 Stunde bei Raumtemperatur, schüttelt 2 mal mit Wasser aus, trocknet die organische Phase über Magnesiumsulfat und engt im Vakuum ein. Der Rückstand wird aus Aceton kristallisiert.
Ausbeute: 660 mg (73,2 % der Theorie),
Schmelzpunkt: 195-196 °C

| | | | | | | |
|---|---|---|---|---|---|---|
| Ber.:<br>Gef.: | C | 69,16<br>69,33 | H | 7,37<br>7,11 | N | 9,31<br>9,16 |

Beispiel 9

3-[(N-(3-(Furyl-2)-propyl)-piperidyl-3)-methyl]-7,8-dimethoxy-2-oxo-1,3,4,5-tetrahydro-2H-3-benzazepin-hydrochlorid

3,2 g (0,010 Mol 3-[(Piperidyl-3)-methyl]-7,8-dimethoxy-2-oxo-1,3,4,5-tetrahydro-2H-3-benzazepin werden in 100 ml absolutem Ethanol in Gegenwart von 1,3 g (0,010 Mol) 3-(Furyl-2)-propanal und 1 g Raney-Nickel bei 80 °C 2 Tage bei 5 bar hydriert. Man saugt vom Katalysator ab, engt ein und reinigt über eine Kieselgelsäule mit Methylenchlorid/Methanol als Eluens. Das Hydrochlorid wird mit etherischer Salzsäure gefällt und aus Aceton kristallisiert.
Ausbeute: 0,50 g (11 % der Theorie),
Schmelzpunkt: 204-206 °C

| | | | | | | | | |
|---|---|---|---|---|---|---|---|---|
| Ber.:<br>Gef.: | C | 64,85<br>64,88 | H | 7,62<br>7,76 | N | 6,05<br>5,93 | Cl | 7,66<br>7,55 |

Rf-Wert: 0,69 (Kieselgel; Methylenchlorid/Methanol = 10:1; Ammoniak/Atmosphäre)

Beispiel 10

2-[(N-(3-(3-Methylphenoxy)-propyl)-piperidyl-3)-methyl]-6,7-dimethoxy-1,2,3,4-tetrahydro-isochinolin-dihydrochlorid

Hergestellt aus 2-[(N-(3-(3-Methylphenoxy)-propyl)-piperidyl-3)-methyl]-6,7-dimethoxy-1-oxo-1,2,3,4-tetrahydro-isochinolin und Lithiumaluminiumhydrid in Diethylether und Tetrahydrofuran analog Beispiel 3.
Ausbeute: 92,9 % der Theorie,
Schmelzpunkt: 100-103 ° C

| | | | | | | | | |
|---|---|---|---|---|---|---|---|---|
| Ber.:<br>Gef.: | C | 61,23<br>61,21 | H | 7,99<br>8,13 | N | 5,29<br>5,10 | Cl | 13,39<br>13,15 |

Beispiel 11

2-[(N-(3-(Naphthyl-2-oxy)-propyl)-pyrrolidyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und 3-Chlormethyl-N-[3-(naphthyl-2-oxy)-propyl]-pyrrolidin analog Beispiel 2.
Ausbeute: 22 % der Theorie,
Schmelzpunkt: 78-80 ° C

| | | | | | | | | |
|---|---|---|---|---|---|---|---|---|
| Ber. (x $H_2O$):<br>Gef.: | C | 65,83<br>65,79 | H | 7,04<br>7,00 | N | 5,29<br>5,03 | Cl | 6,70<br>6,99 |

Beispiel 12

2-[(N-(3-(Naphthyl-2-oxy)-propyl)-pyrrolidyl-3)-methyl]-6,7-methylendioxy-1-oxo-1,2,3,4-tetrahydro-isochinolin-hydrochlorid

Hergestellt aus 6,7-Methylendioxy-1-oxo-1,2,3,4-tetrahydro-isochinolin und 3-Chlormethyl-N-[3-(naphthyl-2-oxy)-propyl]-pyrrolidin analog Beispiel 2.
Ausbeute: 53 % der Theorie,
Schmelzpunkt: 78-80 ° C

| | | | | | | | | |
|---|---|---|---|---|---|---|---|---|
| Ber. (x $H_2O$):<br>Gef.: | C | 65,56<br>65,44 | H | 6,48<br>6,32 | N | 5,46<br>5,38 | Cl | 6,91<br>7,13 |

Beispiel 13

2-[(N-(2-(Naphthyl-2)-ethyl)-pyrrolidyl-3)-methyl]-6,7-dimethoxy-1,2,3,4-tetrahydro-isochinolin-hydrochlorid

Hergestellt aus 2-[(N-(2-(Naphthyl-2)-ethyl)-pyrrolidyl-3)-methyl]-6,7-dimethoxy-1-oxo-1,2,3,4-tetrahydro-isochinolin und Lithiumaluminiumhydrid in Tetrahydrofuran und Ether analog Beispiel 3.
Ausbeute: 66,2 % der Theorie,
Schmelzpunkt: 239-241 ° C

| | | | | | | | | |
|---|---|---|---|---|---|---|---|---|
| Ber. (x $H_2O$):<br>Gef.: | C | 64,48<br>64,30 | H | 7,44<br>7,34 | N | 5,37<br>5,52 | Cl | 13,91<br>13,69 |

Beispiel 14

2-[(N-((2-Methyl-naphthyl-1)-methyl)-hexahydro-azepinyl-3)-methyl]-6,7-dimethoxy-1,2,3,4-tetrahydro-isochinolin-hydrochlorid

Hergestellt aus 2-[(N-((2-Methyl-naphthyl-1)-methyl)-hexahydro-azepinyl-3)-methyl]-6,7-dimethoxy-1-oxo-1,2,3,4-tetrahydro-isochinolin und Lithiumaluminiumhydrid in Tetrahydrofuran und Ether analog Beispiel 3.
Ausbeute: 73,8 % der Theorie,
Schmelzpunkt: 182-184 ° C

| | | | | | | | | |
|---|---|---|---|---|---|---|---|---|
| Ber. (x $H_2O$):<br>Gef.: | C | 65,56<br>65,52 | H | 7,70<br>7,57 | N | 5,09<br>5,32 | Cl | 12,90<br>12,72 |

Beispiel 15

2-[(N-(4-(Naphthyl-2-oxy)-butyl)-pyrrolidyl-3)-methyl]-6,7-dimethyl-1-oxo-1,2,3,4-tetrahydro-isochinolin-hydrochlorid

Hergestellt aus 2-(Pyrrolidyl-3-methyl)-6,7-dimethyl-1-oxo-1,2,3,4-tetrahydro-isochinolin und 2-(4-Brom-butyloxy)-naphthalin analog Beispiel 1.
Ausbeute: 30 % der Theorie,
Schmelzpunkt: 133-136 ° C

| | | | | | | | | |
|---|---|---|---|---|---|---|---|---|
| Ber.:<br>Gef.: | C | 67,02<br>67,26 | H | 6,92<br>7,03 | N | 5,21<br>5,36 | Cl | 14,86<br>14,89 |

Beispiel 16

2-[(N-(2-Methyl-naphthyl-1)-methyl)-pyrrolidyl-3)-methyl]-6,7-dimethyl-1-oxo-1,2,3,4-tetrahydro-isochinolin-hydrochlorid

Hergestellt aus 2-(Pyrrolidyl-3-methyl)-6,7-dimethyl-1-oxo-1,2,3,4-tetrahydro-isochinolin und 1-Chlormethyl-2-methyl-naphthalin analog Beispiel 1.
Ausbeute: 32,5 % der Theorie,
Schmelzpunkt: 142-144 ° C

| | | | | | | | | |
|---|---|---|---|---|---|---|---|---|
| Ber.:<br>Gef.: | C | 69,34<br>69,59 | H | 7,69<br>7,63 | N | 5,77<br>5,72 | Cl | 7,37<br>7,89 |

Beispiel 17

2-[(N-(2-(5-Methyl-6-methoxy-naphthyl-2)-ethyl)-pyrrolidyl-3)-methyl]-6,7-dimethyl-1-oxo-1,2,3,4-tetrahydro-isochinolin-hydrobromid

Hergestellt aus 2-[N-(Pyrrolidyl-3-methyl)]-6,7-dimethyl-1-oxo-1,2,3,4-tetrahydro-isochinolin und 2-(2-Bromethyl)-5-methyl-6-methoxy-naphthalin analog Beispiel 1.
Ausbeute: 19 % der Theorie,
Schmelzpunkt: 230-232 ° C

| | | | | | | | | |
|---|---|---|---|---|---|---|---|---|
| Ber.:<br>Gef.: | C | 67,02<br>67,10 | H | 6,93<br>7,12 | N | 5,21<br>5,33 | Br | 14,86<br>15,01 |

Beispiel 18

2-[(N-(2-(Naphthyl-2)-ethyl)-pyrrolidyl-3)-methyl]-6,7-dimethoxy-1-oxo-1,2,3,4-tetrahydro-isochinolin-hydrochlorid

Hergestellt aus 2-(Pyrrolidyl-3-methyl)-6,7-dimethoxy-1-oxo-1,2,3,4-tetrahydro-isochinolin und 2-(2-Bromethyl)-naphthalin analog Beispiel 1.
Ausbeute: 7,8 % der Theorie,
Schmelzpunkt: 219-221 °C

| | | | | | | | | |
|---|---|---|---|---|---|---|---|---|
| Ber. (x $H_2O$):<br>Gef.: | C | 67,39<br>67,21 | H | 7,07<br>7,23 | N | 5,61<br>5,57 | Cl | 7,10<br>7,63 |

Beispiel 19

2-[(N-(2-(6-Methoxy-naphthyl-2)-ethyl)-pyrrolidyl-3)-methyl]-6,7-dimethoxy-1-oxo-1,2,3,4-tetrahydro-isochinolin-hydrochlorid

Hergestellt aus 2-((Pyrrolidyl-3)-methyl)-6,7-dimethoxy-1-oxo-1,2,3,4-tetrahydro-isochinolin und 2-(2-Bromethyl)-6-methoxy-naphthalin analog Beispiel 1.
Ausbeute: 39,2 % der Theorie,
Schmelzpunkt: 224-226 °C

| | | | | | | | | |
|---|---|---|---|---|---|---|---|---|
| Ber. (x $H_2O$):<br>Gef.: | C | 65,84<br>66,08 | H | 7,05<br>7,13 | N | 5,29<br>5,39 | Cl | 7,05<br>6,77 |

Beispiel 20

2-[(N-(3-(Naphthyl-2)-propyl)-piperidyl-3)-methyl]-6,7-dimethyl-1-oxo-1,2,3,4-tetrahydro-isochinolin-hydrochlorid

Hergestellt aus 6,7-Dimethyl-1-oxo-1,2,3,4-tetrahydro-isochinolin und 3-Chlormethyl-N-[3-(naphthyl-2)-propyl]-piperidin analog Beispiel 2.
Ausbeute: 40,4 % der Theorie,
Schmelzpunkt: 185-187 °C

| | | | | | | | | |
|---|---|---|---|---|---|---|---|---|
| Ber.:<br>Gef.: | C | 75,52<br>75,39 | H | 7,82<br>7,85 | N | 5,87<br>5,82 | Cl | 7,43<br>7,52 |

Beispiel 21

2-[2-(N-(3-(Naphthyl-2-oxy)-propyl)-piperidyl-2)-ethyl]-6,7-methylendioxy-1-oxo-1,2,3,4-tetrahydro-isochinolin-hydrochlorid

Hergestellt aus 2-((Piperidyl-2)-ethyl)-6,7-methylendioxy-1-oxo-1,2,3,4-tetrahydro-isochinolin und 2-(3-Chlorpropoxy)-naphthalin analog Beispiel 1.
Ausbeute: 21,2 % der Theorie,

Schmelzpunkt: 85-87 ° C

| | | | | | | | | |
|---|---|---|---|---|---|---|---|---|
| Ber. (x $H_2O$):<br>Gef.: | C | 66,59<br>66,77 | H | 6,89<br>6,98 | N | 5,17<br>4,95 | Cl | 6,53<br>6,74 |

Beispiel 22

2-[(N-(3-(Naphthyl-2-oxy)-propyl)-piperidyl-3)-methyl]-6,7-dimethyl-1,2,3,4-tetrahydro-isochinolin-dihydrochlorid

Hergestellt aus 2-[(N-(3-(Naphthyl-2-oxy)-propyl)-piperidyl-3)-methyl]-6,7-dimethyl-1-oxo-1,2,3,4-tetrahydro-isochinolin und Lithiumaluminiumhydrid in Tetrahydrofuran/Ether analog Beispiel 3.
Ausbeute: 53 % der Theorie,
Schmelzpunkt: 133-135 ° C

| | | | | | | | | |
|---|---|---|---|---|---|---|---|---|
| Ber. (x $H_2O$):<br>Gef.: | C | 68,30<br>68,05 | H | 6,87<br>6,85 | N | 5,31<br>5,23 | Cl | 13,44<br>13,03 |

Beispiel 23

2-[(N-(3-(Naphthyl-2-oxy)-propyl)-piperidyl-3)-methyl]-6,7-methylendioxy-1,2,3,4-tetrahydro-isochinolin-dihydrochlorid

Hergestellt aus 2-[(N-(3-(Naphthyl-2-oxy)-propyl)-piperidyl-3)-methyl]-6,7-methylendioxy-1-oxo-1,2,3,4-tetrahydro-isochinolin und Lithiumaluminiumhydrid in Tetrahydrofuran/Ether analog Beispiel 3.
Ausbeute: 44,7 % der Theorie,
Schmelzpunkt: 130-132 ° C

| | | | | | | | | |
|---|---|---|---|---|---|---|---|---|
| Ber. (x $H_2O$):<br>Gef.: | C | 63,62<br>63,49 | H | 6,62<br>6,86 | N | 5,11<br>4,97 | Cl | 12,95<br>12,64 |

Beispiel 24

2-[(N-((2-Methyl-naphthyl-1)-methyl)-piperidyl-3)-methyl]-6,7-dimethoxy-1,2,3,4-tetrahydro-isochinolin-dihydrochlorid

Hergestellt aus 2-[(N-((2-Methyl-naphthyl-1)-methyl)-piperidyl-3)-methyl]-6,7-dimethoxy-1-oxo-1,2,3,4-tetrahydro-isochinolin und Lithiumaluminiumhydrid in Tetrahydrofuran/Ether analog Beispiel 3.
Ausbeute: 80,5 % der Theorie,
Schmelzpunkt: 210-212 ° C

| | | | | | | |
|---|---|---|---|---|---|---|
| Ber. (x $H_2O$):<br>Gef.: | C | 65,05<br>65,23 | H | 7,53<br>7,78 | N | 5,23<br>5,03 |

Beispiel 25

2-[2-(N-(2-(6-Methoxy-naphthyl-2)-ethyl)-piperidyl-2)-ethyl]-6,7-methylendioxy-1-oxo-1,2,3,4-tetrahydro-isochinolin-hydrochlorid

Hergestellt aus 2-[2-(Piperidyl-2)-ethyl)-6,7-methylendioxy-1-oxo-1,2,3,4-tetrahydro-isochinolin und 2-(2-Bromethyl)-6-methoxy-naphthalin analog Beispiel 1.
Ausbeute: 27,6 % der Theorie,
Schmelzpunkt: 112-117 ° C

| | | | | | | | | |
|---|---|---|---|---|---|---|---|---|
| Ber. (x 1/2 $H_2O$):<br>Gef.: | C | 67,74<br>67,54 | H | 6,82<br>6,73 | N | 5,26<br>5,47 | Cl | 6,65<br>6,86 |

Beispiel 26

2-[(N-(2-(Naphthyl-1)-ethyl)-piperidyl-3)-methyl]-6,7-dimethoxy-1-oxo-1,2,3,4-tetrahydro-isochinolin-hydrochlorid

Hergestellt aus 2-[(Piperidyl-3)-methyl]-6,7-dimethoxy-1-oxo-1,2,3,4-tetrahydro-isochinolin und Benzolsulfonsäure-2-ethyl-(naphthyl-1)-ester analog Beispiel 1.
Ausbeute: 26,9 % der Theorie,
Schmelzpunkt: 220-225 ° C

| | | | | | | | | |
|---|---|---|---|---|---|---|---|---|
| Ber.:<br>Gef.: | C | 67,89<br>67,75 | H | 7,27<br>6,92 | N | 5,46<br>5,56 | Cl | 6,91<br>7,00 |

Beispiel 27

2-[(N-(2-(Naphthyl-2)-ethyl)-piperidyl-3)-methyl]-6,7-methylendioxy-1-oxo-1,2,3,4-tetrahydro-isochinolin-hydrochlorid

Hergestellt aus 2-[(Piperidyl-3)-methyl]-6,7-methylendioxy-1-oxo-1,2,3,4-tetrahydro-isochinolin und Benzolsulfonsäure-2-ethyl-(naphthyl-2)-ester analog Beispiel 1.
Ausbeute: 27,9 % der Theorie,
Schmelzpunkt: 128-130 ° C

| | | | | | | | | |
|---|---|---|---|---|---|---|---|---|
| Ber. (x $H_2O$):<br>Gef.: | C | 67,66<br>67,64 | H | 6,69<br>6,70 | N | 5,63<br>5,76 | Cl | 7,13<br>7,35 |

Beispiel 28

2-[(N-(2-(5-Methyl-6-methoxy-naphthyl-2)-ethyl)-piperidyl-3)-methyl]-6,7-methylendioxy-1-oxo-1,2,3,4-tetrahydro-isochinolin-hydrochlorid

Hergestellt aus 2-[(Piperidyl-3)-methyl]-6,7-methylendioxy-1-oxo-1,2,3,4-tetrahydro-isochinolin und 2-(2-Bromethyl)-5-methyl-6-methoxy-naphthalin analog Beispiel 1.
Ausbeute: 51,2 % der Theorie,
Schmelzpunkt: 128-131 ° C

| | | | | | | | | |
|---|---|---|---|---|---|---|---|---|
| Ber.:<br>Gef.: | C | 68,88<br>68,90 | H | 6,74<br>6,61 | N | 5,34<br>5,30 | Cl | 6,77<br>7,05 |

Beispiel 29

2-[(N-((2-Methyl-naphthyl-1)-methyl)-piperidyl-3)-methyl]-6,7-dimethoxy-1-oxo-1,2,3,4-tetrahydro-isochinolin-hydrochlorid

Hergestellt aus 2-[(Piperidyl-3)-methyl]-6,7-dimethoxy-1-oxo-1,2,3,4-tetrahydro-isochinolin und 1-Chlormethyl-2-methyl-naphthalin analog Beispiel 1.
Ausbeute: 57,9 % der Theorie,
Schmelzpunkt: 212-214 ° C

| | | | | | | | | |
|---|---|---|---|---|---|---|---|---|
| Ber. (x 2 $H_2O$):<br>Gef.: | C | 67,63<br>67,46 | H | 7,63<br>7,56 | N | 5,44<br>5,54 | Cl | 6,88<br>6,67 |

Beispiel 30

2-[(N-(4-(Naphthyl-2-oxy)-butyl)-piperidyl-3)-methyl]-6,7-dimethoxy-1-oxo-1,2,3,4-tetrahydro-isochinolin-hydrochlorid

Hergestellt aus 2-[(Piperidyl-3)-methyl]-6,7-dimethoxy-1-oxo-1,2,3,4-tetrahydro-isochinolin und 2-(4-Brom-butoxy)-naphthalin analog Beispiel 1.
Ausbeute: 46,3 % der Theorie,
Schmelzpunkt: 80-84 ° C

| | | | | | | | | |
|---|---|---|---|---|---|---|---|---|
| Ber. (x $H_2O$):<br>Gef.: | C | 66,83<br>66,79 | H | 7,41<br>7,22 | N | 5,03<br>4,90 | Cl | 6,36<br>6,64 |

Beispiel 31

2-[(N-(2-(6-Methoxy-naphthyl-2)-ethyl)-piperidyl-3)-methyl]-6,7-dimethoxy-1-oxo-1,2,3,4-tetrahydro-isochinolin-hydrochlorid

Hergestellt aus 2-[(Piperidyl-3)-methyl]-6,7-dimethoxy-1-oxo-1,2,3,4-tetrahydro-isochinolin und 2-(2-Brom-ethyl)-6-methoxy-naphthalin analog Beispiel 1.
Ausbeute: 22,8 % der Theorie,
Schmelzpunkt: 80-85 ° C

| | | | | | | | | |
|---|---|---|---|---|---|---|---|---|
| Ber.: (x $H_2O$ x HCl x $CH_3COCH_3$):<br>Gef.: | C | 64,01<br>64,26 | H | 7,65<br>7,70 | N | 4,52<br>4,62 | Cl | 5,72<br>5,49 |

Beispiel 32

2-[3-(N-(2-(6-Methoxy-naphthyl-2)-ethyl)-piperidyl-3)-propyl]-6,7-methylendioxy-1-oxo-1,2,3,4-tetrahydro-isochinolin

Hergestellt aus 2-[3-(Piperidyl-3)-propyl]-6,7-methylendioxy-1-oxo-1,2,3,4-tetrahydro-isochinolin und 2-(2-Bromethyl)-6-methoxy-naphthalin analog Beispiel 1.
Ausbeute: 36,8 % der Theorie,

Schmelzpunkt: 118-121 °C

| | | | | | | |
|---|---|---|---|---|---|---|
| Ber.:<br>Gef.: | C | 74,37<br>74,60 | H | 7,25<br>7,43 | N | 5,60<br>5,65 |

Beispiel 33

2-[3-(N-(2-(Naphthyl-1)-ethyl)-piperidyl-3)-propyl]-6,7-methylendioxy-1-oxo-1,2,3,4-tetrahydro-isochinolin-hydrochlorid

Hergestellt aus 2-[3-(Piperidyl-3)-propyl]-6,7-methylendioxy-1-oxo-1,2,3,4-tetrahydro-isochinolin und Benzolsulfonsäure-2-ethyl-(naphthyl-1)-ester analog Beispiel 1.
Ausbeute: 20,8 % der Theorie,
Schmelzpunkt: 195-197 °C

| | | | | | | | | |
|---|---|---|---|---|---|---|---|---|
| Ber.:<br>Gef.: | C | 71,07<br>71,30 | H | 6,95<br>6,95 | N | 5,53<br>5,65 | Cl | 6,99<br>6,80 |

Beispiel 34

2-[2-(N-(2-(5-Methyl-6-methoxy-naphthyl-2)-ethyl)-piperidyl-2)-ethyl]-6,7-dimethoxy-1-oxo-1,2,3,4-tetrahydro-isochinolin-hydrochlorid

Hergestellt aus 2-[2-(Piperidyl-2)-ethyl]-6,7-dimethoxy-1-oxo-1,2,3,4-tetrahydro-isochinolin und 2-(2-Brom-ethyl)-5-methyl-6-methoxy-naphthalin analog Beispiel 1.
Ausbeute: 33,6 % der Theorie,
Schmelzpunkt: 95-100 °C

| | | | | | | | | |
|---|---|---|---|---|---|---|---|---|
| Ber. (x 1/2 $H_2O$):<br>Gef.: | C | 68,38<br>68,14 | H | 7,52<br>7,43 | N | 4,98<br>4,92 | Cl | 6,30<br>6,77 |

Beispiel 35

2-[(N-(3-(Naphthyl-2-oxy)-propyl)-piperidyl-3)-methyl]-6,7-methylendioxy-1-oxo-1,2,3,4-tetrahydro-isochinolin-hydrochlorid

Hergestellt aus 6,7-Methylendioxy-1-oxo-1,2,3,4-tetrahydro-isochinolin und 3-Chlormethyl-N-[3-(naphthyl-2-oxy)-propyl]-piperidin analog Beispiel 2.
Ausbeute: 27,3 % der Theorie,
Schmelzpunkt: 104-106 °C

| | | | | | | | | |
|---|---|---|---|---|---|---|---|---|
| Ber. (x $H_2O$):<br>Gef.: | C | 66,09<br>66,19 | H | 6,69<br>6,34 | N | 5,31<br>5,24 | Cl | 6,72<br>7,22 |

Beispiel 36

2-[(N-(3-(Naphthyl-2-oxy)-propyl)-piperidyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und 3-Chlormethyl-N-[3-(naphthyl-2-oxy)-propyl]-piperidin analog Beispiel 2.
Ausbeute: 28,6 % der Theorie,
Schmelzpunkt: 191-193 ° C

| | | | | | | | | |
|---|---|---|---|---|---|---|---|---|
| Ber. (x $H_2O$):<br>Gef.: | C | 66,34<br>66,59 | H | 7,23<br>7,19 | N | 5,15<br>5,03 | Cl | 6,53<br>6,65 |

Beispiel 37

2-[(N-(3-(Naphthyl-2-oxy)-propyl)-hexahydro-azepinyl-3)-methyl]-6,7-dimethoxy-1-oxo-1,2,3,4-tetrahydro-isochinolin-hydrochlorid

Hergestellt aus 2-[(Hexahydro-azepinyl-3)-methyl]-6,7-dimethoxy-1-oxo-1,2,3,4-tetrahydro-isochinolin und 2-(3-Chlorpropoxy)-napthalin analog Beispiel 1.
Ausbeute: 16,1 % der Theorie,
Schmelzpunkt: 86-88 ° C

| | | | | | | | | |
|---|---|---|---|---|---|---|---|---|
| Ber. (x $H_2O$):<br>Gef.: | C | 66,83<br>66,90 | H | 7,42<br>7,40 | N | 5,03<br>5,26 | Cl | 6,36<br>6,87 |

Beispiel 38

2-[(N-(3-(Naphthyl-2-oxy)-propyl)-hexahydro-azepinyl-3)-methyl]-6,7-dimethyl-1-oxo-1,2,3,4-tetrahydro-isochinolin-hydrochlorid

Hergestellt aus 6,7-Dimethyl-1-oxo-1,2,3,4-tetrahydro-isochinolin und 3-Chlormethyl-N-[3-(naphthyl-2-oxy)-propyl]-hexahydro-azepin analog Beispiel 2.
Ausbeute: 22,5 % der Theorie,
Schmelzpunkt: 191-193 ° C

| | | | | | | | | |
|---|---|---|---|---|---|---|---|---|
| Ber.:<br>Gef.: | C | 73,42<br>73,37 | H | 7,75<br>7,67 | N | 5,52<br>5,52 | Cl | 6,99<br>7,12 |

Beispiel 39

2-[(N-(2-(5-Methyl-6-methoxy-naphthyl-2)-ethyl)-hexahydroazepinyl-3)-methyl]-6,7-dimethoxy-1,2,3,4-tetrahydro-isochinolin-dihydrochlorid

Hergestellt aus 2-[(N-(2-(5-Methyl-6-methoxy-naphthyl-2)-ethyl)-hexahydro-azepinyl-3)-methyl]-6,7-dimethoxy-1-oxo-1,2,3,4-tetrahydro-isochinolin und Lithiumaluminiumhydrid in Tetrahydrofuran analog Beispiel 3.
Ausbeute: 77,6 % der Theorie,
Schmelzpunkt: 170-172 ° C

| | | | | | | | | |
|---|---|---|---|---|---|---|---|---|
| Ber. (x $H_2O$):<br>Gef.: | C | 64,96<br>65,11 | H | 7,49<br>7,62 | N | 4,73<br>4,95 | Cl | 11,98<br>11,84 |

Beispiel 40

2-[(N-(2-(5-Methyl-6-methoxy-naphthyl-2)-ethyl)-hexahydro-azepinyl-3)-methyl]-6,7-dimethoxy-1-oxo-1,2,3,4-tetrahydro-isochinolin

Hergestellt aus 2-[(Hexahydro-azepinyl-3)-methyl]-6,7-dimethoxy-1-oxo-1,2,3,4-tetrahydro-isochinolin und 2-(2-Bromethyl)-5-methyl-6-methoxy-naphthalin analog Beispiel 1.
Ausbeute: 43,5 % der Theorie,
Schmelzpunkt: 125-127 ° C

| | | | | | | |
|---|---|---|---|---|---|---|
| Ber.:<br>Gef.: | C | 74,39<br>74,31 | H | 7,80<br>7,82 | N | 5,42<br>5,35 |

Beispiel 41

2-[(N-(2-Methyl-naphthyl-1)-methyl)-hexahydro-azepinyl-3)-methyl]-6,7-dimethoxy-1-oxo-1,2,3,4-tetrahydro-isochinolin-hydrochlorid

Hergestellt aus 2-[(Hexahydro-azepinyl-3)-methyl]-6,7-dimethoxy-1-oxo-1,2,3,4-tetrahydro-isochinolin und 1-Chlormethyl-2-methyl-naphthalin analog Beispiel 1.
Ausbeute: 67,5 % der Theorie,
Schmelzpunkt: 128-130 ° C

| | | | | | | | | |
|---|---|---|---|---|---|---|---|---|
| Ber. (x 2 $H_2O$):<br>Gef.: | C | 66,10<br>66,24 | H | 7,58<br>7,44 | N | 5,13<br>5,23 | Cl | 6,50<br>6,85 |

Beispiel 42

2-[(N-(4-(Naphthyl-2-oxy)-butyl)-hexahydro-azepinyl-3)-methyl]-6,7-dimethoxy-1-oxo-1,2,3,4-tetrahydro-isochinolin-hydrochlorid

Hergestellt aus 2-[(Hexahydro-azepinyl-3)-methyl]-6,7-dimethoxy-1-oxo-1,2,3,4-tetrahydro-isochinolin und 2-(4-Brom-butyloxy)-naphthalin analog Beispiel 1.
Ausbeute: 26 % der Theorie,
Schmelzpunkt: 192-194 ° C

| | | | | | | | | |
|---|---|---|---|---|---|---|---|---|
| Ber.:<br>Gef.: | C | 69,22<br>70,01 | H | 7,80<br>7,70 | N | 5,04<br>5,15 | Cl | 6,38<br>6,48 |

Beispiel 43

2-[(N-(2-(Naphthyl-1)-ethyl)-hexahydro-azepinyl-3)-methyl]-6,7-methylendioxy-1-oxo-1,2,3,4-tetrahydro-isochinolin-hydrochlorid

Hergestellt aus 2-[(Hexahydro-azepinyl-3)-methyl]-6,7-methylendioxy-1-oxo-1,2,3,4-tetrahydro-isochinolin und 2-(2-Benzolsulfonyloxy-ethyl)-naphthalin analog Beispiel 1.
Ausbeute: 15,4 % der Theorie,

Schmelzpunkt: 236-238 °C

| | | | | | | | | |
|---|---|---|---|---|---|---|---|---|
| Ber. (x 1/2 $H_2O$):<br>Gef.: | C | 69,40<br>69,07 | H | 6,82<br>6,74 | N | 5,58<br>6,13 | Cl | 7,06<br>7,29 |

Beispiel 44

2-[(N-(2-(Naphthyl-2)-ethyl)-hexahydro-azepinyl-3)-methyl]-6,7-methylendioxy-1-oxo-1,2,3,4-tetrahydro-isochinolin-hydrobromid

Hergestellt aus 2-[(Hexahydro-azepinyl-3)-methyl]-6,7-methylendioxy-1-oxo-1,2,3,4-tetrahydro-isochinolin und 2-(2-Bromethyl]-naphthalin analog Beispiel 1.
Schmelzpunkt: 100-102 °C
Ausbeute: 31,6 % der Theorie,

| | | | | | | | | |
|---|---|---|---|---|---|---|---|---|
| Ber.:<br>Gef.: | C | 64,80<br>65,02 | H | 6,18<br>6,07 | N | 5,21<br>5,39 | Br | 14,86<br>14,78 |

Beispiel 45

2-[(N-(2-(6-Methoxy-naphthyl-2)-ethyl)-hexahydro-azepinyl-3)-methyl]-6,7-methylendioxy-1-oxo-1,2,3,4-tetrahydro-isochinolin-hydrochlorid

Hergestellt aus 2-[(Hexahydro-azepinyl-3)-methyl]-6,7-methylendioxy-1-oxo-1,2,3,4-tetrahydro-isochinolin und 2-(2-Bromethyl]-6-methoxy-naphthalin analog Beispiel 1.
Ausbeute: 34,1 % der Theorie,
Schmelzpunkt: 147-149 °C

| | | | | | | | | |
|---|---|---|---|---|---|---|---|---|
| Ber. (x $H_2O$):<br>Gef.: | C | 68,62<br>68,88 | H | 7,10<br>6,98 | N | 5,33<br>5,41 | Cl | 6,75<br>6,78 |

Beispiel 46

2-[(N-(2-(5-Methyl-6-methoxy-naphthyl-2)-ethyl)-hexahydro-azepinyl-3)-methyl]-6,7-methylendioxy-1-oxo-1,2,3,4-tetrahydro-isochinolin-hydrochlorid

Hergestellt aus 2-[(Hexahydro-azepinyl-3)-methyl]-6,7-methylendioxy-1-oxo-1,2,3,4-tetrahydro-isochinolin und 2-(2-Bromethyl]-5-methyl-6-methoxy-naphthalin analog Beispiel 1.
Ausbeute: 36,1 % der Theorie,
Schmelzpunkt: 112-114 °C

| | | | | | | | | |
|---|---|---|---|---|---|---|---|---|
| Ber. (x $H_2O$):<br>Gef.: | C | 67,07<br>67,13 | H | 7,08<br>7,15 | N | 5,04<br>4,97 | Cl | 6,38<br>6,56 |

Beispiel 47

2-[(N-(3-(4-Methoxy-phenoxy)-propyl)-piperidyl-3)-methyl]-6,7-dimethoxy-1,2,3,4-tetrahydro-isochinolin-dihydrochlorid

Hergestellt aus 2-[(N-(3-(4-Methoxy-phenoxy)-propyl)-piperidyl-3)-methyl]-6,7-dimethoxy-1-oxo-1,2,3,4-tetrahydro-iso chinolin und Lithiumaluminiumhydrid in Tetrahydrofuran analog Beispiel 3.
Ausbeute: 88,5 % der Theorie,
Schmelzpunkt: 189-191 ° C

| | | | | | | | | |
|---|---|---|---|---|---|---|---|---|
| Ber.:<br>Gef.: | C | 59,44<br>59,55 | H | 7,76<br>7,99 | N | 5,13<br>5,12 | Cl | 12,99<br>12,61 |

Beispiel 48

2-[(N-(2-(3,4-Dimethoxy-phenyl)-ethyl)-piperidyl-3)-methyl]-6,7-dimethoxy-1,2,3,4-tetrahydro-isochinolin-dihydrochlorid

Hergestellt aus 2-[(N-(2-(3,4-Dimethoxy-phenyl)-ethyl)-piperidyl-3)-methyl]-6,7-dimethoxy-1-oxo-1,2,3,4-tetrahydro-isochinolin und Lithiumaluminiumhydrid in Tetrahydrofuran analog Beispiel 3.
Ausbeute: 92,8 % der Theorie,
Schmelzpunkt: 175-176 ° C

| | | | | | | | | |
|---|---|---|---|---|---|---|---|---|
| Ber.:<br>Gef.: | C | 60,66<br>60,58 | H | 7,35<br>7,56 | N | 5,33<br>5,32 | Cl | 13,49<br>13,22 |

Beispiel 49

3-[(N-(3-(Indolyl-3)-propyl)-pyrrolidyl-3)-methyl]-7,8-methylendioxy-1,3,4,5-tetrahydro-2H-3-benzazepin-dihydrochlorid-1,5 x hydrat

Hergestellt aus 7,8-Methylendioxy-1,3,4,5-tetrahydro-2H-3-benzazepin und 3-(Benzolsulfonyloxy-methyl)-N-[3-(indolyl-3)-propyl]-pyrrolidin analog Beispiel 2.
Ausbeute: 75 % der Theorie,
Schmelzpunkt: 191-193 ° C

| | | | | | | | | |
|---|---|---|---|---|---|---|---|---|
| Ber.:<br>Gef.: | C | 61,01<br>60,90 | H | 7,21<br>7,27 | N | 7,90<br>7,85 | Cl | 13,34<br>13,70 |

Beispiel 50

2-[(N-(3-(3-Methoxy-phenoxy)-propyl)-piperidyl-3)-methyl]-6,7-methylendioxy-1,2,3,4-tetrahydro-isochinolin-dihydrochlorid

Hergestellt aus 2-[(N-(3-(3-Methoxy-phenoxy)-propyl)-piperidyl-3)-methyl]-6,7-methylendioxy-1-oxo-1,2,3,4-tetrahydro-isochinolin und Lithiumaluminiumhydrid in Tetrahydrofuran analog Beispiel 3.
Ausbeute: 94,5 % der Theorie,
Schmelzpunkt: 169-171 ° C

| | | | | | | | | |
|---|---|---|---|---|---|---|---|---|
| Ber.:<br>Gef.: | C | 57,03<br>56,91 | H | 7,36<br>7,26 | N | 5,11<br>5,15 | Cl | 13,86<br>13,68 |

Beispiel 51

2-[(N-(2-(3,4-Dimethoxy-phenyl)-ethyl)-piperidyl-3)-methyl]-6,7-dimethyl-1,2,3,4-tetrahydro-isochinolin-dihydrochlorid

Hergestellt aus 2-[(N-(2-(3,4-Dimethoxy-phenyl)-ethyl)-piperidyl-3)-methyl]-6,7-dimethyl-1-oxo-1,2,3,4-tetrahydro-isochinolin und Lithiumaluminiumhydrid in Tetrahydrofuran analog Beispiel 3.
Ausbeute: 91,3 % der Theorie,
Schmelzpunkt: 140-142 °C

| | | | | | | | | |
|---|---|---|---|---|---|---|---|---|
| Ber.:<br>Gef.: | C | 62,00<br>61,85 | H | 8,34<br>8,27 | N | 5,27<br>5,31 | Cl | 13,44<br>13,33 |

Beispiel 52

2-[(N-(3-(4-Methoxy-phenoxy)-propyl)-piperidyl-3)-methyl]-6,7-dimethyl-1,2,3,4-tetrahydro-isochinolin-dihydrochlorid

Hergestellt aus 2-[(N-(3-(4-Methoxy-phenoxy)-propyl)-piperidyl-3)-methyl]-6,7-dimethyl-1-oxo-1,2,3,4-tetrahydro-isochinolin und Lithiumaluminiumhydrid in Tetrahydrofuran analog Beispiel 3.
Ausbeute: 88,3 % der Theorie,
Schmelzpunkt: 170-172 °C

| | | | | | | | | |
|---|---|---|---|---|---|---|---|---|
| Ber.:<br>Gef.: | C | 63,14<br>63,09 | H | 8,24<br>8,33 | N | 5,65<br>5,82 | Cl | 14,31<br>14,02 |

Beispiel 53

2-[(N-(2-(3,4-Dimethoxy-phenyl)-ethyl)-piperidyl-3)-methyl]-6,7-methylendioxy-1,2,3,4-tetrahydro-isochinolin-dihydrochlorid

Hergestellt aus 2-[(N-(3-(3,4-Dimethoxy-phenyl)-ethyl)-piperidyl-3)-methyl]-6,7-methylendioxy-1-oxo-1,2,3,4-tetrahydro-isochinolin und Lithiumaluminiumhydrid in Tetrahydrofuran analog Beispiel 3.
Ausbeute: 93,3 % der Theorie,
Schmelzpunkt: 150-154 °C

| | | | | | | | | |
|---|---|---|---|---|---|---|---|---|
| Ber.:<br>Gef.: | C | 60,01<br>59,96 | H | 7,34<br>7,41 | N | 5,39<br>5,25 | Cl | 13,64<br>13,43 |

Beispiel 54

2-[(N-(3-(4-Methoxy-phenoxy)-propyl)-piperidyl-3)-methyl]-6,7-dimethoxy-1,2,3,4-tetrahydro-isochinolin-dihydrochlorid

Hergestellt aus 2-[(N-(3-(4-Methoxy-phenoxy)-propyl)-piperidyl-3)-methyl]-6,7-dimethoxy-1-oxo-1,2,3,4-tetrahydro-isochinolin und Lithiumaluminiumhydrid in Tetrahydrofuran analog Beispiel 3.
Ausbeute: 95,3 % der Theorie,

Schmelzpunkt: 182-185 °C

| | | | | | | | | |
|---|---|---|---|---|---|---|---|---|
| Ber.:<br>Gef.: | C | 61,05<br>61,10 | H | 7,09<br>6,95 | N | 5,48<br>5,68 | Cl | 13,86<br>13,55 |

Beispiel 55

2-[2-(N-(3-(3,4-Methylendioxy-phenoxy)-propyl)-piperidyl-3)-ethyl]-6,7-dimethoxy-1-oxo-1,2,3,4-tetrahydro-isochinolin-hydrochlorid

Hergestellt aus 2-[(Piperidyl-3)-ethyl]-6,7-dimethoxy-1-oxo-1,2,3,4-tetrahydro-isochinolin und 1-Chlor-3-(3,4-methylendioxy-phenoxy)-propan analog Beispiel 1.
Ausbeute: 35,5 % der Theorie,
Schmelzpunkt: 97-100 °C

| | | | | | | | | |
|---|---|---|---|---|---|---|---|---|
| Ber.:<br>Gef.: | C | 59,09<br>58,97 | H | 7,28<br>7,36 | N | 4,62<br>4,66 | Cl | 6,65<br>6,52 |

Beispiel 56

2-[2-(N-(2-(3,4-Dimethoxy-phenyl)-ethyl)-piperidyl-3)-ethyl]-6,7-dimethoxy-1-oxo-1,2,3,4-tetrahydro-isochinolin-hydrochlorid

Hergestellt aus 2-[(Piperidyl-3)-ethyl]-6,7-dimethoxy-1-oxo-1,2,3,4-tetrahydro-isochinolin und 1-Brom-2-(3,4-dimethoxyphenyl)-ethan analog Beispiel 1.
Ausbeute: 35,9 % der Theorie,
Schmelzpunkt: 103-105 °C

| | | | | | | | | |
|---|---|---|---|---|---|---|---|---|
| Ber.:<br>Gef.: | C | 62,60<br>62,41 | H | 7,79<br>7,82 | N | 5,21<br>5,09 | Cl | 6,83<br>7,19 |

Beispiel 57

2-[3-(N-(2-(3,4-Dimethoxy-phenyl)-ethyl)-piperidyl-3)-propyl]-6,7-dimethoxy-1-oxo-1,2,3,4-tetrahydro-isochinolin-hydrochlorid

Hergestellt aus 2-[3-(Piperidyl-3)-propyl]-6,7-dimethoxy-1-oxo-1,2,3,4-tetrahydro-isochinolin und 1-Brom-2-(3,4-dimethoxy-phenyl)-ethan analog Beispiel 1.
Ausbeute: 32,6 % der Theorie,
Schmelzpunkt: 102-106 °C

| | | | | | | | | |
|---|---|---|---|---|---|---|---|---|
| Ber.:<br>Gef.: | C | 63,20<br>63,39 | H | 7,86<br>7,90 | N | 5,08<br>4,86 | Cl | 6,43<br>6,13 |

Beispiel 58

2-[3-(N-(3-(3,4-Methylendioxy-phenoxy)-propyl)-piperidyl-3)-propyl]-6,7-dimethoxy-1-oxo-1,2,3,4-tetrahydro-isochinolin-hydrochlorid

Hergestellt aus 2-[3-(Piperidyl-3)-propyl]-6,7-dimethoxy-1-oxo-1,2,3,4-tetrahydro-isochinolin und 1-Chlor-3-(3,4-methylendioxy-phenoxy)-propan analog Beispiel 1.
Ausbeute: 29,7 % der Theorie,
Schmelzpunkt: 97-100 ° C

| | | | | | | | | |
|---|---|---|---|---|---|---|---|---|
| Ber.:<br>Gef.: | C | 61,63<br>61,94 | H | 7,31<br>7,46 | N | 4,96<br>5,16 | Cl | 6,47<br>6,48 |

Beispiel 59

2-[(N-(3,4-Dimethoxy-benzyl)-piperidyl-3)-methyl]-6,7-dimethoxy-1-oxo-1,2,3,4-tetrahydro-isochinolin-hydrochlorid

Hergestellt aus 2-(Piperidyl-3-methyl)-6,7-dimethoxy-1-oxo-1,2,3,4-tetrahydro-isochinolin und 3,4-Dimethoxy-benzylbromid analog Beispiel 1.
Ausbeute: 53,3 % der Theorie,
Schmelzpunkt: 127-132 ° C

| | | | | | | | | |
|---|---|---|---|---|---|---|---|---|
| Ber.:<br>Gef.: | C | 63,58<br>63,30 | H | 7,18<br>7,22 | N | 5,70<br>5,52 | Cl | 7,22<br>7,14 |

Beispiel 60

2-[(N-(3-(4-Methoxy-phenyl)-propyl)-piperidyl-3)-methyl]-6,7-dimethoxy-1-oxo-1,2,3,4-tetrahydro-isochinolin-hydrochlorid

Hergestellt aus 2-(Piperidyl-3-methyl)-6,7-dimethoxy-1-oxo-1,2,3,4-tetrahydro-isochinolin und 1-Brom-3-(4-methoxyphenyl)-propan analog Beispiel 1.
Ausbeute: 42 % der Theorie,
Schmelzpunkt: 229-231 ° C

| | | | | | | | | |
|---|---|---|---|---|---|---|---|---|
| Ber.:<br>Gef.: | C | 66,31<br>66,27 | H | 7,63<br>7,64 | N | 5,73<br>5,65 | Cl | 7,25<br>7,33 |

Beispiel 61

2-[2-(N-(3-(3-Methyl-phenoxy)-propyl)-piperidyl-2)-ethyl]-6,7-dimethoxy-1-oxo-1,2,3,4-tetrahydro-isochinolin-hydrochlorid

Hergestellt aus 2-[2-(Piperidyl-2)-ethyl]-6,7-dimethoxy-1-oxo-1,2,3,4-tetrahydro-isochinolin und 1-Chlor-3-(3-methyl-phenoxy)-propan analog Beispiel 1.
Ausbeute: 52,4 % der Theorie,
Schmelzpunkt: 142-144 ° C

| | | | | | | | | |
|---|---|---|---|---|---|---|---|---|
| Ber.:<br>Gef.: | C | 66,85<br>66,73 | H | 7,81<br>7,68 | N | 5,57<br>5,53 | Cl | 7,05<br>6,94 |

Beispiel 62

2-[2-(N-(2-(3,4-Dimethoxy-phenyl)-ethyl)-piperidyl-2)-ethyl]-6,7-dimethoxy-1-oxo-1,2,3,4-tetrahydro-isochinolin-hydrochlorid

Hergestellt aus 2-[2-(Piperidyl-2)-ethyl]-6,7-dimethoxy-1-oxo-1,2,3,4-tetrahydro-isochinolin und 1-Brom-2-(3,4-dimethoxy-phenyl)-ethan analog Beispiel 1.
Ausbeute: 47,3 % der Theorie,
Schmelzpunkt: 150-155 °C

| | | | | | | | | |
|---|---|---|---|---|---|---|---|---|
| Ber.:<br>Gef.: | C | 64,72<br>64,40 | H | 7,68<br>7,83 | N | 5,39<br>5,27 | Cl | 6,82<br>6,90 |

Beispiel 63

2-[2-(N-(3-Benzyloxy-propyl)-piperidyl-2)-ethyl]-6,7-dimethoxy-1-oxo-1,2,3,4-tetrahydro-isochinolin-hydrochlorid

Hergestellt aus 2-[2-(Piperidyl-2)-ethyl]-6,7-dimethoxy-1-oxo-1,2,3,4-tetrahydro-isochinolin und 1-Chlor-3-benzyloxypropan analog Beispiel 1.
Ausbeute: 56,3 % der Theorie,
Schmelzpunkt: 116-120 °C

| | | | | | | | | |
|---|---|---|---|---|---|---|---|---|
| Ber.:<br>Gef.: | C | 66,85<br>66,60 | H | 7,81<br>7,75 | N | 5,57<br>5,25 | Cl | 7,05<br>7,25 |

Beispiel 64

2-[2-(N-(4-(4-Methoxy-phenyl)-butyl)-piperidyl-2)-ethyl]-6,7-dimethoxy-1-oxo-1,2,3,4-tetrahydro-isochinolin-hydrochlorid

Hergestellt aus 2-[2-(Piperidyl-2)-ethyl]-6,7-dimethoxy-1-oxo-1,2,3,4-tetrahydro-isochinolin und 1-Brom-4-(4-methoxy-phenyl)-butan analog Beispiel 1.
Ausbeute: 42,8 % der Theorie,
Schmelzpunkt: 107-112 °C

| | | | | | | | | |
|---|---|---|---|---|---|---|---|---|
| Ber.:<br>Gef.: | C | 67,36<br>67,16 | H | 7,99<br>8,05 | N | 5,42<br>5,35 | Cl | 6,86<br>7,34 |

Beispiel 65

2-[2-(N-(3-(3,5-Dimethoxy-phenoxy)-propyl)-piperidyl-2)-ethyl]-6,7-dimethoxy-1-oxo-1,2,3,4-tetrahydro-isochinolin-hydrochlorid

Hergestellt aus 2-[2-(Piperidyl-2)-ethyl]-6,7-dimethoxy-1-oxo-1,2,3,4-tetrahydro-isochinolin und 1-Chlor-3-(3,5-dimethoxy-phenoxy)-propan analog Beispiel 1.
Ausbeute: 56,3 % der Theorie,

Schmelzpunkt: 127-132 °C

| | | | | | | | | |
|---|---|---|---|---|---|---|---|---|
| Ber.:<br>Gef.: | C | 61,41<br>61,56 | H | 7,64<br>7,65 | N | 5,10<br>5,28 | Cl | 6,46<br>6,89 |

Beispiel 66

2-[2-(N-(3-(3,4-Methylendioxy-phenoxy)-propyl)-piperidyl-2)-ethyl]-6,7-dimethoxy-1-oxo-1,2,3,4-tetrahydro-isochinolin-hydrochlorid

Hergestellt aus 2-[2-(Piperidyl-2)-ethyl]-6,7-dimethoxy-1-oxo-1,2,3,4-tetrahydro-isochinolin und 1-Chlor-3-(3,4-methylendioxy-phenoxy)-propan analog Beispiel 1.
Ausbeute: 49 % der Theorie,
Schmelspunkt: 118-120 °C

| | | | | | | | | |
|---|---|---|---|---|---|---|---|---|
| Ber.:<br>Gef.: | C | 63,09<br>62,90 | H | 7,00<br>7,04 | N | 5,26<br>5,46 | Cl | 6,65<br>6,79 |

Beispiel 67

2-[(N-(3-(3,5-Dimethoxy-phenoxy)-propyl)-piperidyl-3)-methyl]-6,7-dimethoxy-1-oxo-1,2,3,4-tetrahydro-isochinolin-hydrochlorid

Hergestellt aus 2-[(Piperidyl-3)-methyl]-6,7-dimethoxy-1-oxo-1,2,3,4-tetrahydro-isochinolin und 1-Chlor-3-(3,5-dimethoxy-phenoxy)-propan analog Beispiel 1.
Ausbeute: 37,5 % der Theorie,
Schmelzpunkt: 98-102 °C

| | | | | | | | | |
|---|---|---|---|---|---|---|---|---|
| Ber.:<br>Gef.: | C | 62,85<br>62,81 | H | 7,35<br>7,41 | N | 5,24<br>5,10 | Cl | 6,63<br>6,75 |

Beispiel 68

2-[(N-(3-(3,4-Methylendioxy-phenyl)-propyl)-piperidyl-3)-methyl]-6,7-dimethoxy-1-oxo-1,2,3,4-tetrahydro-isochinolin-hydrochlorid

Hergestellt aus 2-[(Piperidyl-2)-methyl]-6,7-dimethoxy-1-oxo-1,2,3,4-tetrahydro-isochinolin und 1-Chlor-3-(3,4-methylendioxyphenyl)-propan analog Beispiel 1.
Ausbeute: 50 % der Theorie,
Schmelzpunkt: 236-238 °C

| | | | | | | | | |
|---|---|---|---|---|---|---|---|---|
| Ber.:<br>Gef.: | C | 64,46<br>64,30 | H | 7,01<br>6,97 | N | 5,57<br>5,59 | Cl | 7,04<br>7,08 |

Beispiel 69

2-[(N-(3-(3,4-Methylendioxy-phenoxy)-propyl)-piperidyl-3)-methyl]-6,7-dimethoxy-1-oxo-1,2,3,4-tetrahydro-isochinolin-hydrochlorid

Hergestellt aus 2-[(Piperidyl-2)-methyl]-6,7-dimethoxy-1-oxo-1,2,3,4-tetrahydro-isochinolin und 1-Chlor-3-(3,4-methylendioxy-phenoxy)-propan analog Beispiel 1.
Ausbeute: 46,2 % der Theorie,
Schmelzpunkt: 149-153 ° C

| | | | | | | | | |
|---|---|---|---|---|---|---|---|---|
| Ber.:<br>Gef.: | C | 60,38<br>60,30 | H | 6,94<br>6,93 | N | 5,21<br>5,29 | Cl | 6,60<br>6,37 |

Beispiel 70

2-[(N-(3-(2,6-Dimethyl-phenoxy)-propyl)-piperidyl-3)-methyl]-6,7-dimethoxy-1-oxo-1,2,3,4-tetrahydro-isochinolin-hydrochlorid

Hergestellt aus 2-[(Piperidyl-3)-methyl]-6,7-dimethoxy-1-oxo-1,2,3,4-tetrahydro-isochinolin und 1-Chlor-3-(2,6-dimethyl-phenoxy)-propan analog Beispiel 1.
Ausbeute: 53,3 % der Theorie,
Schmelzpunkt: 131-135 ° C

| | | | | | | | | |
|---|---|---|---|---|---|---|---|---|
| Ber.:<br>Gef.: | C | 66,85<br>66,88 | H | 7,81<br>7,95 | N | 5,57<br>5,59 | Cl | 7,05<br>6,85 |

Beispiel 71

2-[(N-(4-(2,4-Dichlor-phenoxy)-butyl)-piperidyl-3)-methyl]-6,7-dimethoxy-1-oxo-1,2,3,4-tetrahydro-isochinolin-hydrochlorid

Hergestellt aus 2-[(Piperidyl-3)-methyl]-6,7-dimethoxy-1-oxo-1,2,3,4-tetrahydro-isochinolin und 1-Chlor-4-(2,4-dichlor-phenoxy)-butan analog Beispiel 1.
Ausbeute: 54,1 % der Theorie,
Schmelzpunkt: 125-128 ° C

| | | | | | | | | |
|---|---|---|---|---|---|---|---|---|
| Ber.:<br>Gef.: | C | 58,12<br>58,21 | H | 6,32<br>6,38 | N | 5,02<br>5,08 | Cl | 19,06<br>18,85 |

Beispiel 72

2-[(N-(2-(3,4-Dimethoxy-phenyl)-ethyl)-piperidyl-3)-methyl]-6,7-dimethoxy-1-oxo-1,2,3,4-tetrahydro-isochinolin-hydrochlorid

Hergestellt aus 2-[(Piperidyl-3)-methyl]-6,7-dimethoxy-1-oxo-1,2,3,4-tetrahydro-isochinolin und 1-Brom-2-(3,4-dimethoxy-phenyl)-ethan analog Beispiel 1.
Ausbeute: 57,5 % der Theorie,
Schmelzpunkt: 118-121 ° C

| | | | | | | |
|---|---|---|---|---|---|---|
| Ber.:<br>Gef.: | C | 64,21<br>64,18 | H | 7,38<br>7,36 | N | 5,55<br>5,19 |

Beispiel 73

2-[(N-(3-(3,4-Dimethoxy-phenoxy)-propyl)-piperidyl-3)-methyl]-6,7-dimethoxy-1-oxo-1,2,3,4-tetrahydro-isochinolin-hydrochlorid

Hergestellt aus 2-[(Piperidyl-3)-methyl]-6,7-dimethoxy-1-oxo-1,2,3,4-tetrahydro-isochinolin und 1-Chlor-3-(3,4-dimethoxy-phenoxy)-propan analog Beispiel 1.
Ausbeute: 62,5 % der Theorie,
Schmelzpunkt: 112-115 °C

| | | | | | | |
|---|---|---|---|---|---|---|
| Ber.:<br>Gef.: | C | 60,80<br>60,65 | H | 7,47<br>7,69 | N | 5,24<br>5,27 |

Beispiel 74

2-[(N-(3-(3,4-Dimethoxy-phenoxy)-propyl)-piperidyl-3)-methyl]-6,7-methylendioxy-1-oxo-1,2,3,4-tetrahydro-isochinolin-hydrochlorid

Hergestellt aus 2-[(Piperidyl-3)-methyl]-6,7-methylendioxy-1-oxo-1,2,3,4-tetrahydro-isochinolin und 1-Chlor-3-(3,4-dimethoxy-phenoxy)-propan analog Beispiel 1.
Ausbeute: 60 % der Theorie,
Schmelzpunkt: 97-100 °C

| | | | | | | | | |
|---|---|---|---|---|---|---|---|---|
| Ber.:<br>Gef.: | C | 60,38<br>60,20 | H | 6,94<br>6,97 | N | 5,40<br>5,21 | Cl | 6,60<br>6,83 |

Beispiel 75

2-[(N-(2-(4-Methoxy-phenyl)-ethyl)-piperidyl-3)-methyl]-6,7-methylendioxy-1-oxo-1,2,3,4-tetrahydro-isochinolin-hydrochlorid

Hergestellt aus 2-[(Piperidyl-3)-methyl]-6,7-methylendioxy-1-oxo-1,2,3,4-tetrahydro-isochinolin und 1-Chlor-2-(4-methoxy-phenyl)-ethan analog Beispiel 1.
Ausbeute: 71,4 % der Theorie,
Schmelzpunkt: 195-197 °C

| | | | | | | | | |
|---|---|---|---|---|---|---|---|---|
| Ber.:<br>Gef.: | C | 62,94<br>62,90 | H | 6,97<br>6,98 | N | 5,87<br>5,68 | Cl | 7,73<br>8,04 |

Beispiel 76

2-[(N-(2-(3,4-Dimethoxy-phenyl)-ethyl)-piperidyl-3)-methyl]-6,7-dimethyl-1-oxo-1,2,3,4-tetrahydro-isochinolin-hydrochlorid

Hergestellt aus 2-[(Piperidyl-3)-methyl]-6,7-dimethyl-1-oxo-1,2,3,4-tetrahydro-isochinolin und 1-Brom-2-(3,4-dimethoxy-phenyl)-ethan analog Beispiel 1.
Ausbeute: 41,9 % der Theorie,

Schmelzpunkt: 132-134 °C

| | | | | | | | | |
|---|---|---|---|---|---|---|---|---|
| Ber.:<br>Gef.: | C | 63,57<br>63,70 | H | 8,10<br>8,26 | N | 5,49<br>5,45 | Cl | 6,95<br>7,13 |

Beispiel 77

2-[(N-(3-(4-Methoxy-phenoxy)-propyl)-piperidyl-3)-methyl]-6,7-dimethyl-1-oxo-1,2,3,4-tetrahydro-isochinolin-hydrochlorid

Hergestellt aus 2-[(Piperidyl-3)-methyl]-6,7-dimethyl-1-oxo-1,2,3,4-tetrahydro-isochinolin und 1-Chlor-3-(4-methoxy-phenoxy)-propan analog Beispiel 1.
Ausbeute: 57,8 % der Theorie,
Schmelzpunkt: 144-146 °C

| | | | | | | | | |
|---|---|---|---|---|---|---|---|---|
| Ber.:<br>Gef.: | C | 68,55<br>68,45 | H | 7,88<br>7,80 | N | 5,92<br>6,11 | Cl | 7,49<br>7,33 |

Beispiel 78

2-[(N-(3-(3-Methoxy-phenoxy)-propyl)-piperidyl-3)-methyl]-6,7-methylendioxy-1-oxo-1,2,3,4-tetrahydro-isochinolin-hydrochlorid

Hergestellt aus 2-[(Piperidyl-3)-methyl]-6,7-methylendioxy-1-oxo-1,2,3,4-tetrahydro-isochinolin und 1-Chlor-3-(3-methoxy-phenoxy)-propan analog Beispiel 1.
Ausbeute: 32,5 % der Theorie,
Schmelzpunkt: 142-145 °C

| | | | | | | | | |
|---|---|---|---|---|---|---|---|---|
| Ber.:<br>Gef.: | C | 60,58<br>60,42 | H | 6,95<br>6,92 | N | 5,52<br>5,50 | Cl | 6,99<br>7,18 |

Beispiel 79

2-[(N-(3-(3-Methyl-phenoxy)-propyl)-piperidyl-3)-methyl]-6,7-methylendioxy-1-oxo-1,2,3,4-tetrahydro-isochinolin-hydrochlorid

Hergestellt aus 2-[(Piperidyl-3)-methyl]-6,7-methylendioxy-1-oxo-1,2,3,4-tetrahydro-isochinolin und 3-(3-Methyl-phenoxy)-1-chlor-propan analog Beispiel 1.
Ausbeute: 31,6 % der Theorie,
Schmelzpunkt: 178-180 °C

| | | | | | | | | |
|---|---|---|---|---|---|---|---|---|
| Ber.:<br>Gef.: | C | 63,59<br>63,59 | H | 6,97<br>6,92 | N | 5,70<br>5,86 | Cl | 7,22<br>7,50 |

Beispiel 80

2-[(N-(3-(4-Methoxy-N-methyl-phenylamino)-propyl)-piperidyl-3)-methyl]-6,7-dimethoxy-1-oxo-1,2,3,4-tetrahydro-isochinolin-dihydrochlorid

Hergestellt aus 2-[(Piperidyl-3)-methyl]-6,7-dimethoxy-1-oxo-1,2,3,4-tetrahydro-isochinolin und 1-Chlor-3-(4-methoxy-N-methyl-phenylamino)-propan analog Beispiel 1.
Ausbeute: 52,5 % der Theorie,
Schmelzpunkt: 180-183 ° C

| | | | | | | | | |
|---|---|---|---|---|---|---|---|---|
| Ber.:<br>Gef.: | C | 60,64<br>60,50 | H | 7,45<br>7,35 | N | 7,58<br>7,56 | Cl | 12,79<br>12,87 |

Beispiel 81

2-[(N-(3-(4-Methoxy-phenoxy)-propyl)-pyrrolidyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und N-(3-(4-Methoxy-phenoxy)-propyl)-3-benzolsulfonyloxymethyl-pyrrolidin analog Beispiel 2.
Ausbeute: 84,4 % der Theorie,
Schmelzpunkt: 142-144 ° C

| | | | | | | | | |
|---|---|---|---|---|---|---|---|---|
| Ber.:<br>Gef.: | C | 63,60<br>63,75 | H | 7,18<br>7,12 | N | 5,71<br>5,64 | Cl | 7,22<br>7,32 |

Beispiel 82

2-[(N-(3-(6-Methoxy-naphthyl-2-oxy)-propyl)-pyrrolidyl-3)-methyl]-6,7-dimethoxy-1-oxo-1,2,3,4-tetrahydro-isochinolin

Hergestellt aus 6,7-Dimethoxy-1-oxo-1,2,3,4-tetrahydro-isochinolin und 3-(p-Toluolsulfonyloxymethyl)-N-(6-methoxy-naphthyl-2-oxy)-pyrrolidin analog Beispiel 2.
Ausbeute: 47 % der Theorie,
Schmelzpunkt: 142-144 ° C

| | | | | | | |
|---|---|---|---|---|---|---|
| Ber.:<br>Gef.: | C | 71,41<br>71,14 | H | 7,19<br>7,16 | N | 5,55<br>5,53 |

Beispiel 83

2-[(N-(3-(4-Methoxy-phenoxy)-propyl)-pyrrolidyl-3)-methyl]-6,7-dimethoxy-1,2,3,4-tetrahydro-isochinolin-dihydrochlorid

Hergestellt aus 2-[(N-(3-(4-Methoxy-phenoxy)-propyl)-pyrrolidyl-3)-methyl]-6,7-dimethoxy-1-oxo-1,2,3,4-tetrahydro-isochinolin und Lithiumaluminiumhydrid in Tetrahydrofuran analog Beispiel 3.
Ausbeute: 90,9 % der Theorie,
Schmelzpunkt: 248-250 ° C

| | | | | | | | | |
|---|---|---|---|---|---|---|---|---|
| Ber.:<br>Gef.: | C | 60,81<br>60,79 | H | 7,46<br>7,61 | N | 5,46<br>5,48 | Cl | 13,81<br>13,84 |

Beispiel 84

2-[(N-(3-(4-Methoxy-phenoxy)-propyl)-pyrrolidyl-3)-methyl]-6,7-dimethyl-1-oxo-1,2,3,4-tetrahydro-isochinolin-hydrochlorid

Hergestellt aus 6,7-Dimethyl-1-oxo-1,2,3,4-tetrahydro-isochinolin und N-[3-(4-Methoxy-phenoxy)-propyl]-3-benzolsulfonyloxymethyl-pyrrolidin analog Beispiel 2.
Ausbeute: 60,6 % der Theorie,
Schmelzpunkt: 118-121 °C

| | | | | | | | | |
|---|---|---|---|---|---|---|---|---|
| Ber.:<br>Gef.: | C | 68,03<br>67,90 | H | 7,69<br>7,71 | N | 6,10<br>6,04 | Cl | 7,72<br>7,90 |

Beispiel 85

2-[(N-(2-(3,4-Dimethoxy-phenyl)-ethyl)-pyrrolidyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und N-[2-(3,4-Dimethoxy-phenoxy)-ethyl]-3-benzolsulfonyloxymethyl-pyrrolidin analog Beispiel 2.
Ausbeute: 56,7 % der Theorie,
Schmelzpunkt: 116-118 °C

| | | | | | | | | |
|---|---|---|---|---|---|---|---|---|
| Ber.:<br>Gef.: | C | 63,60<br>63,82 | H | 7,19<br>7,32 | N | 5,71<br>5,60 | Cl | 7,22<br>7,66 |

Beispiel 86

2-[(N-(3-(4-Methoxy-phenoxy)-propyl)-pyrrolidyl-3)-methyl]-6,7-dimethyl-1,2,3,4-tetrahydro-isochinolin-dihydrochlorid

Hergestellt aus 2-[N-(3-(4-Methoxy-phenoxy)-propyl)-pyrrolidyl-3)-methyl]-6,7-dimethyl-1-oxo-1,2,3,4-tetrahydro-isochino lin und Lithiumaluminiumhydrid in Tetrahydrofuran analog Beispiel 3.
Ausbeute: 90,9 % der Theorie,
Schmelzpunkt: 243-246 °C

| | | | | | | | | |
|---|---|---|---|---|---|---|---|---|
| Ber.:<br>Gef.: | C | 64,85<br>64,88 | H | 7,95<br>7,92 | N | 5,82<br>5,63 | Cl | 14,73<br>14,80 |

Beispiel 87

2-[(N-(2-(3,4-Dimethoxy-phenyl)-ethyl)-pyrrolidyl-3)-methyl]-6,7-dimethoxy-1,2,3,4-tetrahydro-isochinolin-dihydrochlorid

Hergestellt aus 2-[(N-(2-(3,4-Dimethoxy-phenyl)-ethyl)-pyrrolidyl-3)-methyl]-6,7-dimethoxy-1-oxo-1,2,3,4-tetrahydro-isochinolin und Lithiumaluminiumhydrid in Tetrahydrofuran analog Beispiel 3.
Ausbeute: 92,9 % der Theorie,

Schmelzpunkt: 240-242 ° C

| | | | | | | | | |
|---|---|---|---|---|---|---|---|---|
| Ber.:<br>Gef.: | C | 60,81<br>60,64 | H | 7,46<br>7,61 | N | 5,46<br>5,31 | Cl | 13,81<br>13,50 |

Beispiel 88

2-[(N-(3-Pyridyl-4)-propyl)-pyrrolidyl-3)-methyl]-5,6-dimethyl-1-oxo-1,3-dihydro-isoindol-dihydrochlorid-semihydrat

Hergestellt aus 2-[(N-(3-Pyridyl-4)-propy)-pyrrolidyl-3)-methyl]-5,6-dimethyl-phthalimid und Zink/Eisessig analog Beispiel 4.
Ausbeute: 65 % der Theorie,
Schmelzpunkt: 119-122 ° C

| | | | | | | | | |
|---|---|---|---|---|---|---|---|---|
| Ber.:<br>Gef.: | C | 62,02<br>62,25 | H | 7,24<br>7,47 | N | 9,43<br>9,39 | Cl | 15,92<br>15,90 |

Beispiel 89

2-[3-(N-(3-(Pyridyl-3)-propyl)-piperidyl-3)-propyl]-5,6-dimethoxy-1-oxo-1,3-dihydro-isoindol-dihydrochlorid-monohydrat

Hergestellt aus 2-[3-(N-(3-Pyridyl-3)-propyl)-piperidyl-3)-propyl]-5,6-dimethoxy-phthalimid und Zink/Eisessig analog Beispiel 4.
Ausbeute: 72 % der Theorie,
Schmelzpunkt: 118-121 ° C

| | | | | | | | | |
|---|---|---|---|---|---|---|---|---|
| Ber.:<br>Gef.: | C | 59,08<br>59,02 | H | 7,43<br>7,23 | N | 7,95<br>7,12 | Cl | 13,41<br>13,27 |

Beispiel 90

2-[(N-(3-(Pyridyl-3)-propyl)-piperidyl-3)-methyl]-5,6-dimethoxy-1-oxo-1,3-dihydro-isoindol-dihydrochlorid-monohydrat

Hergestellt aus 2-[(N-(3-Pyridyl-3)-propyl)-piperidyl-3)-methyl]-5,6-dimethoxy-phthalimid und Zink/Eisessig analog Beispiel 4.
Ausbeute: 42 % der Theorie,
Schmelzpunkt: 91-96 ° C

| | | | | | | | | |
|---|---|---|---|---|---|---|---|---|
| Ber.:<br>Gef.: | C | 59,08<br>59,02 | H | 7,43<br>7,23 | N | 7,95<br>7,12 | Cl | 13,41<br>13,27 |

Beispiel 91

2-[(N-(2-(6,7-Dimethoxy-isochinolyl-4)-ethyl)-piperidyl-3)-methyl]-5,6-dimethoxy-1-oxo-1,3-dihydro-isoindol

Hergestellt aus 2-[(N-(2-(6,7-Dimethoxy-isochinolyl-4)-ethyl)-piperidyl-3)-methyl]-5,6-dimethoxy-phthalimid und Zink/Eisessig analog Beispiel 4.

Ausbeute: 64 % der Theorie,
Schmelzpunkt: 85-88 ° C

| | | | | | | |
|---|---|---|---|---|---|---|
| Ber.:<br>Gef.: | C | 65,39<br>65,16 | H | 7,19<br>7,27 | N | 7,88<br>7,53 |

Beispiel 92

2-[2-(N-(3-(Pyridyl-4)-propyl)-piperidyl-2)-ethyl]-5,6-dimethyl-1-oxo-1,3-dihydro-isoindol

Hergestellt aus 2-[2-(N-(3-(Pyridyl-4)-propyl)-piperidyl-2)-ethyl]-5,6-dimethyl-phthalimid und Zink/Eisessig analog Beispiel 4.
Ausbeute: 73 % der Theorie,
Schmelzpunkt: 103-104 ° C

| | | | | | | |
|---|---|---|---|---|---|---|
| Ber.:<br>Gef.: | C | 76,68<br>76,57 | H | 8,49<br>8,54 | N | 10,73<br>10,60 |

Beispiel 93

2-[(N-(3-(Pyridyl-4)-propyl)-pyrrolidyl-3)-methyl]-5,6-dimethyl-1,3-dihydro-isoindol-trihydrochlorid-semihydrat

Hergestellt aus 2-[(N-(3-(Pyridyl-4)-propyl)-pyrrolidyl-3)-methyl]-5,6-dimethyl-1-oxo-1,3-dihydro-isoindol und Lithiumaluminiumhydrid in Tetrahydrofuran/Ether analog Beispiel 3.
Ausbeute: 68 % der Theorie,
Schmelzbereich: 118-127 ° C (amorph)

| | | | | | | | | |
|---|---|---|---|---|---|---|---|---|
| Ber.:<br>Gef.: | C | 59,03<br>58,93 | H | 7,54<br>7,48 | N | 8,98<br>8,84 | Cl | 22,73<br>22,92 |

Beispiel 94

2-[2-(N-(3-(Pyridyl-4)-propyl)-piperidyl-2)-ethyl]-5,6-dimethyl-1,3-dihydro-isoindol

Hergestellt aus 2-[2-(N-(3-(Pyridyl-4)-propyl)-piperidyl-2)-ethyl]-5,6-dimethyl-1-oxo-1,3-dihydro-isoindol und Lithiumaluminiumhydrid in Tetrahydrofuran/Ether analog Beispiel 3.
Ausbeute: 70 % der Theorie,
Schmelzbereich: 135-148 ° C (amorph)

| | | | | | | | | |
|---|---|---|---|---|---|---|---|---|
| Ber.:<br>Gef.: | C | 54,59<br>54,48 | H | 8,24<br>8,26 | N | 7,63<br>7,51 | Cl | 19,33<br>19,60 |

Beispiel 95

2-[(N-(1-(Pyridyl-4)-methyl)-piperidyl-3)-methyl]-5,6-dimethoxy-1,3-dihydro-isoindol-trihydrochlorid-trihydrat

Hergestellt aus 2-[(N-(1-(Pyridyl-4)-methyl)-piperidyl-3)-methyl]-5,6-dimethoxy-phthalimid und Lithiumaluminiumhydrid in Ether analog Beispiel 3.
Ausbeute: 68 % der Theorie,
Schmelzbereich: 176-189 ° C (amorph)

| | | | | | | | | |
|---|---|---|---|---|---|---|---|---|
| Ber.:<br>Gef.: | C | 49,76<br>49,93 | H | 7,21<br>7,12 | N | 7,91<br>8,00 | Cl | 20,03<br>20,44 |

Beispiel 96

2-[(N-(2-(6,7-Dimethoxy-isochinolyl-4)-ethyl)-piperidyl-3)-methyl]-5,6-dimethyl-1,3-dihydro-isoindol-dihydrochlorid-semihydrat

Hergestellt aus 2-[(N-(2-(6,7-Dimethoxy-isochinolyl-4)-ethyl)-piperidyl-3)-methyl]-5,6-dimethyl-1-oxo-1,3-dihydro-isoindol und Lithiumaluminiumhydrid in Tetrahydrofuran/Ether analog Beispiel 3.
Ausbeute: 22,7 % der Theorie,
Schmelzbereich: 222-236 °C (amorph)

| | | | | | | | | |
|---|---|---|---|---|---|---|---|---|
| Ber.:<br>Gef.: | C | 62,22<br>62,01 | H | 7,20<br>7,64 | N | 7,50<br>7,08 | Cl | 15,83<br>15,79 |

Beispiel 97

2-[(N-(1-(Pyridyl-4)-methyl)-piperidyl-3)-methyl]-5,6-methylendioxy-1,3-dihydro-isoindol-trihydrochlorid-semihydrat

Hergestellt aus 2-[(N-(1-(Pyridyl-4)-methyl)-piperidyl-3)-methyl]-5,6-methylendioxy-phthalimid und Lithiumaluminiumhydrid in Tetrahydrofuran/Ether analog Beispiel 3.
Ausbeute: 55 % der Theorie,
Schmelzbereich: 215-225 °C (amorph)

| | | | | | | | | |
|---|---|---|---|---|---|---|---|---|
| Ber.:<br>Gef.: | C | 53,68<br>53,60 | H | 6,22<br>6,45 | N | 8,94<br>8,65 | Cl | 22,63<br>22,28 |

Beispiel 98

2-[(N-(3-(Pyridyl-4)-propyl)-pyrrolidyl-3)-methyl]-6,7-dimethyl-1,2,3,4-tetrahydro-isochinolin-trihydrochlorid-monohydrat

Hergestellt aus 2-[(N-(3-(Pyridyl-4)-propyl)-pyrrolidyl-3)-methyl]-6,7-dimethyl-1-oxo-1,2,3-4-tetrahydro-isochinolin und Lithiumaluminiumhydrid in Tetrahydrofuran/Ether analog Beispiel 3.
Ausbeute: 63 % der Theorie,
Schmelzpunkt: 254-256 °C

| | | | | | | | | |
|---|---|---|---|---|---|---|---|---|
| Ber.:<br>Gef.: | C | 58,71<br>58,53 | H | 7,80<br>7,72 | N | 8,56<br>8,25 | Cl | 21,66<br>21,53 |

Beispiel 99

2-[(N-(1-(Pyridyl-4)-methyl)-piperidyl-3)-methyl]-6,7-dimethoxy-1,2,3,4-tetrahydro-isochinolin-trihydrochlorid-dihydrat

Hergestellt aus 2-[(N-(1-(Pyridyl-4)-methyl)-piperidyl-3)-methyl]-6,7-dimethoxy-1-oxo-1,2,3-4-tetrahydro-isochinolin und Lithiumaluminiumhydrid in Tetrahydrofuran/Ether analog Beispiel 3.
Ausbeute: 63 % der Theorie,

Schmelzbereich: 158-169 °C (amorph)

| | | | | | | | | |
|---|---|---|---|---|---|---|---|---|
| Ber.:<br>Gef.: | C | 52,42<br>52,55 | H | 7,27<br>7,49 | N | 7,97<br>7,57 | Cl | 20,18<br>20,25 |

Beispiel 100

2-[2-(N-(3-(Pyridyl-4)-propyl)-piperidyl-2)-ethyl]-6,7-methylendioxy-1,2,3,4-tetrahydro-isochinolin-trihydrochlorid-trihydrat

Hergestellt aus 2-[2-(N-(3-(Pyridyl-4)-propyl)-piperidyl-2)-ethyl]-6,7-methylendioxy-1-oxo-1,2,3-4-tetrahydro-isochinolin und Lithiumaluminiumhydrid in Tetrahydrofuran/Ether analog Beispiel 3.
Ausbeute: 90 % der Theorie,
Schmelzbereich: 108-119 °C (amorph)

| | | | | | | | | |
|---|---|---|---|---|---|---|---|---|
| Ber.:<br>Gef.: | C | 52,58<br>52,56 | H | 7,41<br>7,25 | N | 7,36<br>7,38 | Cl | 18,62<br>19,49 |

Beispiel 101

2-[(N-(3-(Pyridyl-3)-propyl)-piperidyl-3)-methyl]-6,7-methylendioxy-1,2,3,4-tetrahydro-isochinolin-dihydrochlorid-monohydrat

Hergestellt aus 2-[(N-(3-(Pyridyl-3)-propyl)-piperidyl-3)-methyl]-6,7-methylendioxy-1-oxo-1,2,3-4-tetrahydro-isochinolin und Lithiumaluminiumhydrid in Tetrahydro£uran/Ether analog Beispiel 3.
Ausbeute: 72 % der Theorie,
Schmelzbereich: 126-138 °C (amorph)

| | | | | | | | | |
|---|---|---|---|---|---|---|---|---|
| Ber.:<br>Gef.: | C | 59,50<br>59,57 | H | 7,28<br>7,29 | N | 8,67<br>8,49 | Cl | 14,64<br>14,51 |

Beispiel 102

2-[(N-(3-(Pyridyl-3)-propyl)-piperidyl-3)-methyl]-6,7-dimethyl-1,2,3,4-tetrahydro-isochinolin-trihydrochlorid

Hergestellt aus 2-[(N-(3-(Pyridyl-3)-propyl)-piperidyl-3)-methyl]-6,7-dimethyl-1-oxo-1,2,3-4-tetrahydro-isochinolin und Lithiumaluminiumhydrid in Tetrahydrofuran/Ether analog Beispiel 3.
Ausbeute: 59 % der Theorie,
Schmelzbereich: 138-154 °C (amorph)

| | | | | | | | | |
|---|---|---|---|---|---|---|---|---|
| Ber.:<br>Gef.: | C | 61,66<br>61,53 | H | 7,86<br>8,00 | N | 8,62<br>8,64 | Cl | 21,84<br>21,35 |

Beispiel 103

2-[(N-(3-(Pyridyl-4)-propyl)-pyrrolidyl-3)-methyl]-6,7-dimethyl-1-oxo-1,2,3,4-tetrahydro-isochinolin-dihydrochlorid-monohydrat

Hergestellt aus 6,7-Dimethyl-1-oxo-1,2,3-4-tetrahydro-isochinolin und 3-Chlormethyl-N-[3-(pyridyl-4)-propyl]-pyrrolidin analog Beispiel 2.

Ausbeute: 39 % der Theorie,
Schmelzbereich: 74-86 ° C (amorph)

| | | | | | | | | |
|---|---|---|---|---|---|---|---|---|
| Ber.:<br>Gef.: | C | 61,53<br>61,42 | H | 7,53<br>7,62 | N | 8,97<br>8,83 | Cl | 15,13<br>15,05 |

Beispiel 104

2-[(N-(3-(Pyridyl-3)-propyl)-piperidyl-3)-methyl]-6,7-dimethoxy-1-oxo-1,2,3,4-tetrahydro-isochinolin-dihydrochlorid-monohydrat

Hergestellt aus 6,7-Dimethoxy-1-oxo-1,2,3-4-tetrahydro-isochinolin und 3-Benzolsulfonsäure-N-[3-(pyridyl-3)-propyl)-piperidyl-3]-methylester in Dimethylsulfoxid mit Kalium-tert.butylat analog Beispiel 2.
Ausbeute: 45 % der Theorie,
Schmelzbereich: 140-148 ° C (amorph)

| | | | | | | | | |
|---|---|---|---|---|---|---|---|---|
| Ber. (x 2 HCl x $H_2O$):<br>Gef.: | C | 58,36<br>58,35 | H | 7,25<br>7,32 | N | 8,16<br>8,04 | Cl | 13,78<br>13,65 |

Beispiel 105

2-[3-(N-(3-(Pyridyl-4)-propyl)-piperidyl-3)-propyl]-6,7-dimethoxy-1-oxo-1,2,3,4-tetrahydro-isochinolin-dihydrochlorid-dihydrat

Hergestellt aus 2-[3-(Piperidyl-3)-propyl]-6,7-dimethoxy-1-oxo-1,2,3-4-tetrahydro-isochinolin und 4-(3-Chlorpropyl)-pyridin analog Beispiel 1.
Ausbeute: 48 % der Theorie,
Schmelzbereich: 85-96 ° C (amorph)

| | | | | | | | | |
|---|---|---|---|---|---|---|---|---|
| Ber.:<br>Gef.: | C | 57,84<br>57,71 | H | 7,73<br>7,91 | N | 7,49<br>7,35 | Cl | 12,65<br>13,04 |

Beispiel 106

2-[3-(N-(2-(6,7-Dimethoxy-isochinolyl-4)-ethyl)-piperidyl-3)-propyl]-6,7-dimethoxy-1-oxo-1,2,3,4-tetrahydro-isochinolin-dihydrochlorid-monohyrat

Hergestellt aus 2-[3-(Pyridyl-3)-propyl]-6,7-dimethoxy-1-oxo-1,2,3-4-tetrahydro-isochinolin und 4-(2-Chlorethyl)-6,7-dimethoxy-isochinolin analog Beispiel 1.
Ausbeute: 34 % der Theorie,
Schmelzbereich: 162-171 ° C (amorph)

| | | | | | | | | |
|---|---|---|---|---|---|---|---|---|
| Ber.:<br>Gef.: | C | 60,17<br>59,85 | H | 7,10<br>7,00 | N | 6,57<br>6,86 | Cl | 11,10<br>11,04 |

Beispiel 107

2-[(N-(3-(Pyridyl-3)-propyl)-piperidyl-3)-methyl]-6,7-methylendioxy-1-oxo-1,2,3,4-tetrahydro-isochinolin-hydrochlorid

Hergestellt aus 6,7-Methylendioxy-1-oxo-1,2,3-4-tetrahydro-isochinolin und 3-Chlormethyl-N-[3-(pyridyl-3)-propyl]-piperidin analog Beispiel 2.
Ausbeute: 60 % der Theorie,
Schmelzbereich: 194-196 ° C (amorph)

| | | | | | | | | |
|---|---|---|---|---|---|---|---|---|
| Ber.:<br>Gef.: | C | 64,92<br>64,91 | H | 6,81<br>6,95 | N | 9,46<br>9,67 | Cl | 7,98<br>7,80 |

Beispiel 108

2-[(N-(1-(Pyridyl-4)-methyl)-piperidyl-3)-methyl]-6,7-dimethoxy-1-oxo-1,2,3,4-tetrahydro-isochinolin-dihydrochlorid-monohydrat

Hergestellt aus 6,7-Dimethoxy-1-oxo-1,2,3-4-tetrahydro-isochinolin in Dimethylsulfoxid mit Kalium-tert.butylat und 3-Chlormethyl-N-[1-(pyridyl-4)-methyl]-piperidin analog Beispiel 2.
Ausbeute: 41 % der Theorie,
Schmelzbereich: 142-158 ° C (amorph)

| | | | | | | | | |
|---|---|---|---|---|---|---|---|---|
| Ber.:<br>Gef.: | C | 56,78<br>56,45 | H | 6,83<br>6,59 | N | 8,63<br>8,66 | Cl | 14,58<br>14,62 |

Beispiel 109

2-[(N-(2-(6,7-Dimethoxy-isochinolyl-4)-ethyl)-piperidyl-3)-methyl]-6,7-dimethyl-1-oxo-1,2,3,4-tetrahydro-isochinolin-dihydrochlorid

Hergestellt aus 6,7-Dimethyl-1-oxo-1,2,3-4-tetrahydro-isochinolin in Dimethylsulfoxid mit Kalium-tert.butylat und 3-Chlormethyl-N-[2-(6,7-dimethoxy-isochinolyl-4)-ethyl]-piperidin analog Beispiel 2.
Ausbeute: 62 % der Theorie,
Schmelzbereich: 148-162 ° C (amorph)

| | | | | | | | | |
|---|---|---|---|---|---|---|---|---|
| Ber.:<br>Gef.: | C | 64,27<br>64,11 | H | 7,01<br>7,20 | N | 7,49<br>7,59 | Cl | 12,65<br>12,89 |

Beispiel 110

2-[2-(N-(3-(Pyridyl-4)-propyl)-piperidyl-2)-ethyl]-6,7-methylendioxy-1-oxo-1,2,3,4-tetrahydro-isochinolin-dihydrochlorid-dihydrat

Hergestellt aus 6,7-Methylendioxy-1-oxo-1,2,3-4-tetrahydro-isochinolin und 2-(2-Chlorethyl)-N-[3-(pyridyl-4)-propyl]-piperidin analog Beispiel 2.
Schmelzbereich: 115-128 ° C (amorph)

| | | | | | | | | |
|---|---|---|---|---|---|---|---|---|
| Ber.:<br>Gef.: | C | 56,59<br>56,61 | H | 7,03<br>6,90 | N | 7,92<br>7,84 | Cl | 13,37<br>13,41 |

Beispiel 111

2-[(N-(3-(Pyridyl-3)-propyl)-piperidyl-3)-methyl]-6,7-dimethyl-1-oxo-1,2,3,4-tetrahydro-isochinolin-dihydrochlorid-dihydrat

Hergestellt aus 6,7-Dimethyl-1-oxo-1,2,3-4-tetrahydro-isochinolin in Dimethylsulfoxid mit Kalium-tert.butylat und 3-Chlormethyl)-N-[3-(pyridyl-3)-propyl]-piperidin analog Beispiel 2.
Ausbeute: 62 % der Theorie,
Schmelzbereich: 118-127 ° C (amorph)

| | | | | | | | | |
|---|---|---|---|---|---|---|---|---|
| Ber.: | C | 60,00 | H | 7,85 | N | 8,39 | Cl | 14,16 |
| Gef.: | | 60,24 | | 8,07 | | 8,36 | | 14,62 |

Beispiel 112

2-[(N-(3-(Pyridyl-3)-propyl)-pyrrolidyl-3)-methyl]-6,7-methylendioxy-1-oxo-1,2,3,4-tetrahydro-isochinolin-dihydrochlorid

Hergestellt aus 6,7-Methylendioxy-1-oxo-1,2,3-4-tetrahydro-isochinolin und 3-Chlormethyl-N-[3-(pyridyl-3)-propyl]-pyrrolidin analog Beispiel 2.
Ausbeute: 26 % der Theorie,
Schmelzbereich: 102-113 ° C (amorph)

| | | | | | | | | |
|---|---|---|---|---|---|---|---|---|
| Ber.: | C | 59,22 | H | 6,27 | N | 9,01 | Cl | 15,20 |
| Gef.: | | 59,27 | | 6,49 | | 8,92 | | 14,48 |

Beispiel 113

2-[(N-(3-(Pyridyl-4)-propyl)-pyrrolidyl-3)-methyl]-7,8-methylendioxy-2-oxo-1,3-dihydro-2H-3-benzazepin-dihydrochlorid

Hergestellt aus 7,8-Methylendioxy-2-oxo-1,3-dihydro-2H-3-benzazepin und 3-Chlormethyl-N-[3-(pyridyl-4)-propyl]-pyrrolidin analog Beispiel 2.
Ausbeute: 58 % der Theorie,
Schmelzbereich: 132-141 ° C (amorph)

| | | | | | | | | |
|---|---|---|---|---|---|---|---|---|
| Ber.: | C | 60,25 | H | 6,11 | N | 8,78 | Cl | 14,82 |
| Gef.: | | 60,00 | | 6,40 | | 8,52 | | 14,56 |

Beispiel 114

2-[(N-(3-(Pyridyl-3)-propyl)-piperid-3-yl)-methyl]-7,8-dimethoxy-2-oxo-1,3-dihydro-2H-3-benzazepin-dihydrochlorid-dihydrat

Hergestellt aus 7,8-Dimethoxy-2-oxo-1,3-dihydro-2H-3-benzazepin und 3-Chlormethyl-N-[3-(pyridyl-3)-propyl]-piperidin analog Beispiel 2.
Ausbeute: 67 % der Theorie,
Schmelzbereich: 128-134 ° C (amorph)

| | | | | | | | | |
|---|---|---|---|---|---|---|---|---|
| Ber.:<br>Gef.: | C | 57,34<br>57,80 | H | 7,21<br>7,37 | N | 7,71<br>7,92 | Cl | 13,02<br>13,06 |

Beispiel 115

3-[(N-(3-(Pyridyl-3)-propyl)-piperidyl-3)-methyl]-7,8-dimethyl-2-oxo-1,3-dihydro-2H-3-benzazepin-dihydrochlorid-dihydrat

Hergestellt aus 7,8-Dimethyl-2-oxo-1,3-dihydro-2H-3-benzazepin und 3-Chlormethyl-N-[3-(pyridyl-3)-propyl]-piperidin analog Beispiel 2.
Ausbeute: 94 % der Theorie,
Schmelzbereich: 77-86 ° C (amorph)

| | | | | | | | | |
|---|---|---|---|---|---|---|---|---|
| Ber.:<br>Gef.: | C | 60,92<br>60,75 | H | 7,67<br>7,60 | N | 8,19<br>8,37 | Cl | 13,83<br>13,72 |

Beispiel 116

3-[2-(N-(3-(Pyridyl-4)-propyl)-piperidyl-2)-ethyl]-7,8-dimethyl-2-oxo-1,3-dihydro-2H-3-benzazepin-dihydrochlorid

Hergestellt aus 7,8-Dimethyl-2-oxo-1,3-dihydro-2H-3-benzazepin und 2-(2-Chlorethyl)-N-[3-(pyridyl-4)-propyl]-piperidin analog Beispiel 2.
Ausbeute: 94 % der Theorie,
Schmelzbereich: 118-130 ° C (amorph)

| | | | | | | | | |
|---|---|---|---|---|---|---|---|---|
| Ber.:<br>Gef.: | C | 66,11<br>65,92 | H | 7,60<br>7,86 | N | 8,56<br>8,33 | Cl | 14,45<br>14,09 |

Beispiel 117

3-[(N-(2-(6,7-Dimethoxy-isochinolyl-4)-ethyl)-piperidyl-3)-methyl]-7,8-dimethoxy-2-oxo-1,3-dihydro-2H-3-benzazepin-monohydrat

Hergestellt aus 7,8-Dimethoxy-2-oxo-1,3-dihydro-2H-3-benzazepin und 3-Chlormethyl-N-[2-(6,7-dimethoxy-isochinolyl-4)-ethyl]-piperidin analog Beispiel 2.
Ausbeute: 69 % der Theorie,
Schmelzbereich: 85-96 ° C (amorph)

| | | | | | | |
|---|---|---|---|---|---|---|
| Ber.:<br>Gef.: | C | 67,73<br>67,96 | H | 7,15<br>7,19 | N | 7,64<br>7,75 |

Beispiel 118

3-[3-(N-(3-(Pyridyl-3)-propyl)-piperidyl-3)-propyl]-7,8-dimethoxy-2-oxo-1,3-dihydro-2H-3-benzazepin-dihydrochlorid-monohydrat

Hergestellt aus 7,8-Dimethoxy-2-oxo-1,3-dihydro-2H-3-benzazepin und 3-(3-Chlorpropyl)-N-[3-(pyridyl-3)-propyl]-piperidin analog Beispiel 2.
Ausbeute: 72 % der Theorie,

Schmelzbereich: 94-106 °C (amorph)

| | | | | | | | | |
|---|---|---|---|---|---|---|---|---|
| Ber.:<br>Gef.: | C | 60,64<br>60,80 | H | 7,45<br>7,44 | N | 7,57<br>7,46 | Cl | 12,78<br>12,59 |

Beispiel 119

3-[2-(N-(3-(Pyridyl-4)-propyl)-piperidyl-2)-ethyl]-7,8-methylendioxy-2-oxo-1,3-dihydro-2H-3-benzazepin-dihydrochlorid-dihydrat

Hergestellt aus 7,8-Methylendioxy-2-oxo-1,3-dihydro-2H-3-benzazepin und 2-(2-Chlorethyl)-N-[3-(pyridyl-4)-propyl]-piperidin in Dimethylsulfoxid und Kalium-tert.butylat analog Beispiel 2.
Ausbeute: 67 % der Theorie,
Schmelzbereich: 148-161 °C (amorph)

| | | | | | | | | |
|---|---|---|---|---|---|---|---|---|
| Ber.:<br>Gef.: | C | 57,56<br>57,72 | H | 6,87<br>7,03 | N | 7,74<br>7,61 | Cl | 13,07<br>13,62 |

Beispiel 120

3-[(N-(1-(Pyridyl-4)-methyl)-piperidyl-3)-methyl]-7,8-dimethyl-2-oxo-1,3-dihydro-2H-3-benzazepin-dihydrochlorid-monohydrat

Hergestellt aus 7,8-Dimethoxy-2-oxo-1,3-dihydro-2H-3-benzazepin und 3-Chlormethyl-N-[1-(pyridyl-4)-methyl]-piperidin in Dimethylsulfoxid und Kalium-tert.butylat analog Beispiel 2.
Ausbeute: 75 % der Theorie,
Schmelzbereich: 113-127 °C (amorph)

| | | | | | | | | |
|---|---|---|---|---|---|---|---|---|
| Ber.:<br>Gef.: | C | 61,79<br>61,55 | H | 7,13<br>7,32 | N | 9,01<br>9,04 | Cl | 15,20<br>15,11 |

Beispiel 121

3-[(N-(3-(Pyridyl-4)-propyl)-pyrrolidyl-3)-methyl]-7,8-methylendioxy-2-oxo-1,3,4,5-tetrahydro-2H-3-benzazepin-dihydrochlorid-monohydrat

Hergestellt aus 3-[(N-(3-(Pyridyl-4)-propyl)-pyrrolidyl-3)-methyl]-7,8-methylendioxy-2-oxo-1,3-dihydro-2H-3H-benzazepin und 10%iger Palladium/Kohle und einem Wasserstoffdruck von 5 bar bei Raumtemperatur analog Beispiel 5.
Ausbeute: 71 % der Theorie,
Schmelzbereich: 95-106 °C (amorph)

| | | | | | | | | |
|---|---|---|---|---|---|---|---|---|
| Ber.:<br>Gef.: | C | 57,83<br>57,67 | H | 6,67<br>6,82 | N | 8,43<br>8,27 | Cl | 14,22<br>14,05 |

Beispiel 122

3-[2-(N-(3-(Pyridyl-4)-propyl)-piperidyl-2)-ethyl]-7,8-dimethyl-2-oxo-1,3,4,5-tetrahydro-2H-3-benzazepin-dihydrochlorid-semihydrat

Hergestellt aus 3-[2-(N-(3-(Pyridyl-4)-propyl)-piperidyl-2)-ethyl]-7,8-dimethyl-2-oxo-1,3-dihydro-2H-3-benzazepin und 10%iger Palladium/Kohle und einem Wasserstoffdruck von 5 bar bei 80 ° C analog Beispiel 5.
Ausbeute: 73 % der Theorie,
Schmelzpunkt: 236-238 ° C

| | | | | | | | | |
|---|---|---|---|---|---|---|---|---|
| Ber.:<br>Gef.: | C | 64,65<br>64,91 | H | 8,04<br>8,02 | N | 8,37<br>8,25 | Cl | 14,14<br>13,92 |

Beispiel 123

3-[3-(N-(2-(2-Methyl-pyridyl-6)-ethyl)-piperidyl-3)-propyl]-7,8-dimethoxy-2-oxo-1,3,4,5-tetrahydro-2H-3-benzazepin-dihydrochlorid-monohydrat

Hergestellt aus 3-[3-(Piperidyl-3)-propyl)-7,8-dimethoxy-2-oxo-1,3,4,5-tetrahydro-2H-3-benzazepin und 2-Benzolsulfonsäure-(6-methyl-pyridyl-2)-ethylester in Dimethylsulfoxid mit Kaliumcarbonat bei 120 ° C analog Beispiel 1.
Ausbeute: 29 % der Theorie,
Schmelzbereich: 105-113 ° C (amorph)

| | | | | | | |
|---|---|---|---|---|---|---|
| Ber.:<br>Gef.: | C | 60,42<br>60,68 | H | 7,78<br>7,50 | N | 7,55<br>7,42 |

Beispiel 124

3-[(N-(3-(Pyridyl-3)-propyl)-piperidyl-3)-methyl]-7,8-dimethoxy-2-oxo-1,3,4,5-tetrahydro-2H-3-benzazepin-dihydrochlorid

Hergestellt aus 3-[(N-(3-Pyridyl-3)-propyl)-piperidyl-3)-methyl]-7,8-dimethoxy-2-oxo-1,3-dihydro-2H-3-benzazepin durch Hydrierung bei 5 bar Wasserstoffdruck in Ethanol mit 10%iger Palladium/Kohle bei 70 ° C analog Beispiel 5.
Ausbeute: 52 % der Theorie,
Schmelzbereich: 113-122 ° C (amorph)

| | | | | | | |
|---|---|---|---|---|---|---|
| Ber.:<br>Gef.: | C | 61,17<br>61,31 | H | 7,30<br>7,50 | N | 8,23<br>8,28 |

Beispiel 125

3-[(N-(2-(6,7-Dimethoxy-isochinolyl-4)-ethyl)-piperidyl-3)-methyl]-7,8-dimethoxy-2-oxo-1,3,4,5-tetrahydro-2H-3-benzazepin x 1/2 $H_2O$

Hergestellt aus 3-[(N-(2-(6,7-Dimethoxy-isochinolyl-4)-ethyl)-piperidyl-3)-methyl]-7,8-dimethoxy-2-oxo-1,3-dihydro-2H-3-benzazepin durch Hydrierung bei 5 bar Wasserstoffdruck mit 10%iger Palladium/Kohle in Ethanol bei 70 ° C analog Beispiel 5.
Ausbeute: 50 % der Theorie,
Schmelzbereich: 82-86 ° C (amorph)

| | | | | | | |
|---|---|---|---|---|---|---|
| Ber.:<br>Gef.: | C | 66,40<br>66,26 | H | 7,55<br>7,50 | N | 7,48<br>7,59 |

Beispiel 126

3-[3-(N-(3-(Pyridyl-3)-propyl)-piperidyl-3)-propyl]-7,8-dimethoxy-2-oxo-1,3,4,5-tetrahydro-2H-3-benzazepin-dihydro-chlorid-dihydrat

Hergestellt aus 3-[3-(N-(3-(Pyridyl-3)-propyl)-piperidyl-3)-propyl]-7,8-dimethoxy-2-oxo-1,3-dihydro-2H-3-benzazepin durch Hydrierung bei 5 bar Wasserstoffdruck mit 10%iger Palladium/Kohle in Ethanol bei 70 °C analog Beispiel 5.
Ausbeute: 64 % der Theorie,
Schmelzbereich: 106-115 °C (amorph)

| | | | | | | | | |
|---|---|---|---|---|---|---|---|---|
| Ber.:<br>Gef.: | C | 58,53<br>58,46 | H | 7,89<br>7,61 | N | 7,31<br>7,14 | Cl | 12,34<br>12,57 |

Beispiel 127

3-[(N-(3-(Pyridyl-3)-propyl)-piperidyl-3)-methyl]-7,8-methylendioxy-2-oxo-1,3,4,5-tetrahydro-2H-3-benzazepin-dihydro-chlorid-monohydrat

Hergestellt aus 3-[(N-(3-(Pyridyl-3)-propyl)-piperidyl-3)-methyl]-7,8-methylendioxy-2-oxo-1,3-dihydro-2H-3-benzazepin bei 5 bar Wasserstoffdruck mit 10%iger Palladium/Kohle in Ethanol bei 80 °C analog Beispiel 5.
Ausbeute: 81 % der Theorie,
Schmelzbereich: 126-138 °C (amorph)

| | | | | | | | | |
|---|---|---|---|---|---|---|---|---|
| Ber.:<br>Gef.: | C | 58,58<br>58,43 | H | 6,88<br>7,00 | N | 8,19<br>7,85 | Cl | 13,83<br>13,71 |

Beispiel 128

3-[2-(N-(3-(Pyridyl-4)-propyl)-piperidyl-2)-ethyl]-7,8-methylendioxy-2-oxo-1,3,4,5-tetrahydro-2H-3-benzazepin-dihydrochlorid-monohydrat

Hergestellt aus 3-[2-(N-(3-(Pyridyl-4)-propyl)-piperidyl-2)-ethyl]-7,8-methylendioxy-2-oxo-1,3-dihydro-2H-3-benzazepin bei 5 bar Wasserstoffdruck mit 10%iger Palladium/Kohle in Ethanol bei 80 °C analog Beispiel 5.
Ausbeute: 74 % der Theorie,
Schmelzbereich: 132-146 °C (amorph)

| | | | | | | | | |
|---|---|---|---|---|---|---|---|---|
| Ber.:<br>Gef.: | C | 59,31<br>59,18 | H | 7,08<br>7,41 | N | 7,98<br>7,80 | Cl | 13,46<br>13,25 |

Beispiel 129

3-[(N-(3-(Pyridyl-3)-propyl)-piperidyl-3)-methyl]-7,8-dimethyl-2-oxo-1,3,4,5-tetrahydro-2H-3-benzazepin-dihydrochlorid

Hergestellt aus 3-[(N-(3-(Pyridyl-3)-propyl)-piperidyl-3)-methyl]-7,8-dimethyl-2-oxo-1,3-dihydro-2H-3-benzazepin und 5 bar Wasserstoffdruck mit 10%iger Palladium/Kohle in Ethanol bei 80 °C analog Beispiel 5.
Ausbeute: 54 % der Theorie,
Schmelzbereich: 92-105 °C (amorph)

| | | | | | | | | |
|---|---|---|---|---|---|---|---|---|
| Ber.:<br>Gef.: | C | 62,90<br>63,19 | H | 7,92<br>7,90 | N | 8,46<br>8,45 | Cl | 14,28<br>14,30 |

Beispiel 130

3-[(N-(3-(Pyridyl-4)-propyl)-pyrrolidyl-3)-methyl]-7,8-methylendioxy-1,3,4,5-tetrahydro-2H-3-benzazepin-trihydrochlorid-monohydrat

Hergestellt aus 3-[(N-(3-(Pyridyl-4)-propyl)-pyrrolidyl-3)-methyl]-7,8-methylendioxy-2-oxo-1,3,4,5-tetrahydro-2H-3-benzazepin und Lithiumaluminiumhydrid in Tetrahydrofuran/Ether analog Beispiel 3.
Ausbeute: 73 % der Theorie,
Schmelzbereich: 96-108 °C (amorph)

| | | | | | | | | |
|---|---|---|---|---|---|---|---|---|
| Ber.:<br>Gef.: | C | 55,33<br>55,06 | H | 6,97<br>7,28 | N | 8,06<br>7,77 | Cl | 20,42<br>20,07 |

Beispiel 131

3-[(N-(3-(Pyridyl-3)-propyl)-piperidyl-3)-methyl]-7,8-dimethoxy-1,3,4,5-tetrahydro-2H-3-benzazepin-dihydrochlorid

Hergestellt aus 3-[(N-(3-(Pyridyl-3)-propyl)-piperidyl-3)-methyl]-7,8-dimethoxy-2-oxo-1,3,4,5-tetrahydro-2H-3-benzazepin und Lithiumaluminiumhydrid in Tetrahydrofuran/Ether analog Beispiel 3.
Ausbeute: 71 % der Theorie,
Schmelzpunkt: 208-210 °C

| | | | | | | | | |
|---|---|---|---|---|---|---|---|---|
| Ber.:<br>Gef.: | C | 62,89<br>62,70 | H | 7,91<br>7,53 | N | 8,46<br>8,22 | Cl | 14,28<br>14,50 |

Beispiel 132

3-[2-(N-(3-(Pyridyl-4)-propyl)-piperidyl-2)-ethyl]-7,8-methylendioxy-1,3,4,5-tetrahydro-2H-3-benzazepin-trihydrochlorid

Hergestellt aus 3-[2-(N-(3-(Pyridyl-4)-propyl)-piperidyl-2)-ethyl]-7,8-methylendioxy-2-oxo-1,3,4,5-tetrahydro-2H-3-benzazepin und Lithiumaluminiumhydrid in Tetrahydrofuran/Ether analog Beispiel 3.
Ausbeute: 63 % der Theorie,
Schmelzbereich: 123-136 °C (amorph)

| | | | | | | | | |
|---|---|---|---|---|---|---|---|---|
| Ber.:<br>Gef.: | C | 58,81<br>58,51 | H | 7,21<br>7,41 | N | 7,91<br>7,92 | Cl | 20,00<br>19,86 |

Beispiel 133

3-[(N-(3-(Pyridyl-3)-propyl)-piperidyl-3)-methyl]-7,8-methylendioxy-1,3,4,5-tetrahydro-2H-3-benzazepin-trihydrochlorid-semihydrat

Hergestellt aus 3-[(N-(3-(Pyridyl-3)-propyl)-piperidyl-3)-methyl]-7,8-methylendioxy-2-oxo-1,3,4,5-tetrahydro-2H-3-benzazepin und Lithiumaluminiumhydrid in Tetrahydrofuran/Ether analog Beispiel 3.
Ausbeute: 75 % der Theorie,
Schmelzbereich: 126-138 °C (amorph)

| | | | | | | | | |
|---|---|---|---|---|---|---|---|---|
| Ber.:<br>Gef.: | C | 57,09<br>57,05 | H | 7,10<br>7,32 | N | 7,99<br>8,05 | Cl | 20,22<br>20,37 |

Beispiel 134

3-[2-(N-(3-(Pyridyl-4)-propyl)-piperidyl-2)-ethyl]-7,8-dimethyl-1,3,4,5-tetrahydro-2H-3-benzazepin-trihydrochlorid

Hergestellt aus 3-[2-(N-(3-(Pyridyl-4)-propyl)-piperidyl-2)-ethyl]-7,8-dimethyl-2-oxo-1,3,4,5-tetrahydro-2H-3-benzazepin und Lithiumaluminiumhydrid in Tetrahydrofuran/Ether analog Beispiel 3.
Ausbeute: 92 % der Theorie,
Schmelzpunkt: 180-182 °C

| | | | | | | | | |
|---|---|---|---|---|---|---|---|---|
| Ber.:<br>Gef.: | C | 62,96<br>63,00 | H | 8,22<br>8,29 | N | 8,16<br>8,16 | Cl | 20,65<br>20,34 |

Beispiel 135

3-[(N-(3-(Pyridyl-3)-propyl)-piperidyl-3)-methyl]-7,8-dimethyl-1,3,4,5-tetrahydro-2H-3-benzazepin-trihydrochlorid

Hergestellt aus 3-[(N-(3-(Pyridyl-3)-propyl)-piperidyl-3)-methyl]-7,8-dimethyl-2-oxo-1,3,4,5-tetrahydro-2H-3-benzazepin und Lithiumaluminiumhydrid in Tetrahydrofuran/Ether analog Beispiel 3.
Ausbeute: 81 % der Theorie,
Schmelzbereich: 184-196 °C (amorph)

| | | | | | | | | |
|---|---|---|---|---|---|---|---|---|
| Ber.:<br>Gef.: | C | 60,17<br>60,28 | H | 8,16<br>8,25 | N | 8,10<br>8,00 | Cl | 20,49<br>20,39 |

Beispiel 136

3-[2-(N-(2-(6-Methoxy-naphthyl-2)-ethyl)-piperidyl-2)-ethyl]-7,8-dimethoxy-2-oxo-1,3,4,5-tetrahydro-2H-3-benzazepin-hydrochlorid

Hergestellt aus 3-[2-(Piperidyl-2)-ethyl]-7,8-dimethoxy-2-oxo-1,3,4,5-tetrahydro-2H-3-benzazepin und 2-(6-Methoxy-naphthyl-2)-ethylbromid analog Beispiel 1.
Ausbeute: 20 % der Theorie,

Schmelzpunkt: 158-160 ° C

| | | | | | | | | |
|---|---|---|---|---|---|---|---|---|
| Ber.:<br>Gef.: | C | 69,48<br>69,40 | H | 7,47<br>7,56 | N | 5,06<br>5,17 | Cl | 6,41<br>6,62 |

Beispiel 137

3-[2-(N-(2-(5-Methyl-6-methoxy-naphthyl-2)-ethyl)-piperidyl-2)-ethyl]-7,8-dimethoxy-2-oxo-1,3,4,5-tetrahydro-2H-3-benzazepin-hydrochlorid

Hergestellt aus 3-[2-(Piperidyl-2)-ethyl]-7,8-dimethoxy-2-oxo-1,3,4,5-tetrahydro-2H-3-benzazepin und 2-(5-Methyl-6-methoxy-naphthyl-2)-ethylbromid analog Beispiel 1.
Ausbeute: 28 % der Theorie,
Schmelzpunkt: 140-143 ° C

| | | | | | | | | |
|---|---|---|---|---|---|---|---|---|
| Ber.:<br>Gef.: | C | 69,88<br>69,06 | H | 7,64<br>7,57 | N | 4,94<br>4,84 | Cl | 6,25<br>6,44 |

Beispiel 138

3-[2-(N-(2-(Naphthyl-1-oxy)-ethyl)-piperidyl-2)-ethyl]-7,8-dimethoxy-2-oxo-1,3,4,5-tetrahydro-2H-3-benzazepin-hydrochlorid

Hergestellt aus 3-[2-(Piperidyl-2)-ethyl]-7,8-dimethoxy-2-oxo-1,3,4,5-tetrahydro-2H-3-benzazepin und 2-(Naphthyl-1-oxy)-ethylbromid analog Beispiel 1.
Ausbeute: 27 % der Theorie,
Schmelzpunkt: 146-148 ° C

| | | | | | | | | |
|---|---|---|---|---|---|---|---|---|
| Ber.:<br>Gef.: | C | 69,06<br>69,00 | H | 7,29<br>7,07 | N | 5,20<br>5,31 | Cl | 6,58<br>6,68 |

Beispiel 139

3-[2-(N-(2-(Naphthyl-1)-ethyl)-piperidyl-2)-ethyl]-7,8-dimethoxy-2-oxo-1,3,4,5-tetrahydro-2H-3-benzazepin-hydrochlorid

Hergestellt aus 3-[2-(Piperidyl-2)-ethyl]-7,8-dimethoxy-2-oxo-1,3,4,5-tetrahydro-2H-3-benzazepin und 2-(Naphthyl-1)-ethylbromid analog Beispiel 1.
Ausbeute: 24 % der Theorie,
Schmelzpunkt: 148-150 ° C

| | | | | | | | | |
|---|---|---|---|---|---|---|---|---|
| Ber.:<br>Gef.: | C | 71,18<br>70,92 | H | 7,51<br>7,44 | N | 5,36<br>5,57 | Cl | 6,78<br>7,06 |

Beispiel 140

3-[2-(N-(4-(Naphthyl-2-oxy)-butyl)-piperidyl-2)-ethyl]-7,8-dimethoxy-2-oxo-1,3,4,5-tetrahydro-2H-benzazepin-hydrochlorid

Hergestellt aus 3-[2-(Piperidyl-2)-ethyl]-7,8-dimethoxy-2-oxo-1,3,4,5-tetrahydro-2H-3-benzazepin und 4-(Naphthyl-2-oxy)-butylbromid analog Beispiel 1.
Ausbeute: 39 % der Theorie,
Schmelzpunkt: 112-114 °C

| | | | | | | | | |
|---|---|---|---|---|---|---|---|---|
| Ber.:<br>Gef.: | C | 69,88<br>69,69 | H | 7,64<br>7,58 | N | 4,64<br>4,82 | Cl | 6,25<br>6,52 |

Beispiel 141

3-[2-(N-(2-(Naphthyl-2)-ethyl)-piperidyl-2)-ethyl]-7,8-dimethoxy-2-oxo-1,3,4,5-tetrahydro-2H-3-benzazepin-hydrochlorid

Hergestellt aus 3-[2-(Piperidyl-2)-ethyl]-7,8-dimethoxy-2-oxo-1,3,4,5-tetrahydro-2H-3-benzazepin und 2-(Naphthyl-2)-ethylbromid analog Beispiel 1.
Ausbeute: 41 % der Theorie,
Schmelzpunkt: 120-122 °C

| | | | | | | | | |
|---|---|---|---|---|---|---|---|---|
| Ber.:<br>Gef.: | C | 71,18<br>71,10 | H | 7,51<br>7,31 | N | 5,36<br>5,40 | Cl | 6,78<br>7,05 |

Beispiel 142

3-[2-(N-((2-Methyl-naphthyl-1)-methyl)-piperidyl-2)-ethyl]-7,8-dimethoxy-2-oxo-1,3,4,5-tetrahydro-2H-3-benzazepin-hydrochlorid

Hergestellt aus 3-[2-(Piperidyl-2)-ethyl]-7,8-dimethoxy-2-oxo-1,3,4,5-tetrahydro-2H-3-benzazepin und 1-Chlormethyl-2-methyl-naphthalin analog Beispiel 1.
Ausbeute: 24 % der Theorie,
Schmelzpunkt: 144-146 °C

| | | | | | | | | |
|---|---|---|---|---|---|---|---|---|
| Ber.:<br>Gef.: | C | 71,18<br>70,93 | H | 7,51<br>7,38 | N | 5,36<br>5,48 | Cl | 6,78<br>6,89 |

Beispiel 143

3-[(N-(2-(Naphthyl-2)-ethyl)-hexahydroazepin-3-yl)-methyl]-7,8-dimethoxy-2-oxo-1,3,4,5-tetrahydro-2H-3-benzazepin-hydrochlorid

Hergestellt aus 3-[(Hexahydro-azepin-3-yl)-methyl]-7,8-dimethoxy-2-oxo-1,3,4,5-tetrahydro-2H-3-benzazepin und 2-(Naphthyl-2)-ethylbromid analog Beispiel 1.
Ausbeute: 58 % der Theorie,
Schmelzpunkt: 204-205 °C

| | | | | | | | | |
|---|---|---|---|---|---|---|---|---|
| Ber.:<br>Gef.: | C | 71,18<br>71,41 | H | 7,52<br>7,51 | N | 5,36<br>5,35 | Cl | 6,78<br>6,50 |

Beispiel 144

3-[(N-(2-(Naphthyl-2)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 2-(Naphthyl-2)-ethylbromid analog Beispiel 1.
Ausbeute: 35 % der Theorie,
Schmelzpunkt: 239-240 ° C

| | | | | | | | | |
|---|---|---|---|---|---|---|---|---|
| Ber.:<br>Gef.: | C | 70,78<br>70,70 | H | 7,33<br>7,10 | N | 5,50<br>5,46 | Cl | 6,96<br>7,16 |

Beispiel 145

3-[(N-((Naphthyl-2)-methyl)-piperidyl-3)-methyl]-7,8-dimethoxy-2-oxo-1,3,4,5-tetrahydro-2H-3-benzazepin

Hergestellt aus 3-[(Piperidyl-3)-methyl]-7,8-dimethoxy-2-oxo-1,3,4,5-tetrahydro-2H-3-benzazepin und 2-Brommethyl-naphthalin analog Beispiel 1.
Ausbeute: 27 % der Theorie,
Schmelzpunkt: 176-177 ° C

| | | | | | | |
|---|---|---|---|---|---|---|
| Ber.:<br>Gef.: | C | 75,95<br>76,11 | H | 7,47<br>7,28 | N | 6,11<br>6,10 |

Beispiel 146

3-[(N-(4-(Naphthyl-2-oxy)-butyl)-piperidyl-3)-methyl)-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 4-(Naphthyl-2-oxy)-butylbromid analog Beispiel 1.
Ausbeute: 24 % der Theorie,
Schmelzpunkt: 196-197 ° C

| | | | | | | | | |
|---|---|---|---|---|---|---|---|---|
| Ber.:<br>Gef.: | C | 69,48<br>69,30 | H | 7,47<br>7,36 | N | 5,06<br>4,99 | Cl | 6,41<br>6,56 |

Beispiel 147

3-[(N-(4-(Naphthyl-1)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 2-(Naphthyl-1)-ethylbromid analog Beispiel 1.
Ausbeute: 18 % der Theorie,
Schmelzpunkt: 230-231 ° C

| | | | | | | | | |
|---|---|---|---|---|---|---|---|---|
| Ber.:<br>Gef.: | C | 70,78<br>70,71 | H | 7,33<br>7,07 | N | 5,50<br>5,67 | Cl | 6,96<br>6,99 |

Beispiel 148

3-[(N-(2-(Naphthyl-1-oxy)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 2-(Naphthyl-1-oxy)-ethylbromid analog Beispiel 1.
Ausbeute: 40 % der Theorie,
Schmelzpunkt: 214-215 °C

| | | | | | | | | |
|---|---|---|---|---|---|---|---|---|
| Ber.:<br>Gef.: | C | 68,62<br>68,40 | H | 7,10<br>7,10 | N | 5,34<br>5,21 | Cl | 6,75<br>6,77 |

Beispiel 149

3-[(N-((2-Methyl-naphthyl-1)-methyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 1-Chlormethyl-2-methyl-naphthalin analog Beispiel 1.
Ausbeute: 67 % der Theorie,
Schmelzpunkt: 242-243 °C

| | | | | | | | | |
|---|---|---|---|---|---|---|---|---|
| Ber.:<br>Gef.: | C | 70,78<br>70,50 | H | 7,33<br>7,22 | N | 5,50<br>5,34 | Cl | 6,96<br>6,89 |

Beispiel 150

3-[(N-(2-(6-Methoxy-naphthyl-2)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 2-(6-Methoxy-naphthyl-2)-ethylbromid analog Beispiel 1.
Ausbeute: 50 % der Theorie,
Schmelzpunkt: 156-157 °C

| | | | | | | |
|---|---|---|---|---|---|---|
| Ber.:<br>Gef.: | C | 74,07<br>73,90 | H | 7,62<br>7,55 | N | 5,57<br>5,64 |

Beispiel 151

3-[(N-(2-(5-Methyl-6-methoxy-naphth-2-yl)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 2-(5-Methyl-6-methoxy-naphth-2-yl)-ethylbromid analog Beispiel 1.
Ausbeute: 53 % der Theorie,

Schmelzpunkt: 240-241 ° C

| | | | | | | | | |
|---|---|---|---|---|---|---|---|---|
| Ber.:<br>Gef.: | C | 69,48<br>69,58 | H | 7,47<br>7,48 | N | 5,06<br>5,00 | Cl | 6,41<br>6,54 |

Beispiel 152

2-[N-(2-(3,4-Dimethoxy-phenyl)-ethyl)-piperidyl-3)-methyl]-5,6-dimethoxy-1-oxo-1,3-dihydro-isoindol-hydrochlorid

Hergestellt aus 2-[(Piperidyl-3)-methyl]-5,6-dimethoxy-1,3-dihydro-isoindol und 2-(3,4-Dimethoxy-phenyl)-ethylbromid analog Beispiel 1.
Ausbeute: 68 % der Theorie,
Schmelzpunkt: 225-226 ° C

| | | | | | | | | |
|---|---|---|---|---|---|---|---|---|
| Ber.:<br>Gef.: | C | 63,60<br>63,61 | H | 7,18<br>7,30 | N | 5,20<br>5,70 | Cl | 7,22<br>7,44 |

Beispiel 153

2-[(N-(2-(6-Methoxy-naphthyl-2)-ethyl)-piperidyl-3)-methyl]-5,6-dimethoxy-1-oxo-1,3-dihydro-isoindol-hydrochlorid

Hergestellt aus 2-[(Piperidyl-3)-methyl]-5,6-dimethoxy-1-oxo-1,3-dihydro-isoindol und 2-(6-Methoxy-naphthyl-2)-ethylbromid analog Beispiel 1.
Ausbeute: 75 % der Theorie,
Schmelzpunkt: 234-236 ° C

| | | | | | | | | |
|---|---|---|---|---|---|---|---|---|
| Ber.:<br>Gef.: | C | 68,16<br>68,10 | H | 6,90<br>7,10 | N | 5,48<br>5,39 | Cl | 6,94<br>7,10 |

Beispiel 154

2-[(N-(2-(Naphthyl-1-oxy)-ethyl)-piperidyl-3)-methyl]-5,6-dimethoxy-1-oxo-1,3-dihydro-isoindol-hydrochlorid

Hergestellt aus 2-[(Piperidyl-3)-methyl]-5,6-dimethoxy-1-oxo-1,3-dihydro-isoindol und 2-(Naphthyl-1-oxy)-ethylbromid analog Beispiel 1.
Ausbeute: 60 % der Theorie,
Schmelzpunkt: 150-152 ° C

| | | | | | | | | |
|---|---|---|---|---|---|---|---|---|
| Ber.:<br>Gef.: | C | 67,66<br>67,50 | H | 6,69<br>6,76 | N | 5,63<br>5,74 | Cl | 7,13<br>7,54 |

Beispiel 155

2-[(N-(2-(4-Methyl-phenyl)-ethyl)-piperidyl-3)-methyl]-5,6-dimethoxy-1-oxo-1,3-dihydro-isoindol

Hergestellt aus 2-[(Piperidyl-3)-methyl]-5,6-dimethoxy-1-oxo-isoindol und 2-(4-Methyl-phenyl)-ethylbromid analog Beispiel 1.
Ausbeute: 57 % der Theorie,

Schmelzpunkt: 134-136 ° C

| | | | | | | |
|---|---|---|---|---|---|---|
| Ber.:<br>Gef.: | C | 73,50<br>73,40 | H | 7,90<br>8,04 | N | 6,86<br>7,06 |

Beispiel 156

2-[(N-(2-(3-Methoxy-phenyl)-ethyl)-piperidyl-3)-methyl]-5,6-dimethoxy-1-oxo-1,3-dihydro-isoindol-hydrochlorid

Hergestellt aus 2-[(Piperidyl-3)-methyl]-5,6-dimethoxy-1-oxo-isoindol und 2-(3-Methoxy-phenyl)-ethylbromid analog Beispiel 1.
Ausbeute: 54 % der Theorie,
Schmelzpunkt: 226-228 ° C

| | | | | | | | | |
|---|---|---|---|---|---|---|---|---|
| Ber.:<br>Gef.: | C | 65,28<br>65,30 | H | 7,01<br>7,37 | N | 6,09<br>5,91 | Cl | 7,71<br>7,61 |

Beispiel 157

2-[(N-(2-(5-Methyl-6-methoxy-naphthyl-2)-ethyl)-piperidyl-3)-methyl]-5,6-dimethoxy-1-oxo-1,3-dihydro-isoindol-hydrochlorid

Hergestellt aus 2-[(Piperidyl-3)-methyl]-5,6-dimethoxy-1-oxo-1,3-dihydro-isoindol und 2-(5-Methyl-6-methoxy-naphthyl-2)-ethylbromid analog Beispiel 1.
Ausbeute: 38 % der Theorie,
Schmelzpunkt: 214-216 ° C

| | | | | | | | | |
|---|---|---|---|---|---|---|---|---|
| Ber.:<br>Gef.: | C | 68,62<br>68,94 | H | 7,10<br>7,23 | N | 5,33<br>4,98 | Cl | 6,75<br>6,61 |

Beispiel 158

2-[(N-(2-(4-Nitro-phenyl)-ethyl)-piperidyl-3)-methyl]-5,6-dimethoxy-1-oxo-1,3-dihydro-isoindol-hydrochlorid

Hergestellt aus 2-[(Piperidyl-3)-methyl]-5,6-dimethoxy-1-oxo-1,3-dihydro-isoindol und 2-(4-Nitro-phenyl)-ethylbromid analog Beispiel 1.
Ausbeute: 79 % der Theorie,
Schmelzpunkt: 215-218 ° C

| | | | | | | | | |
|---|---|---|---|---|---|---|---|---|
| Ber.:<br>Gef.: | C | 60,56<br>60,41 | H | 6,35<br>6,26 | N | 8,83<br>8,84 | Cl | 7,45<br>7,62 |

Beispiel 159

3-[(N-(2-(Thienyl-2)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 2-(2-Brom-ethyl)-thiophen analog Beispiel 1.

Ausbeute: 43 % der Theorie,
Schmelzpunkt: 232-236 °C

| | | | | | | | | | | |
|---|---|---|---|---|---|---|---|---|---|---|
| Ber.:<br>Gef.: | C | 61,99<br>61,90 | H | 7,15<br>7,06 | N | 6,02<br>5,78 | Cl | 7,62<br>7,96 | S | 6,89<br>6,84 |

Beispiel 160

3-[(N-(2-(Thienyl-3)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 3-(2-Brom-ethyl)-thiophen analog Beispiel 1.
Ausbeute: 36 % der Theorie,
Schmelzpunkt: sintert bei 75-80 °, schmilzt bei 225-230 °C

| | | | | | | | | | | |
|---|---|---|---|---|---|---|---|---|---|---|
| Ber.:<br>Gef.: | C | 61,99<br>62,00 | H | 7,15<br>7,08 | N | 6,02<br>5,98 | Cl | 7,62<br>8,43 | S | 6,89<br>6,62 |

Beispiel 161

3-[(N-(4-(Thienyl-2)-butyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 2-(4-Brom-butyl)-thiophen analog Beispiel 1.
Ausbeute: 68 % der Theorie,
Schmelzbereich: 190-196 °C

| | | | | | | | | | | |
|---|---|---|---|---|---|---|---|---|---|---|
| Ber.:<br>Gef.: | C | 63,33<br>63,18 | H | 7,56<br>7,72 | N | 5,68<br>5,72 | Cl | 7,19<br>7,29 | S | 6,50<br>6,59 |

Beispiel 162

3-[(N-(2-(Benzo[b]furyl-2)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 2-(2-Brom-ethyl)-benzo[b]furan analog Beispiel 1.
Ausbeute: 22 % der Theorie,
Schmelzpunkt: oberhalb 216 °C (Zers.)

| | | | | | | |
|---|---|---|---|---|---|---|
| Ber.:<br>Gef.: | C | 67,39<br>67,14 | H | 7,07<br>7,36 | N | 5,61<br>5,53 |

Beispiel 163

3-[(N-(2-(Benzo[b]thienyl-3)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 3-(2-Brom-ethyl)-benzo[b]thiophen analog Beispiel 1.
Ausbeute: 73 % der Theorie,
Schmelzbereich: 70-75 ° C (Zers.)

| | | | | | | |
|---|---|---|---|---|---|---|
| Ber.:<br>Gef.: | C | 65,29<br>65,10 | H | 6,85<br>6,87 | N | 5,44<br>5,73 |

Beispiel 164

3-[(N-(2-(4-Methoxy-benzo[b]thienyl-3)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 3-(2-Chlorethyl)-4-methoxy-benzo[b]thiophen analog Beispiel 1.
Ausbeute: 25 % der Theorie,
Schmelzbereich: 85-105 (Zers.)

| | | | | | | | | | | |
|---|---|---|---|---|---|---|---|---|---|---|
| Ber.:<br>Gef.: | C | 63,96<br>63,95 | H | 6,84<br>6,85 | N | 5,14<br>4,99 | Cl | 6,50<br>6,53 | S | 5,88<br>5,75 |

Beispiel 165

3-[(N-(2-(6-Methylsulfonyloxy-benzo[b]thienyl-3)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 3-[2-(Methylsulfonyloxy)-ethyl]-6-methylsulfonyloxy-benzo[b]thiophen analog Beispiel 1.
Ausbeute: 55 % der Theorie,
Schmelzpunkt: 90 ° C (Zers.)

| | | | | | | | | | | |
|---|---|---|---|---|---|---|---|---|---|---|
| Ber.:<br>Gef.: | C | 57,18<br>57,25 | H | 6,12<br>6,14 | N | 4,60<br>4,50 | Cl | 5,82<br>5,97 | S | 10,53<br>10,36 |

Beispiel 166

3-[(N-(5-(Thienyl-2)-pentyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 2-(5-Methylsulfonyloxy-pentyl)-thiophen analog Beispiel 1.
Ausbeute: 39 % der Theorie,
Schmelzpunkt: 177 ° C

| | | | | | | | | | | |
|---|---|---|---|---|---|---|---|---|---|---|
| Ber.:<br>Gef.: | C | 63 95<br>63,70 | H | 7,75<br>7,92 | N | 5,52<br>5,40 | Cl | 6,99<br>7,24 | S | 6,32<br>6,62 |

Beispiel 167

3-[(N-(2-(Furyl-2)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 2-(2-Methylsulfonyloxy-ethyl)-furan analog Beispiel 1.
Ausbeute: 44 % der Theorie,
Schmelzbereich: 205-215 ° C

| | | | | | | | | |
|---|---|---|---|---|---|---|---|---|
| Ber.:<br>Gef.: | C | 64 20<br>64,00 | H | 7,41<br>7,45 | N | 6,24<br>6,00 | Cl | 7,90<br>7,80 |

Beispiel 168

3-[(N-(3-(Furyl-2)-propyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 3-(Furyl-2)-propionaldehyd analog Beispiel 9.
Ausbeute: 11 % der Theorie,
Schmelzpunkt: 201-206 ° C

| | | | | | | | | |
|---|---|---|---|---|---|---|---|---|
| Ber.:<br>Gef.: | C | 64 85<br>64,88 | H | 7,62<br>7,76 | N | 6,05<br>5,93 | Cl | 7,66<br>7,55 |

Beispiel 169

3-[(N-(6-(Thienyl-2)-hexyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 2-(6-Methylsulfonyloxy-hexyl)-thiophen analog Beispiel 1.
Ausbeute: 27 % der Theorie,
Schmelzpunkt: 160 ° C

| | | | | | | | | |
|---|---|---|---|---|---|---|---|---|
| Ber.:<br>Gef.: | C | 64,39<br>64,55 | H | 8,10<br>7,90 | N | 5,40<br>5,23 | Cl | 6,79<br>7,00 |

Beispiel 170

3-[(N-(3-(Indolyl-3)-propyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 3-(3-Methylsulfonyloxy-propyl)-indol analog Beispiel 1.
Ausbeute: 19 % der Theorie,

Schmelzpunkt: > 80 ° C (Zers.)

| | | | | | | |
|---|---|---|---|---|---|---|
| Ber.:<br>Gef.: | C | 60,42<br>60,32 | H | 6,30<br>6,57 | N | 6,45<br>6,67 |

Beispiel 171

3-[(N-(2-(Indolyl-3)-ethyl)-piperidyl-3)-methyl]-7,8-dimethoxy-2-oxo-1,3,4,5-tetrahydro-2H-3-benzazepin-hydrochlorid

Hergestellt aus 3-[(Piperidyl-3)-methyl]-7,8-dimethoxy-2-oxo-1,3,4,5-tetrahydro-2H-3-benzazepin und 3-(2-Methylsulfonyloxy-ethyl)-indol analog Beispiel 1.
Ausbeute: 23 % der Theorie,
Schmelzpunkt: > 80 ° C (Zers.)

| | | | | | | |
|---|---|---|---|---|---|---|
| Ber.:<br>Gef.: | C | 65,53<br>65,33 | H | 6,42<br>6,55 | N | 7,69<br>7,80 |

Beispiel 172

2-[(N-(3-(Naphthyl-2-oxy)-propyl)-hexahydro-azepinyl-3)-methyl]-6,7-methylendioxy-1-oxo-1,2,3,4-tetrahydro-isochinolin

Hergestellt aus 6,7-Methylendioxy-1-oxo-1,2,3,4-tetrahydro-isochinolin und 3-Chlormethyl-[N-(3-naphthyl-2-oxy)-propyl]-hexahydro-azepin analog Beispiel 2.
Ausbeute: 21 % der Theorie,
Schmelzpunkt: 74-76 ° C

| | | | | | | | | |
|---|---|---|---|---|---|---|---|---|
| Ber. (x 2 $H_2O$):<br>Gef.: | C | 64,45<br>64,32 | H | 7,02<br>7,20 | N | 5,01<br>5,28 | Cl | 6,34<br>6,44 |

Beispiel 173

2-[(N-)3-(Naphthyl-2)-propyl)-pyrrolidyl-3)-methyl]-6,7-dimethyl-1-oxo-1,2,3,4-tetrahydro-isochinolin

Hergestellt aus 6,7-Dimethyl-1-oxo-1,2,3,4-tetrahydro-isochinolin und 3-(p-Toluolsulfonyloxymethyl)-N-[3-(naphthyl-2)-propyl]-pyrrolidin analog Beispiel 2.
Ausbeute: 20 % der Theorie,
Schmelzpunkt: 72-76 ° C

| | | | | | | | | |
|---|---|---|---|---|---|---|---|---|
| Ber. (x $H_2O$):<br>Gef.: | C | 72,41<br>72,27 | H | 7,75<br>7,85 | N | 5,82<br>5,70 | Cl | 7,36<br>7,96 |

Beispiel 174

2-[(N-(3-(5,6-Dimethoxy-naphthyl-2-oxy)-propyl)-pyrrolidyl-3)-methyl]-6,7-dimethoxy-1-oxo-1,2,3,4-tetrahydro-isochinolin-hydrochlorid

Hergestellt aus 6,7-Dimethoxy-1-oxo-1,3,4,5-tetrahydro-isochinolin und 3-(p-Toluolsulfonyloxymethyl)-N-[3-(5,6-dimethoxy-naphthyl-2-oxy)-propyl]-pyrrolidin analog Beispiel 2.

Ausbeute: 9,5 % der Theorie,
Schmelzpunkt: 60-63 ° C

| | | | | | | | | |
|---|---|---|---|---|---|---|---|---|
| Ber. (x $H_2O$): | C | 63,20 | H | 7,01 | N | 4,75 | Cl | 6,02 |
| Gef.: | | 63,40 | | 7,04 | | 4,49 | | 6,38 |

Beispiel 175

2-[(N-(3-(5,6-Dimethoxy-naphthyl-2-oxy)-propyl)-hexahydroazepinyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und 3-(p-Toluolsulfonyloxymethyl)-N-[3-(5,6-dimethoxy-naphthyl-2-oxy)-propyl]-hexahydro-azepin analog Beispiel 2.
Ausbeute: 63,2 % der Theorie,
Schmelzpunkt: 199-201 ° C

| | | | | | | | | |
|---|---|---|---|---|---|---|---|---|
| Ber.: | C | 66,15 | H | 7,23 | N | 4,67 | Cl | 5,92 |
| Gef.: | | 65,99 | | 7,00 | | 4,44 | | 6,02 |

Beispiel 176

2-[(N-(3-(5,6-Dimethoxy-naphthyl-2-oxy)-propyl)-hexahydro-azepinyl-3)-methyl]-6,7-dimethoxy-1,2,3,4-tetrahydro-isochinolin-dihydrochlorid

Hergestellt aus 2-[(N-(3-(5,6-Dimethoxy-naphthyl-2-oxy)-propyl)-hexahydro-azepinyl-3)-methyl]-6,7-dimethoxy-1-oxo-1,2,3,4-tetrahydro-isochinolin und Lithiumaluminiumhydrid in Tetrahydrofuran/Ether analog Beispiel 3.
Ausbeute: 86,3 % der Theorie,
Schmelzpunkt: 114-116 ° C

| | | | | | | | | |
|---|---|---|---|---|---|---|---|---|
| Ber.: | C | 61,97 | H | 7,56 | N | 4,38 | Cl | 11,08 |
| Gef.: | | 62,05 | | 7,65 | | 4,06 | | 10,84 |

Beispiel 177

2-[(N-(2-(4-Methoxy-phenyl)-ethyl)-pyrrolidyl-3)-methyl]-6,7-methylendioxy-1,2,3,4-tetrahydro-isochinolin-dihydrochlorid

Hergestellt aus 2-[(N-(2-(4-Methoxy-phenyl)-ethyl)-pyrrolidyl-3)-methyl]-6,7-methylendioxy-1-oxo-1,2,3,4-tetrahydro-isochinolin und Lithiumaluminiumhydrid/Tetrahydrofuran analog Beispiel 3.
Ausbeute: 86,7 % der Theorie,
Schmelzpunkt: 213-215 ° C

| | | | | | | | | |
|---|---|---|---|---|---|---|---|---|
| Ber.: | C | 60,49 | H | 6,98 | N | 5,87 | Cl | 14,88 |
| Gef.: | | 60,59 | | 6,96 | | 5,84 | | 14,98 |

Beispiel 178

2-[(N-(3-(3,4-Dimethoxy-phenoxy)-propyl)-pyrrolidyl-3)-methyl]-6,7-methylendioxy-1-oxo-1,2,3,4-tetrahydro-isochinolin-hydrochlorid

Hergestellt aus 1-Oxo-1,2,3,4-tetrahydro-6,7-methylendioxy-isochinolin und N-[3-(3,4-Dimethoxy-phenoxy)-propyl]-3-benzosulfonyloxymethyl-pyrrolidin analog Beispiel 2.
Ausbeute: 57,4 % der Theorie,
Schmelzpunkt: 108-110 ° C

| | | | | | | | | |
|---|---|---|---|---|---|---|---|---|
| Ber.:<br>Gef.: | C | 59,76<br>60,07 | H | 6,74<br>6,87 | N | 5,35<br>5,23 | Cl | 7,02<br>7,61 |

Beispiel 179

2-[(N-(2-(4-Methoxy-phenyl)-ethyl)-pyrrolidyl-3)-methyl]-6,7-methylendioxy-1-oxo-1,2,3,4-tetrahydro-isochinolin-hydrochlorid

Hergestellt aus 1-Oxo-1,2,3,4-tetrahydro-6,7-methylendioxy-isochinolin und N-[2-(4-Methoxyphenyl)-ethyl]-3-benzolsulfonyloxymethyl-pyrrolidin analog Beispiel 2.
Ausbeute: 54,7 % der Theorie,
Schmelzpunkt: 105-108 ° C

| | | | | | | | | |
|---|---|---|---|---|---|---|---|---|
| Ber.:<br>Gef.: | C | 62,26<br>62,34 | H | 6,74<br>6,74 | N | 6,05<br>5,88 | Cl | 7,97<br>8,05 |

Beispiel 180

3-[(N-(2-(6-Methyl-pyridyl-2)-ethyl)-pyrrolidyl-3)-methyl]-7,8-dimethyl-1,3,4,5-tetrahydro-2H-3-benzazepin x 2,5 HCl x $H_2O$

Hergestellt aus 3-[(N-(2-(6-Methyl-pyridyl-2)-ethyl)-pyrrolidyl-3)-methyl]-7,8-dimethyl-2-oxo-1,3,4,5-tetrahydro-2H-3-benzazepin und Lithiumaluminiumhydrid in Diethylether und Tetrahydrofuran analog Beispiel 3.
Ausbeute: 50 % der Theorie,
Schmelzpunkt: 80-91 ° C amorph

| | | | | | | | | |
|---|---|---|---|---|---|---|---|---|
| Ber.:<br>Gef.: | C | 61,68<br>61,55 | H | 8,18<br>8,36 | N | 8,63<br>8,44 | Cl | 18,21<br>18,10 |

Beispiel 181

3-[(N-(2-(6-Methyl-pyridyl-2)-ethyl)-pyrrolidyl-3)-methyl]-7,8-dimethyl-2-oxo-1,3,4,5-tetrahydro-2H-3-benzazepin-dihydrochlorid-semihydrat

Hergestellt aus 3-[(N-(2-(6-Methyl-pyridyl-2)-ethyl)-pyrrolidyl-3)-methyl]-7,8-dimethyl-2-oxo-1,3-dihydro-2H-3-benzazepin mit 10%igem Palladium/Kohle bei 5 bar Wasserstoff und bei 80 ° C in Ethanol analog Beispiel 5.
Ausbeute: 92 % der Theorie,
Schmelzpunkt: 86-94 ° C amorph

| | | | | | | | | |
|---|---|---|---|---|---|---|---|---|
| Ber.:<br>Gef.: | C | 63,41<br>63,52 | H | 7,66<br>7,14 | N | 8,87<br>8,81 | Cl | 14,97<br>14,94 |

Beispiel 182

2-[(N-(3-(6-Methoxy-naphthyl-2-oxy)-propyl)-pyrrolidyl-3)-methyl]-6,7-dimethyl-1-oxo-1,2,3,4-tetrahydro-isochinolin

Hergestellt aus 6,7-Dimethoxy-1-oxo-1,2,3,4-tetrahydro-isochinolin und 3-(p-Toluolsulfonyloxymethyl)-N-[3-(6-methoxy-naphthyl-2-oxy)-propyl]-pyrrolidin analog Beispiel 2.
Ausbeute: 44,7 % der Theorie,
Schmelzpunkt: 102-104 °C

| | | | | | | |
|---|---|---|---|---|---|---|
| Ber.:<br>Gef.: | C | 76,24<br>75,90 | H | 7,68<br>7,62 | N | 5,93<br>5,94 |

Beispiel 183

2-[(N-(3-(6-Methoxy-naphthyl-2-oxy)-propyl)-pyrrolidyl-3)-methyl]-6,7-dimethoxy-1,2,3,4-tetrahydro-isochinolin

Hergestellt aus 2-[N-(3-(6-Methoxy-naphthyl-2-oxy)-propyl)-pyrrolidyl-3)-methyl]-6,7-dimethoxy-1-oxo-1,2,3,4-tetrahydro-isochinolin und Lithiumaluminiumhydrid in Tetrahydrofuran und Ether analog Beispiel 3.
Ausbeute: 68 % der Theorie,
Schmelzpunkt: 106-108 °C

| | | | | | | |
|---|---|---|---|---|---|---|
| Ber.:<br>Gef.: | C | 73,44<br>73,26 | H | 7,82<br>7,72 | N | 5,71<br>5,81 |

Beispiel 184

3-[(N-(3-(Indolyl-3)-propyl)-pyrrolidyl-3)-methyl]-7,8-methylendioxy-2-oxo-1,3,4,5-tetrahydro-2H-3-benzazepin-monohydrat

Hergestellt aus 3-[N-(3-(Indolyl-3)-propyl)-pyrrolidyl-3)-methyl]-7,8-methylendioxy-2-oxo-1,3-dihydro-2H-3-benzazepin mit 20%igem Palladium/Kohle bei 5 bar Wasserstoff in Ethanol bei 80 °C analog Beispiel 5.
Ausbeute: 40 % der Theorie,
Schmelzbereich: 67-74 °C

| | | | | | | |
|---|---|---|---|---|---|---|
| Ber.:<br>Gef.: | C | 69,95<br>70,00 | H | 7,17<br>7,03 | N | 9,06<br>8,97 |

Beispiel 185

3-[(N-(2-(6-Methyl-pyridyl-2)-ethyl)-pyrrolid-3-yl)-methyl]-7,8-dimethyl-2-oxo-1,3-dihydro-2H-3-benzazepin-dihydrochlorid-semihydrat

Hergestellt aus 7,8-Dimethyl-2-oxo-1,3-dihydro-2H-3-benzazepin und 3-Chlormethyl-N-[2-(6-methyl-pyridyl-2)-ethyl]-pyrrolidin analog Beispiel 2.
Ausbeute: 81 % der Theorie,

Schmelzpunkt: 86-98 ° C amorph

| | | | | | | | | |
|---|---|---|---|---|---|---|---|---|
| Ber.: | C | 63,68 | H | 7,27 | N | 8,91 | Cl | 15,04 |
| Gef.: | | 63,39 | | 7,43 | | 8,87 | | 14,93 |

Beispiel 186

2-[(N-(3-(3-Methylphenoxy)propyl)-piperidyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und N-[3-(3-Methylphenoxy)-propyl]-3-benzolsulfonyloxymethyl-piperidin analog Beispiel 2.
Ausbeute: 75,6 % der Theorie,
Schmelzpunkt: 106-109 ° C amorph

| | | | | | | | | |
|---|---|---|---|---|---|---|---|---|
| Ber.: | C | 63,95 | H | 7,75 | N | 5,52 | Cl | 7,25 |
| Gef.: | | 64,66 | | 7,91 | | 5,48 | | 7,28 |

Beispiel 187

2-[(N-(2-(5-Methyl-6-methoxy-naphth-2-yl)-pyrrolidyl-3)-me -pyrrolidyl-3) -methyl]-6,7-methylendioxy-1-oxo-1,2,3,4-tetrahydro-isochinolin-hydrochlorid

Hergestellt aus 6,7-Methylendioxy-1-oxo-1,2,3,4-tetrahydro-isochinolin und 3-(p-Toluolsulfonyloxymethyl)-N-[2-(5-methyl-6-methoxy-naphth-2-yl)-ethyl]-pyrrolidin analog Beispiel 2.
Ausbeute: 27,1 % der Theorie,
Schmelzpunkt: 249-251 ° C

| | | | | | | | | |
|---|---|---|---|---|---|---|---|---|
| Ber.: | C | 68,43 | H | 6,53 | N | 5,50 | Cl | 6,96 |
| Gef.: | | 68,47 | | 6,66 | | 5,30 | | 7,16 |

Beispiel 188

2-[(N-(3-(Naphthyl-2)-propyl)-pyrrolidyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und 3-(p-Toluolsulfonyloxymethyl)-N-[3-(naphthyl-2)-propyl]-pyrrolidin analog Beispiel 2.
Ausbeute: 20,2 % der Theorie,
Schmelzpunkt: 84-86 ° C

| | | | | | | | | |
|---|---|---|---|---|---|---|---|---|
| Ber.: | C | 67,88 | H | 7,26 | N | 5,46 | Cl | 6,91 |
| Gef.: | | 67,86 | | 7,40 | | 5,40 | | 7,17 |

Beispiel 189

2-[(N-(3-(3,4-Dimethoxyphenoxy)-propyl)-pyrrolidyl-3)-methyl]-6,7-dimethoxy-1,2,3,4-tetrahydro-isochinolin-dihydrochlorid

Hergestellt aus 2-[(N-[3-(3,4-Dimethoxyphenoxy)-propyl)-pyrrolidyl-3)-methyl]-6,7-dimethoxy-1-oxo-1,2,3,4-tetrahydro-isochinolin und Lithiumaluminiumhydrid in Diethylether und Tetrahydrofuran analog Beispiel 3.
Ausbeute: 92,3 % der Theorie,
Schmelzpunkt: 220-221 ° C

| | | | | | | | | |
|---|---|---|---|---|---|---|---|---|
| Ber.: | C | 59,20 | H | 6,88 | N | 5,31 | Cl | 13,44 |
| Gef.: | | 59,28 | | 6,97 | | 5,20 | | 13,44 |

Beispiel 190

2-[(N-(2-(3,4-Dimethoxyphenyl)-ethyl)-pyrrolidyl-3)-methyl]-6,7-dimethyl-1-oxo-1,2,3,4-tetrahydro-isochinolin

Hergestellt aus 6,7-Dimethyl-1-oxo-1,2,3,4-tetrahydro-isochinolin und N-[2-(3,4-Dimethoxyphenyl)-ethyl]-3-benzosulfonyloxymethyl-pyrrolidin analog Beispiel 2.
Ausbeute: 70 % der Theorie,
Schmelzpunkt: 168-170 ° C

| | | | | | | |
|---|---|---|---|---|---|---|
| Ber.: | C | 73,90 | H | 8,11 | N | 6,63 |
| Gef.: | | 73,96 | | 8,11 | | 6,55 |

Beispiel 191

2-[(N-(3-(4-Methoxyphenoxy)-propyl)-piperidyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und N-[3-(4-Methoxyphenoxy)-propyl]-3-benzosulfonyloxymethyl-piperidin analog Beispiel 2.
Ausbeute: 78,7 % der Theorie,
Schmelzpunkt: 98-102 ° C

| | | | | | | | | |
|---|---|---|---|---|---|---|---|---|
| Ber.: | C | 63,08 | H | 7,51 | N | 5,35 | Cl | 7,02 |
| Gef.: | | 62,87 | | 7,69 | | 5,16 | | 7,28 |

Beispiel 192

2-[(N-(3-(3-Methoxyphenoxy)-propyl)-piperidyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und N-[3-(3-Methoxyphenoxy)-propyl]-3-benzosulfonyloxymethyl-piperidin analog Beispiel 2.
Ausbeute: 87 % der Theorie,
Schmelzpunkt: 103-105 ° C

| | | | | | | | | |
|---|---|---|---|---|---|---|---|---|
| Ber.:<br>Gef.: | C | 64,21<br>64,00 | H | 7,38<br>7,55 | N | 5,55<br>5,37 | Cl | 7,02<br>7,12 |

Beispiel 193

2-[(N-(3-(3-Methoxyphenoxy)-propyl)-piperidyl-3)-methyl]-6,7-dimethoxy-1,2,3,4-tetrahydro-isochinolin-dihydrochlorid

Hergestellt aus 2-[(N-(3-(3-Methoxyphenoxy)-propyl)-piperidyl-3)-methyl]-6,7-dimethoxy-1-oxo-1,2,3,4-tetrahydro-isochinolin und Lithiumaluminiumhydrid in Diethylether und Tetrahydrofuran analog Beispiel 3.
Ausbeute: 95,4 % der Theorie,
Schmelzpunkt: 170-173 °C

| | | | | | | | | |
|---|---|---|---|---|---|---|---|---|
| Ber.:<br>Gef.: | C | 60,43<br>60,50 | H | 7,70<br>7,71 | N | 5,22<br>4,91 | Cl | 13,21<br>12,97 |

Beispiel 194

2-[(N-(2-(3,4-Dimethoxyphenyl)-ethyl)-pyrrolidyl-3)-methyl]-6,7-dimethyl-1,2,3,4-tetrahydro-isochinolin-dihydrochlorid

Hergestellt aus 2-[(N-(2-(3,4-Dimethoxyphenyl)-ethyl)-pyrrolidyl-3)-methyl]-6,7-dimethyl-1-oxo-1,2,3,4-tetrahydro-isochinolin und Lithiumaluminiumhydrid in Diethylether und Tetrahydrofuran analog Beispiel 3.
Ausbeute: 94,1 % der Theorie,
Schmelzpunkt: 260-262 °C

| | | | | | | | | |
|---|---|---|---|---|---|---|---|---|
| Ber.:<br>Gef.: | C | 64,85<br>64,60 | H | 7,96<br>8,11 | N | 5,82<br>5,91 | Cl | 14,73<br>14,67 |

Beispiel 195

2-[(N-(3-(6-Methoxy-naphthyl-2-oxy)-propyl)-azacyclooctyl-3)-methyl]-6,7-dimethoxy-1-oxo-1,2,3,4-tetrahydro-isochinolin-hydrochlorid

Hergestellt aus 2-[(Azacyclooctyl-3)-methyl)-6,7-dimethoxy-1-oxo-1,2,3,4-tetrahydro-isochinolin und 2-(3-Chlorpropoxy)-6-methoxy-naphthalin analog Beispiel 1.
Ausbeute: 24,4 % der Theorie,
Schmelzpunkt: 176-178 °C

| | | | | | | | | |
|---|---|---|---|---|---|---|---|---|
| Ber.:<br>Gef.: | C | 67,96<br>67,74 | H | 7,43<br>7,29 | N | 4,80<br>4,71 | Cl | 6,08<br>6,23 |

Beispiel 196

3-[(N-(3-(Indolyl-3)-propyl)-pyrrolidyl-3)-methyl]-7,8-dimethyl-2-oxo-1,3,4,5-tetrahydro-2H-3-benzazepin-hydrochlorid

Hergestellt aus 3-[(Pyrrolidyl-3)-methyl]-7,8-dimethyl-2-oxo-1,2,3,4-tetrahydro-2H-3-benzazepin und 3-(3-Benzolsulfonyloxy-propyl)-indol analog Beispiel 1.
Ausbeute: 62 % der Theorie,

Schmelzpunkt: 106-108 °C

| | | | | | | | | |
|---|---|---|---|---|---|---|---|---|
| Ber.:<br>Gef.: | C | 69,47<br>69,57 | H | 7,91<br>7,80 | N | 8,68<br>8,67 | Cl | 7,32<br>8,51 |

Beispiel 197

3-[(N-(3-(6-Methoxy-naphthyl-2-oxy)-propyl)-hexahydro-azepinyl-3)-methyl]-7,8-dimethoxy-1,2,3,4-tetrahydro-2H-3-benzazepin

Hergestellt aus 3-[(N-(3-(6-Methoxy-naphthyl-2-oxy)-propyl)-hexahydro-azepinyl-3)-methyl]-7,8-dimethoxy-1,2,3,4-tetrahydro-2-oxo-2H-3-benzazepin und Lithiumaluminiumhydrid in Tetrahydrofuran/Ether analog Beispiel 3.
Ausbeute: 26,8 % der Theorie,
Schmelzpunkt: 98-100 °C

| | | | | | | |
|---|---|---|---|---|---|---|
| Ber. (x $H_2O$):<br>Gef.: | C | 71,97<br>72,07 | H | 8,42<br>8,23 | N | 5,09<br>5,10 |

Beispiel 198

3-[(N-(3-(6-Methoxy-naphthyl-2-oxy)-propyl)-hexahydro-azepinyl-3)-methyl]-1,2,3,4-tetrahydro-2-oxo-2H-3-benzazepin

Hergestellt aus 3-[(Hexahydro-azepinyl-3)-methyl]-1,2,3,4-tetrahydro-2-oxo-2H-3-benzazepin und 2-(3-Chlorpropoxy)-6-methoxy-naphthalin analog Beispiel 1.
Ausbeute: 45 % der Theorie,
Schmelzpunkt: 109-111 °C

| | | | | | | |
|---|---|---|---|---|---|---|
| Ber.:<br>Gef.: | C | 72,50<br>72,35 | H | 7,74<br>7,68 | N | 5,12<br>4,93 |

Beispiel 199

2-[(N-(3-(6-Methoxy-naphthyl-2-oxy)-propyl)-hexahydro-azepinyl-3)-methyl]-1,3-dihydro-5,6-dimethoxy-isoindol

Hergestellt aus 2-[(N-(3-(6-Methoxy-naphthyl-2-oxy)-propyl)-hexahydro-azepinyl-3)-methyl]-5,6-dimethoxy-phthalimid und Lithiumaluminiumhydrid in Tetrahydrofuran/Ether analog Beispiel 3.
Ausbeute: 49,2 % der Theorie,
Schmelzpunkt: 104-146 °C

| | | | | | | |
|---|---|---|---|---|---|---|
| Ber.:<br>Gef.: | C | 73,78<br>73,63 | H | 7,99<br>7,99 | N | 5,55<br>5,39 |

Beispiel 200

3-[(N-(3-(Indolyl-3)-propyl)-pyrrolidyl-3)-methyl]-7,8-dimethyl-1,3,4,5-tetrahydro-2H-3-benzazepin-dihydrochlorid-monohydrat

Hergestellt aus 3-[(N-(3-(Indolyl-3)-propyl)-pyrrolidyl-3)-methyl]-7,8-dimethyl-2-oxo-1,3,4,5-tetrahydro-2H-3-benzazepin und Lithiumaluminiumhydrid in Tetrahydrofuran/Ether analog Beispiel 3.
Ausbeute: 65 % der Theorie,
Schmelzpunkt: 168-170 °C

| | | | | | | | | |
|---|---|---|---|---|---|---|---|---|
| Ber.:<br>Gef.: | C | 66,39<br>66,29 | H | 8,16<br>8,44 | N | 8,29<br>8,08 | Cl | 13,99<br>14,08 |

Beispiel 201

3-[(N-(3-(6-Methoxy-naphthyl-2-oxy)-propyl)-hexahydro-azepinyl-3)-methyl]-1,3,4,5-tetrahydro-7,8-methylendioxy-2H-3-benzazepin

Hergestellt aus 3-[(N-(3-(6-Methoxy-naphthyl-2-oxy)-propyl)-hexahydro-azepinyl-3)-methyl]-1,3,4,5-tetrahydro-7,8-methylendioxy-2-oxo-2H-3-benzazepin und Lithiumaluminiumhydrid/Tetrahydrofuran/Ether analog Beispiel 3.
Ausbeute: 25 % der Theorie,
Schmelzpunkt: 109-111 °C

| | | | | | | |
|---|---|---|---|---|---|---|
| Ber.:<br>Gef.: | C | 74,39<br>74,27 | H | 7,80<br>7,94 | N | 5,42<br>5,43 |

Beispiel 202

3-[(N-(3-(6-Methoxy-naphthyl-2-oxy)-propyl)-hexahydro-azepin -hexahydro-azepinyl-3)-methyl]-1,3,4,5-tetrahydro-7,8-methylendioxy-2-oxo-2H-3-benzazepin-hydrochlorid

Hergestellt aus 3-[(Hexahydro-azepinyl-3)-methyl]-1,3,4,5-tetrahydro-7,8-methylendioxy-2-oxo-2H-3-benzazepin und 2-(3-Chlorpropoxy)-6-methoxy-naphthalin analog Beispiel 1.
Ausbeute: 68,1 % der Theorie,
Schmelzbereich: 104-108 °C

| | | | | | | | | |
|---|---|---|---|---|---|---|---|---|
| Ber. (x 2 $H_2O$):<br>Gef.: | C | 65,46<br>65,60 | H | 7,38<br>7,25 | N | 4,79<br>5,01 | Cl | 6,06<br>6,43 |

Beispiel 203

2-[(N-(3-(6-Methoxy-naphthyl-2-oxy)-propyl)-hexahydro-azepinyl-3)-methyl]-6,7-methylendioxy-1-oxo-1,2,3,4-tetrahydro-isochinolin-hydrochlorid

Hergestellt aus 2-[(Hexahydro-azepinyl-3)-methyl]-6,7-methylendioxy-1-oxo-1,2,3,4-tetrahydro-isochinolin und 2-(3-Chlorpropoxy)-6-methoxy-naphthalin analog Beispiel 1.
Ausbeute: 18,2 % der Theorie,
Schmelzpunkt: 65-67 °C

| | | | | | | | | |
|---|---|---|---|---|---|---|---|---|
| Ber. (x $H_2O$):<br>Gef.: | C | 65,19<br>65,20 | H | 6,88<br>6,75 | N | 4,90<br>4,82 | Cl | 6,20<br>6,54 |

Beispiel 204

2-[(N-(3-(6-Methoxy-naphthyl-2-oxy)-propyl)-piperidyl-3)-methyl]-6,7-methylendioxy-1-oxo-1,2,3,4-tetrahydro-isochinolin

Hergestellt aus 6,7-Methylendioxy-1-oxo-1,2,3,4-tetrahydro-isochinolin und 3-(Benzolsulfonyloxymethyl)-N-[3-(6-methoxy-naphthyl-2-oxy)-propyl]-piperidin analog Beispiel 2.
Ausbeute: 5,3 % der Theorie,
Schmelzpunkt: 144-146 °C

| | | | | | | |
|---|---|---|---|---|---|---|
| Ber.:<br>Gef.: | C | 71,69<br>71,52 | H | 6,82<br>6,62 | N | 5,57<br>5,46 |

Beispiel 205

2-[(N-(3-(6-Methoxy-naphthyl-2-oxy)-propyl)-hexahydro-azepinyl-3)-methyl]-5,6-dimethyl-1,3-dihydro-1-oxo-isoindol

Hergestellt aus 2-[(N-(3-(6-Methoxy-naphthyl-2-oxy)-propyl)-hexahydro-azepinyl-3)-methyl]-5,6-dimethyl-phthalimid und Zink/Eisessig analog Beispiel 4.
Ausbeute: 18,2 % der Theorie,
Schmelzpunkt: 232-234 °C

| | | | | | | |
|---|---|---|---|---|---|---|
| Ber. (x Aceton):<br>Gef.: | C | 74,97<br>74,96 | H | 8,14<br>7,90 | N | 5,14<br>5,30 |

Beispiel 206

2-[(N-(3-(6-Methoxy-naphthyl-2-oxy)-propyl)-hexahydro-azepinyl-3)-methyl]-6,7-dimethyl-1-oxo-1,2,3,4-tetrahydro-isochinolin-hydrochlorid

Hergestellt aus 2-[(Hexahydro-azepinyl-3)-methyl]-6,7-dimethyl-1-oxo-1,2,3,4-tetrahydro-isochinolin und 2-(3-Chlorpropoxy)-6-methoxy-naphthalin analog Beispiel 1.
Ausbeute: 17,3 % der Theorie,
Schmelzbereich: 68-73 °C

| | | | | | | | | |
|---|---|---|---|---|---|---|---|---|
| Ber. (x 2 $H_2O$):<br>Gef.: | C | 67,06<br>67,05 | H | 7,93<br>7,73 | N | 4,46<br>4,88 | Cl | 6,66<br>6,18 |

Beispiel 207

2-[(N-(3-(6-Methoxy-naphthyl-2-oxy)-propyl)-piperidyl-3)-methyl]-6,7-dimethoxy-1-oxo-1,2,3,4-tetrahydro-isochinolin

Hergestellt aus 6,7-Dimethoxy-1-oxo-1,2,3,4-tetrahydro-isochinolin und 3-(Benzolsulfonyloxymethyl)-N-[3-(6-methoxynaphthyl-2-oxy)-propyl]-piperidin analog Beispiel 2.
Ausbeute: 12,9 % der Theorie,

Schmelzpunkt: 124-126 °C

| | | | | | | |
|---|---|---|---|---|---|---|
| Ber.:<br>Gef.: | C | 71,79<br>71,83 | H | 7,38<br>7,33 | N | 5,40<br>5,21 |

Beispiel 208

3-[(N-(3-(5,6-Dimethoxy-naphthyl-2-oxy)-propyl)-hexahydro-azepinyl-3)-methyl]-1,3,4,5-tetrahydro-7,8-dimethoxy-2H-3-benzazepin

Hergestellt aus 3-[(N-(3-(5,6-Dimethoxy-naphthyl-2-oxy)-propyl)-hexahydro-azepinyl-3)-methyl]-1,3,4,5-tetrahydro-7,8-di methoxy-2-oxo-2H-3-benzazepin und Lithiumaluminiumhydrid/Tetrahydrofuran/Ether analog Beispiel 3.
Ausbeute: 29,1 % der Theorie,
Schmelzpunkt: 94-96 °C

| | | | | | | |
|---|---|---|---|---|---|---|
| Ber.:<br>Gef.: | C | 72,57<br>72,56 | H | 8,24<br>8,35 | N | 4,98<br>4,94 |

Beispiel 209

2-[(N-(3-(6-Methoxy-naphthyl-2-oxy)-propyl)-hexahydro-azepinyl-3)-methyl]-1,3-dihydro-5,6-dimethoxy-1-oxo-isoindol

Hergestellt aus 2-[(N-(3-(6-Methoxy-naphthyl-2-oxy)-propyl)-hexahydro-azepinyl-3)-methyl)-5,6-dimethoxy-phthalimid und Zink/Eisessig analog Beispiel 4.
Ausbeute: 20,5 % der Theorie,
Schmelzpunkt: 265-267 °C

| | | | | | | |
|---|---|---|---|---|---|---|
| Ber. (x 2 $H_2O$):<br>Gef.: | C | 67,13<br>67,11 | H | 7,63<br>7,64 | N | 5,05<br>5,07 |

Beispiel 210

3-[(N-(3-(5,6-Dimethoxy-naphthyl-2-oxy)-propyl)-hexahydro-azepinyl-3)-methyl]-1,3,4,5-tetrahydro-7,8-dimethoxy-2-oxo-2H-3-benzazepin

Hergestellt aus 3-[(Hexahydro-azepinyl-3)-methyl]-1,3,4,5-tetrahydro-7,8-dimethoxy-2-oxo-2H-3-benzazepin und 2-(3-Chlorpropoxy)-5,6-dimethoxy-naphthalin analog Beispiel 1.
Ausbeute: 52,4 % der Theorie,
Schmelzpunkt: 129-131 °C

| | | | | | | |
|---|---|---|---|---|---|---|
| Ber.:<br>Gef.: | C | 70,81<br>70,66 | H | 7,69<br>7,84 | N | 4,86<br>4,63 |

Beispiel 211

2-[(N-(3-(5,6-Dimethoxy-naphthyl-2-oxy)-propyl)-hexahydro-azepinyl-3)-methyl]-5,6-dimethoxy-1,3-dihydro-isoindol-dihydrochlorid

Hergestellt aus 2-[(N-(3-(5,6-Dimethoxy-naphthyl-2-oxy)-propyl)-hexahydro-azepinyl-3)-methyl]-5,6-dimethoxy-phthalimid und Lithiumaluminiumhydrid in Tetrahydrofuran/Ether analog Beispiel 3.
Ausbeute: 48,2 % der Theorie,
Schmelzbereich: 172-177 ° C

| | | | | | | | | |
|---|---|---|---|---|---|---|---|---|
| Ber. (x $H_2O$):<br>Gef.: | C | 61,43<br>61,50 | H | 7,41<br>7,71 | N | 4,47<br>4,59 | Cl | 11,33<br>11,45 |

Beispiel 212

2-[(N-(3-(5,6-Dimethoxy-naphthyl-2-oxy)-propyl)-hexahydro-azepinyl-3)-methyl]-5,6-dimethoxy-1,3-dihydro-1-oxo-isoindol-hydrochlorid

Hergestellt aus 2-[(N-(3-(5,6-Dimethoxy-naphthyl-2-oxy)-propyl)-hexahydro-azepinyl-3)-methyl]-5,6-dimethoxy-phthalimid und Zink/Eisessig analog Beispiel 4.
Ausbeute: 36,4 % der Theorie,
Schmelzbereich: 215-223 ° C

| | | | | | | | | |
|---|---|---|---|---|---|---|---|---|
| Ber. (x $H_2O$):<br>Gef.: | C | 61,87<br>62,08 | H | 7,30<br>7,15 | N | 4,50<br>4,55 | Cl | 5,70<br>5,80 |

Beispiel 213

2-[(N-(3-(Indolyl-3)-propyl)-pyrrolidyl-3)-methyl]-5,6-dimethoxy-1,3-dihydro-isoindol-dihydrochlorid-monohydrat

Hergestellt aus 2-[(N-(3-(Indolyl-3)-propyl)-pyrrolidyl-3)-methyl]-5,6-dimethoxy-phthalimid und Lithiumaluminiumhydrid in Tetrahydrofuran analog Beispiel 3.
Ausbeute: 87 % der Theorie,
Schmelzpunkt: 262-264 ° C

| | | | | | | | | |
|---|---|---|---|---|---|---|---|---|
| Ber.:<br>Gef.: | C | 61,17<br>61,34 | H | 7,30<br>7,26 | N | 8,23<br>7,92 | Cl | 13,89<br>13,90 |

Beispiel 214

3-[(N-(3-(5,6-Dimethoxy-naphthyl-2-oxy)-propyl)-hexahydro-azepinyl-3)-methyl]-1,3,4,5-tetrahydro-7,8-dimethyl-2H-3-benzazepin-dihydrochlorid

Hergestellt aus 3-[(N-(3-(5,6-Dimethoxy-naphthyl-2-oxy)-propyl)-hexahydro-azepinyl-3)-methyl]-1,3,4,5-tetrahydro-7,8-dimethyl-2-oxo-2H-3-benzazepin und Lithiumaluminiumhydrid/Tetrahydrofuran/Ether analog Beispiel 3.
Ausbeute: 76,9 % der Theorie,
Schmelzbereich: 138-144 ° C

| | | | | | | | | |
|---|---|---|---|---|---|---|---|---|
| Ber.:<br>Gef.: | C | 67,64<br>67,73 | H | 8,01<br>8,26 | N | 4,64<br>4,47 | Cl | 11,74<br>11,50 |

Beispiel 215

3-[(N-(3-(5,6-Dimethoxy-naphthyl-2-oxy)-propyl)-hexahydro-azepinyl-3)-methyl]-1,3,4,5-tetrahydro-7,8-dimethyl-2-oxo-2H-3-benzazepin-hydrochlorid

Hergestellt aus 3-[(Hexahydro-azepinyl-3)-methyl]-1,3,4,5-tetrahydro-7,8-dimethyl-2-oxo-2H-3-benzazepin und 2-(3-Chlorpropoxy)-5,6-dimethoxy-naphthalin analog Beispiel 1.
Ausbeute: 47,5 % der Theorie,
Schmelzbereich: 90-96 ° C

| | | | | | | | | |
|---|---|---|---|---|---|---|---|---|
| Ber. (x $H_2O$):<br>Gef.: | C | 68,15<br>67,93 | H | 7,90<br>7,94 | N | 4,67<br>5,05 | Cl | 5,91<br>6,09 |

Beispiel 216

2-[(N-(3-(5,6-Dimethoxy-naphthyl-2-oxy)-propyl)-piperidyl-3)-methyl]-6,7-methylendioxy-1-oxo-1,2,3,4-tetrahydro-isochinolin-hydrochlorid

Hergestellt aus 2-[(Piperidyl-3)-methyl]-6,7-methylendioxy-1-oxo-1,2,3,4-tetrahydro-isochinolin und 2-(3-Chlorpropoxy)-5,6-dimethoxy-naphthalin analog Beispiel 1.
Ausbeute: 22,5 % der Theorie,
Schmelzbereich: 93-98 ° C

| | | | | | | | | |
|---|---|---|---|---|---|---|---|---|
| Ber. (x $H_2O$):<br>Gef.: | C | 63,42<br>63,59 | H | 6,69<br>6,80 | N | 4,77<br>4,70 | Cl | 6,03<br>6,28 |

Beispiel 217

2-[(N-(3-(5,6-Dimethoxy-naphthyl-2-oxy)-propyl)-pyrrolidyl-3)-methyl]-6,7-methylendioxy-1-oxo-1,2,3,4-tetrahydro-isochinolin-hydrochlorid

Hergestellt aus 2-[(Pyrrolidyl-3)-methyl]-6,7-methylendioxy-1-oxo-1,2,3,4-tetrahydro-isochinolin und 2-(3-Chlorpropoxy)-5,6-dimethoxy-naphthalin analog Beispiel 1.
Ausbeute: 22,5 % der Theorie,
Schmelzpunkt: 87-90 ° C

| | | | | | | | | |
|---|---|---|---|---|---|---|---|---|
| Ber. (x $H_2O$):<br>Gef.: | C | 62,87<br>62,72 | H | 6,50<br>6,38 | N | 4,88<br>4,93 | Cl | 6,18<br>6,30 |

Beispiel 218

2-[(N-(3-(5,6-Dimethoxy-naphthyl-2-oxy)-propyl)-piperidyl-3)-methyl]-6,7-dimethyl-1-oxo-1,2,3,4-tetrahydro-isochinolin-hydrochlorid

Hergestellt aus 2-[(Piperidyl-3)-methyl]-6,7-dimethyl-1-oxo-1,2,3,4-tetrahydro-isochinolin und 2-(3-Chlorpropoxy)-5,6-dimethoxy-naphthalin analog Beispiel 1.
Ausbeute: 21,3 % der Theorie,

Schmelzbereich: 72-78 °C

| | | | | | | | | |
|---|---|---|---|---|---|---|---|---|
| Ber. (x $H_2O$):<br>Gef.: | C | 67,30<br>67,44 | H | 7,59<br>7,74 | N | 4,90<br>5,06 | Cl | 6,21<br>6,53 |

Beispiel 219

2-[(N-(3-(5,6-Dimethoxy-naphthyl-2-oxy)-propyl)-hexahydro-azepinyl-3)-methyl]-6,7-dimethyl-1-oxo-1,2,3,4-tetrahydro-isochinolin-hydrochlorid

Hergestellt aus 2-[(Hexahydro-azepinyl-3)-methyl]-6,7-dimethyl-1-oxo-1,2,3,4-tetrahydro-isochinolin und 2-(3-Chlorpropoxy)-5,6-dimethoxy-naphthalin analog Beispiel 1.
Ausbeute: 12 % der Theorie,
Schmelzbereich: 84-90 °C

| | | | | | | | | |
|---|---|---|---|---|---|---|---|---|
| Ber. (x $H_2O$):<br>Gef.: | C | 67,73<br>67,64 | H | 7,73<br>7,81 | N | 4,78<br>4,94 | Cl | 6,06<br>6,20 |

Beispiel 220

2-[(N-(2-(4-Methoxy-phenyl)-ethyl)-hexahydro-azepinyl-3)-methyl]-6,7-methylendioxy-1-oxo-1,2,3,4-tetrahydro-isochinolin-hydrochlorid

Hergestellt aus 6,7-Methylendioxy-1-oxo-1,2,3,4-tetrahydro-isochinolin und N-[2-(4-Methoxy-phenyl)-ethyl]-3-chlormethyl-hexahydro-azepin analog Beispiel 2.
Ausbeute: 46,7 % der Theorie,
Schmelzpunkt: 101-105 °C

| | | | | | | | | |
|---|---|---|---|---|---|---|---|---|
| Ber.:<br>Gef.: | C | 62,44<br>62,62 | H | 7,26<br>7,27 | N | 5,60<br>5,48 | Cl | 7,50<br>7,66 |

Beispiel 221

2-[(N-(3-(4-Methoxy-phenoxy)-propyl)-hexahydro-azepinyl-3)-methyl]-6,7-methylendioxy-1-oxo-1,2,3,4-tetrahydro-isochinolin-hydrochlorid

Hergestellt aus 6,7-Methylendioxy-1-oxo-1,2,3,4-tetrahydro-isochinolin und N-[3-(4-Methoxy-phenoxy)-propyl]-3-chlormethyl-hexahydro-azepinanalog Beispiel 2.
Ausbeute: 43,8 % der Theorie,
Schmelzpunkt: 123-126 °C

| | | | | | | | | |
|---|---|---|---|---|---|---|---|---|
| Ber.:<br>Gef.: | C | 62,23<br>62,42 | H | 7,16<br>7,34 | N | 5,37<br>5,30 | Cl | 7,05<br>6,94 |

Beispiel 222

2-[(N-(3-(4-Methoxy-phenoxy)-propyl)-hexahydro-azepinyl-3)-methyl]-6,7-dimethyl-1-oxo-1,2,3,4-tetrahydro-isochinolin-hydrochlorid

Hergestellt aus 6,7-Dimethyl-1-oxo-1,2,3,4-tetrahydro-isochinolin und N-[3-(4-Methoxy-phenoxy)-propyl]-3-chlormethyl-hexahydro-azepinanalog Beispiel 2.
Ausbeute: 42 % der Theorie,
Schmelzpunkt: 172-173 ° C

| | | | | | | | | |
|---|---|---|---|---|---|---|---|---|
| Ber.:<br>Gef.: | C | 69,04<br>69,06 | H | 8,07<br>8,25 | N | 5,75<br>5,57 | Cl | 7,28<br>7,39 |

Beispiel 223

2-[(N-(3-(4-Methoxy-phenoxy)-propyl)-hexahydro-azepinyl-3)-methyl]-6,7-dimethoxy-1-oxo-1,2,3,4-tetrahydro-isochinolin-hydrochlorid

Hergestellt aus 6,7-Dimethyl-1-oxo-1,2,3,4-tetrahydro-isochinolin und N-[3-(4-Methoxy-phenoxy)-propyl]-3-chlormethyl-hexahydro-azepinanalog Beispiel 2.
Ausbeute: 63,3 % der Theorie,
Schmelzbereich: 115-120 ° C

| | | | | | | | | |
|---|---|---|---|---|---|---|---|---|
| Ber.:<br>Gef.: | C | 62,58<br>62,44 | H | 7,67<br>7,68 | N | 5,21<br>4,91 | Cl | 6,83<br>6,81 |

Beispiel 224

2-[(N-(2-(4-Methoxy-phenyl)-ethyl)-hexahydro-azepinyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und N-[2-(4-Methoxy-phenyl)-ethyl]-3-chlormethyl-hexahydro-azepin analog Beispiel 2.
Ausbeute: 51,6 % der Theorie,
Schmelzpunkt: 110-113 ° C

| | | | | | | | | |
|---|---|---|---|---|---|---|---|---|
| Ber.:<br>Gef.: | C | 61,75<br>61,84 | H | 7,87<br>8,02 | N | 5,28<br>5,16 | Cl | 7,25<br>7,22 |

Beispiel 225

2-[(N-(2-(3,4-Dimethoxy-phenyl)-ethyl)-hexahydro-azepinyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und N-[2-(3,4-Dimethoxy-phenyl)-ethyl]-3-chlormethyl-hexahydro-azepin analog Beispiel 2.
Ausbeute: 45,5 % der Theorie,
Schmelzpunkt: 107-111 ° C

| | | | | | | | | |
|---|---|---|---|---|---|---|---|---|
| Ber.:<br>Gef.: | C | 62,61<br>62,78 | H | 7,69<br>8,00 | N | 5,21<br>5,00 | Cl | 6,60<br>6,43 |

Beispiel 226

2-[(N-(2-(3,4-Dimethoxy-phenyl)-ethyl)-hexahydro-azepinyl-3)-methyl]-6,7-dimethyl-1-oxo-1,2,3,4-tetrahydro-isochinolin-hydrochlorid

Hergestellt aus 6,7-Dimethyl-1-oxo-1,2,3,4-tetrahydro-isochinolin und N-[2-(3,4-Dimethoxy-phenyl)-ethyl]-3-chlormethyl-hexahydro-azepin analog Beispiel 2.
Ausbeute: 44,8 % der Theorie,
Schmelzpunkt: 162-163 ° C

| | | | | | | | | |
|---|---|---|---|---|---|---|---|---|
| Ber.:<br>Gef.: | C | 66,57<br>66,67 | H | 8,18<br>8,47 | N | 5,54<br>5,35 | Cl | 7,02<br>7,11 |

Beispiel 227

2-[(N-(3-(3-Methyl-phenoxy)-propyl)-hexahydro-azepinyl-3)-methyl]-6,7-methylendioxy-1-oxo-1,2,3,4-tetrahydro-isochinolin-hydrochlorid

Hergestellt aus 6,7-Methylendioxy-1-oxo-1,2,3,4-tetrahydro-isochinolin und N-[3-(3-Methyl-phenoxy)-propyl]-3-chlormethyl-hexahydro-azepin analog Beispiel 2.
Ausbeute: 46,8 % der Theorie,
Schmelzpunkt: 159-161 ° C

| | | | | | | | | |
|---|---|---|---|---|---|---|---|---|
| Ber.:<br>Gef.: | C | 65,37<br>65,44 | H | 7,31<br>7,40 | N | 5,64<br>5,39 | Cl | 7,28<br>7,23 |

Beispiel 228

2-[(N-(3-(3-Methyl-phenoxy)-propyl)-hexahydro-azepinyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und N-[3-(3-Methoxy-phenoxy)-propyl]-3-chlormethyl-hexahydro-azepinanalog Beispiel 2.
Ausbeute: 55,2 % der Theorie,
Schmelzpunkt: 107-111 ° C

| | | | | | | | | |
|---|---|---|---|---|---|---|---|---|
| Ber.:<br>Gef.: | C | 65,67<br>65,47 | H | 7,87<br>8,00 | N | 5,47<br>5,50 | Cl | 7,05<br>6,97 |

Beispiel 229

2-[(N-(3-(3,4-Methylendioxy-phenoxy)-propyl)-hexahydro-azepinyl-3)-methyl]-6,7-dimethyl-1-oxo-1,2,3,4-tetrahydro-isochinolin-hydrochlorid

Hergestellt aus 6,7-Dimethyl-1-oxo-1,2,3,4-tetrahydro-isochinolin und N-[3-(3,4-Methylendioxy-phenoxy)-propyl]-3-chlormethyl-hexahydro-azepin analog Beispiel 2.
Ausbeute: 40,6 % der Theorie,

Schmelzpunkt: 123-126 °C

| | | | | | | | | |
|---|---|---|---|---|---|---|---|---|
| Ber.:<br>Gef.: | C | 64,78<br>64,88 | H | 7,57<br>7,59 | N | 5,39<br>5,29 | Cl | 6,83<br>7,18 |

Beispiel 230

2-[(N-(3-(3,4-Dimethoxy-phenoxy)-propyl)-hexahydro-azepinyl-3)-methyl]-6,7-dimethoxy-1-oxo-1,2,3,4-tetrahydro-isochinolin-hydrochlorid

Hergestellt aus 6,7-Dimethoxy-1-oxo-1,2,3,4-tetrahydro-isochinolin und N-[3-(3,4-Dimetnoxy-phenoxy)-propyl]-3-chlormethyl-hexahydro-azepin analog Beispiel 2.
Ausbeute: 30,6 % der Theorie,
Schmelzpunkt: 101-105 °C

| | | | | | | | | |
|---|---|---|---|---|---|---|---|---|
| Ber.:<br>Gef.: | C | 62,61<br>62,56 | H | 7,59<br>7,76 | N | 5,01<br>4,94 | Cl | 6,46<br>6,39 |

Beispiel 231

2-[(N-(2-(3,4-Dimethoxy-phenyl)-ethyl)-hexahydro-azepinyl-3)-methyl]-5,6-dimethoxy-1-oxo-1,3-dihydro-isoindol-hydrochlorid

Hergestellt aus 2-[(N-(2-(3,4-Dimethoxy-phenyl)-ethyl)-hexahydro-azepinyl-3)-methyl]-5,6-dimethoxy-phthalimid und Zink/Eisessig analog Beispiel 4.
Ausbeute: 41,8 % der Theorie,
Schmelzpunkt: 150-151 °C

| | | | | | | | | |
|---|---|---|---|---|---|---|---|---|
| Ber.:<br>Gef.: | C | 61,99<br>61,89 | H | 7,57<br>7,48 | N | 5,35<br>5,17 | Cl | 6,57<br>6,52 |

Beispiel 232

2-[(N-(3-(3-Methyl-phenoxy)-propyl)-hexahydro-azepinyl-3)-methyl]-5,6-dimethoxy-1-oxo-1,3-dihydro-isoindol-hydrochlorid

Hergestellt aus 2-[(N-(3-(3-Methyl-phenoxy)-propyl)-hexahydro-azepinyl-3)-methyl]-5,6-dimethoxy-phthalimid und Zink/Eisessig analog Beispiel 4.
Ausbeute: 88,9 % der Theorie,
Schmelzpunkt: 95-100 °C

| | | | | | | | | |
|---|---|---|---|---|---|---|---|---|
| Ber.:<br>Gef.: | C | 63,95<br>64,09 | H | 7,75<br>7,64 | N | 5,52<br>5,35 | Cl | 7,25<br>7,37 |

Beispiel 233

2-[(N-(3-(4-Methoxy-phenoxy)-propyl)-hexahydro-azepinyl-3)-methyl]-5,6-dimethoxy-1-oxo-1,3-dihydro-isoindol-hydrochlorid

Hergestellt aus 2-[(N-(3-(4-Methoxy-phenoxy)-propyl)-hexahydro-azepinyl-3)-methyl]-5,6-dimethoxy-phthalimid und Zink/Eisessig analog Beispiel 4.
Ausbeute: 87,1 % der Theorie,
Schmelzpunkt: 107-112 ° C

| | | | | | | | | |
|---|---|---|---|---|---|---|---|---|
| Ber.:<br>Gef.: | C | 61,99<br>62,07 | H | 7,51<br>7,37 | N | 5,35<br>5,28 | Cl | 7,02<br>7,31 |

Beispiel 234

2-[(N-(3-(3,4-Methylendioxy-phenoxy)-propyl)-hexahydro-azepinyl-3)-methyl]-5,6-dimethoxy-1-oxo-1,3-dihydro-isoindol-hydrochlorid

Hergestellt aus 2-[(N-(3-(3,4-Methylendioxy-phenoxy)-propyl)-hexahydro-azepinyl-3)-methyl]-5,6-dimethoxy-phthalimid und Zink/Eisessig analog Beispiel 4.
Ausbeute: 59 % der Theorie,
Schmelzpunkt: 104-107 ° C

| | | | | | | | | |
|---|---|---|---|---|---|---|---|---|
| Ber.:<br>Gef.: | C | 60,38<br>60,40 | H | 6,94<br>7,15 | N | 5,20<br>4,95 | Cl | 7,31<br>7,25 |

Beispiel 235

2-[(N-(3-(3,4-Methylendioxy-phenoxy)-propyl)-hexahydro-azepinyl-3)-methyl]-5,6-dimethoxy-1-oxo-1,3-dihydro-isoindol-hydrochlorid

Hergestellt aus 2-[(N-(3-(3,4-Methylendioxy-phenoxy)-propyl)-hexahydro-azepinyl-3)-methyl]-5,6-dimethoxy-phthalimid und Zink/Eisessig analog Beispiel 4.
Ausbeute: 79,4 % der Theorie,
Schmelzpunkt: 112-116 ° C

| | | | | | | | | |
|---|---|---|---|---|---|---|---|---|
| Ber.:<br>Gef.: | C | 62,23<br>62,16 | H | 7,15<br>7,25 | N | 5,37<br>4,96 | Cl | 7,05<br>7,03 |

Beispiel 236

3-[(N-(3-(Pyridyl-3)-propyl)-hexahydro-azepinyl-3)-methyl]-7,8-dimethoxy-2-oxo-1,3-dihydro-2H-3-benzazepin-dihydrochlorid-dihydrat

Hergestellt aus 7,8-Dimethoxy-2-oxo-1,3-dihydro-2H-3-benzazepin und N-[3-(Pyridyl-3)-propyl]-3-chlormethyl-hexahydro-azepin in Dimethylsulfoxid mit Kalium-tert.butylat analog Beispiel 2.
Ausbeute: 86 % der Theorie,
Schmelzpunkt: 106-108 ° C

| | | | | | | | | |
|---|---|---|---|---|---|---|---|---|
| Ber.:<br>Gef.: | C | 58,05<br>57,94 | H | 7,40<br>7,55 | N | 7,52<br>7,75 | Cl | 12,69<br>12,42 |

Beispiel 237

3-[(N-(3-(Pyridyl-3)-propyl)-hexahydro-azepinyl-3)-methyl]-7,8-dimethoxy-2-oxo-1,3,4,5-tetrahydro-2H-3-benzazepin-dihydrochlorid-dihydrat

Hergestellt aus 3-[(N-(3-(Pyridyl-3)-propyl)-hexahydro-azepinyl-3)-methyl]-7,8-dimethoxy-2-oxo-1,3-dihydro-2H-3-benzazepin und 5 bar Wasserstoff in Gegenwart von 10%igem Palladium auf Kohle in Dimethylformamid bei 80 °C analog Beispiel 5.
Ausbeute: 43 % der Theorie,
Schmelzpunkt: 119-121 °C

| | | | | | | | | |
|---|---|---|---|---|---|---|---|---|
| Ber.:<br>Gef.: | C | 57,85<br>57,74 | H | 7,73<br>7,79 | N | 7,49<br>7,23 | Cl | 12,65<br>12,50 |

Beispiel 238

3-[(N-(3-(Pyridyl-3)-propyl)-hexahydro-azepinyl-3)-methyl]-7,8-dimethoxy-1,3,4,5-tetrahydro-2H-3-benzazepin-trihydrochlorid-monohydrat

Hergestellt aus 3-[(N-(3-(Pyridyl-3)-propyl)-hexahydro-azepinyl-3)-methyl]-7,8-dimethoxy-2-oxo-1,3,4,5-tetrahydro-2H-3-benzazepin und Lithiumaluminiumhydrid in Tetrahydrofuran und Diethylether analog Beispiel 3.
Ausbeute: 82 % der Theorie,
Schmelzpunkt: 139-141 °C

| | | | | | | | | |
|---|---|---|---|---|---|---|---|---|
| Ber.:<br>Gef.: | C | 57,39<br>57,42 | H | 7,85<br>8,15 | N | 7,43<br>7,56 | Cl | 18,82<br>19,04 |

Beispiel 239

2-[(N-(1-(Pyridyl-3)-methyl)-pyrrolidyl-3)-methyl]-6,7-methylendioxy-1-oxo-1,2,3,4-tetrahydro-isochinolin-dihydrochlorid-dihydrat

Hergestellt aus 6,7-Methylendioxy-1-oxo-1,2,3,4-tetrahydro-isochinolin und 3-Chlormethyl-N-[(pyridyl-3)-methyl]-pyrrolidin in Dimethylsulfoxid mit Kalium-tert.butylat analog Beispiel 2.
Ausbeute: 46 % der Theorie,
Schmelzpunkt: 91-93 °C

| | | | | | | | | |
|---|---|---|---|---|---|---|---|---|
| Ber.:<br>Gef.: | C | 53,17<br>53,31 | H | 6,16<br>5,93 | N | 8,85<br>8,71 | Cl | 14,95<br>15,01 |

Beispiel 240

2-[(N-(1-(Pyridyl-3)-methyl)-pyrrolidyl-3)-methyl]-6,7-methylendioxy-1,2,3,4-tetrahydro-isochinolin-trihydrochlorid - 1,5 x hydrat

Hergestellt aus 2-[(N-(1-(Pyridyl-3)-methyl)-pyrrolidyl-3)-methyl]-6,7-methylendioxy-1-oxo-1,2,3,4-tetrahydro-isochinolin und Lithiumaluminiumhydrid in Tetrahydrofuran und Diethylether analog Beispiel 3.
Ausbeute: 67 % der Theorie,
Schmelzpunkt: 178-181 ° C

| | | | | | | | | |
|---|---|---|---|---|---|---|---|---|
| Ber.: | C | 51,70 | H | 6,41 | N | 8,61 | Cl | 21,80 |
| Gef.: | | 51,63 | | 6,62 | | 8,45 | | 21,07 |

Beispiel 241

3-[(N-(2-(6-Methyl-pyridyl-2)-ethyl)-hexahydro-azepinyl-3)-methyl]-7,8-dimethoxy-2-oxo-1,3-dihydro-2H-3-benzazepin-dihydrochlorid-dihydrat

Hergestellt aus 7,8-Dimethoxy-2-oxo-1,3-dihydro-2H-3-benzazepin und N-[2-(6-Methyl-pyridyl-2)-ethyl]-3-chlormethyl-hexahydro-azepin in Dimethylsulfoxid mit Kalium-tert.butylat analog Beispiel 2.
Ausbeute: 63 % der Theorie,
Schmelzpunkt: 134-136 ° C

| | | | | | | | | |
|---|---|---|---|---|---|---|---|---|
| Ber.: | C | 58,05 | H | 7,40 | N | 7,52 | Cl | 12,69 |
| Gef.: | | 58,15 | | 7,60 | | 7,45 | | 12,45 |

Beispiel 242

3-[(N-(2-(6-Methyl-pyridyl-2)-ethyl)-pyrrolidyl-3)-methyl]-7,8-dimethoxy-2-oxo-1,3-dihydro-2H-3-benzazepin-dihydrochlorid x 1,5 hydrat

Hergestellt aus 7,8-Dimethoxy-2-oxo-1,3-dihydro-2H-3-benzazepin und 3-Chlormethyl-N-[2-(6-methyl-pyridyl-2)-ethyl]-pyrrolidin in Dimethylsulfoxid mit Kalium-tert.butylat analog Beispiel 2.
Ausbeute: 61 % der Theorie,
Schmelzpunkt: 78-80 ° C

| | | | | | | | | |
|---|---|---|---|---|---|---|---|---|
| Ber.: | C | 57,58 | H | 6,96 | N | 8,06 | Cl | 13,60 |
| Gef.: | | 57,40 | | 7,18 | | 8,24 | | 13,39 |

Beispiel 243

3-[(N-(2-(6-Methyl-pyridyl-2)-ethyl)-hexahydro-azepinyl-3)-methyl]-7,8-dimethoxy-2-oxo-1,3,4,5-tetrahydro-2H-3-benzazepin-dihydrochlorid x 2,5 hydrat

Hergestellt aus 3-[(N-(2-(6-Methyl-pyridyl-2)-ethyl)-hexahydro-azepinyl-3)-methyl]-7,8-dimethoxy-2-oxo-1,3-dihydro-2H-3-benzazepin und 5 bar Wasserstoff in Gegenwart von Palladium/Kohle und Dimethylformamid analog Beispiel 5.
Ausbeute: 34 % der Theorie,
Schmelzpunkt: 112-114 ° C

| | | | | | | | | |
|---|---|---|---|---|---|---|---|---|
| Ber.:<br>Gef.: | C | 56,93<br>56,83 | H | 7,78<br>8,04 | N | 7,38<br>7,43 | Cl | 12,45<br>12,26 |

Beispiel 244

3-[(N-(2-(6-Methyl-pyridyl-2)-ethyl)-hexahydro-azepinyl-3)-methyl]-7,8-dimethoxy-1,3,4,5-tetrahydro-2H-3-benzazepin-trihydrochlorid-dihydrat

Hergestellt aus 3-[(N-(2-(6-Methyl-pyridyl-2)-ethyl)-hexahydro-azepinyl-3)-methyl]-7,8-dimethoxy-2-oxo-1,3,4,5-tetrahydro-2H-3-benzazepin und Lithiumaluminiumhydrid in Tetrahydrofuran und Diethylether analog Beispiel 3.
Ausbeute: 74 % der Theorie,
Schmelzpunkt: 148-150 °C

| | | | | | | | | |
|---|---|---|---|---|---|---|---|---|
| Ber.:<br>Gef.: | C | 55,61<br>55,72 | H | 7,95<br>8,10 | N | 7,20<br>7,03 | Cl | 18,24<br>18,00 |

Beispiel 245

3-[(N-(2-(6-Methyl-pyridyl-2)-ethyl)-pyrrolidyl-3)-methyl]-7,8-dimethoxy-2-oxo-1,3,4,5-tetrahydro-2H-3-benzazepin-dihydrochlorid-dihydrat

Hergestellt aus 3-[(N-(2-(6-Methyl-pyridyl-2)-ethyl)-pyrrolidyl-3)-methyl]-7,8-dimethoxy-2-oxo-1,3-dihydro-2H-3-benzazepin und Palladium/Kohle in Dimethylformamid bei 50 °C und 6 bar Wasserstoffdruck analog Beispiel 5.
Ausbeute: 28 % der Theorie,
Schmelzpunkt: 87-90 °C

| | | | | | | | | |
|---|---|---|---|---|---|---|---|---|
| Ber.:<br>Gef.: | C | 56,38<br>56,33 | H | 7,38<br>7,53 | N | 7,89<br>8,09 | Cl | 13,31<br>13,43 |

Beispiel 246

3-[(N-(2-(6-Methyl-pyridyl-2)-ethyl)-pyrrolidyl-3)-methyl]-7,8-dimethoxy-1,3,4,5-tetrahydro-2H-3-benzazepin-trihydrochlorid-dihydrat

Hergestellt aus 7,8-Dimethoxy-1,3,4,5-tetrahydro-2H-3-benzazepin und N-[2-(6-Methyl-pyridyl-2)-ethyl]-3-(benzolsulfonyloxy-methyl)-pyrrolidin in Dimethylformamid und Triethylamin analog Beispiel 2.
Ausbeute: 42 % der Theorie,
Schmelzpunkt: 225-227 °C

| | | | | | | | | |
|---|---|---|---|---|---|---|---|---|
| Ber.:<br>Gef.: | C | 54,10<br>54,18 | H | 7,62<br>7,55 | N | 7,57<br>7,51 | Cl | 19,16<br>19,30 |

Beispiel 247

2-[(N-(3-(Indolyl-3)-propyl)-piperidyl-3)-methyl]-5,6-dimethoxy-1-oxo-1,3-dihydro-isoindol x 1,5 benzolsulfonat-trihydrat

Hergestellt aus 2-[(Piperidyl-3)-methyl]-5,6-dimethoxy-1-oxo-1,3-dihydro-isoindol und 3-(3-Benzolsulfonyloxy-propyl)-indol in Dimethylformamid und Triethylamin analog Beispiel 1.
Ausbeute: 45 % der Theorie,
Schmelzpunkt: 87-89 ° C

| | | | | | | |
|---|---|---|---|---|---|---|
| Ber.:<br>Gef.: | C | 58,51<br>58,66 | H | 6,54<br>6,43 | N | 5,68<br>5,34 |

Beispiel 248

2-[(N-(3-(Indolyl-3)-propyl)-piperidyl-3)-methyl]-6,7-dimethyl-1-oxo-1,2,3,4-tetrahydro-isochinolin-benzolsulfonat-dihydrat

Hergestellt aus 2-[(Piperidyl-3)-methyl]-6,7-dimethyl-1-oxo-1,2,3,4-tetrahydro-isochinolin und 3-(3-Benzolsulfonyloxypropyl)-indol in Dimethylformamid und Triethylamin analog Beispiel 1.
Ausbeute: 94 % der Theorie,
Schmelzpunkt: 117-119 ° C

| | | | | | | |
|---|---|---|---|---|---|---|
| Ber.:<br>Gef.: | C | 65,46<br>65,51 | H | 7,27<br>6,91 | N | 6,73<br>6,72 |

Beispiel 249

3-[(N-(3-(Indolyl-3)-propyl)-pyrrolidyl-3)-methyl]-7,8-dimethoxy-2-oxo-1,3,4,5-tetrahydro-2H-3-benzazepin-hydrochlorid-monohydrat

Hergestellt aus 3-[(N-(3-(Indolyl-3)-propyl)-pyrrolidyl-3)-methyl]-7,8-dimethoxy-2-oxo-1,3-dihydro-2H-3-benzazepin und 5 bar Wasserstoff in Gegenwart von Palladium/Kohle in Dimethylformamid bei 80 ° C analog Beispiel 5.
Ausbeute: 58 % der Theorie,
Schmelzpunkt: 128-130 ° C

| | | | | | | | | |
|---|---|---|---|---|---|---|---|---|
| Ber.:<br>Gef.: | C | 65,16<br>65,12 | H | 7,42<br>7,55 | N | 8,14<br>8,11 | Cl | 6,87<br>7,06 |

Beispiel 250

3-[(N-(3-(Indolyl-3)-propyl)-hexahydro-azepinyl-3)-methyl]-7,8-dimethoxy-2-oxo-1,3,4,5-tetrahydro-2H-3-benzazepin-monohydrat

Hergestellt aus 3-[(Hexahydro-azepinyl-3)-methyl]-7,8-dimethoxy-2-oxo-1,3,4,5-tetrahydro-2H-3-benzazepin und 3-(3-Benzolsulfonyloxy-propyl)-indol in Dimethylformamid und Triethylamin analog Beispiel 1.
Ausbeute: 49 % der Theorie,
Schmelzpunkt: 56-58 ° C

| | | | | | | |
|---|---|---|---|---|---|---|
| Ber.: Gef.: | C | 70,98 71,08 | H | 8,14 8,10 | N | 8,27 8,16 |

Beispiel 251

3-[(N-(3-(Indolyl-3)-propyl)-hexahydro-azepinyl-3)-methyl]-7,8-methylendioxy-1,3,4,5-tetrahydro-2H-3-benzazepin-dihydrochlorid-monohydrat

Hergestellt aus 3-[(N-(3-(Indolyl-3)-propyl)-hexahydro-azepinyl-3)-methyl]-7,8-methylendioxy-2-oxo-1,3,4,5-tetrahydro-2H-3-benzazepin und Lithiumaluminiumhydrid in Diethylether und Tetrahydrofuran analog Beispiel 3.
Ausbeute: 56 % der Theorie,
Schmelzpunkt: 155-158 °C

| | | | | | | | | |
|---|---|---|---|---|---|---|---|---|
| Ber.: Gef.: | C | 62,26 62,31 | H | 7,50 7,82 | N | 7,63 7,40 | Cl | 12,88 12,89 |

Beispiel 252

3-[(N-(3-(Indolyl-3)-propyl)-pyrrolidyl-3)-methyl]-7,8-dimethoxy-1,3,4,5-tetrahydro-2H-3-benzazepin-dihydrochlorid-monohydrat

Hergestellt aus 3-[(N-(3-(Indolyl-3)-propyl)-pyrrolidyl-3)-methyl]-7,8-dimethoxy-2-oxo-1,3,4,5-tetrahydro-2H-3-benzazepin und Lithiumaluminiumhydrid in Diethylether und Tetrahydrofuran analog Beispiel 3.
Ausbeute: 60 % der Theorie,
Schmelzpunkt: 125-128 °C

| | | | | | | | | |
|---|---|---|---|---|---|---|---|---|
| Ber.: Gef.: | C | 62,44 62,35 | H | 7,67 7,87 | N | 7,80 7,59 | Cl | 13,16 13,67 |

Beispiel 253

2-[(N-(3-(Indolyl-3)-propyl)-piperidyl-3)-methyl]-6,7-dimethyl-1,2,3,4-tetrahydro-isochinolin-dihydrochlorid-monohydrat

Hergestellt aus 2-[(N-(3-(Indolyl-3)-propyl)-piperidyl-3)-methyl]-6,7-dimethyl-1-oxo-1,2,3,4-tetrahydro-isochinolin und Lithiumaluminiumhydrid in Diethylether und Tetrahydrofuran analog Beispiel 3.
Ausbeute: 90 % der Theorie,
Schmelzpunkt: 198-201 °C

| | | | | | | | | |
|---|---|---|---|---|---|---|---|---|
| Ber.: Gef.: | C | 66,38 66,29 | H | 8,16 8,21 | N | 8,29 8,46 | Cl | 14,00 13,82 |

Beispiel 254

3-[(N-(3-(Indolyl-3)-propyl)-hexahydro-azepinyl-3)-methyl]-7,8-methylendioxy-2-oxo-1,3,4,5-tetrahydro-2H-3-benzazepin-monohydrat

Hergestellt aus 3-[(Hexahydro-azepinyl-3)-methyl]-7,8-methylendioxy-2-oxo-1,3,4,5-tetrahydro-2H-3-benzazepin und 3-(3-Benzolsulfonyloxy-propyl)-indol analog Beispiel 1.

Ausbeute: 61,5 % der Theorie,
Schmelzpunkt: 62-64 ° C

| | | | | | | |
|---|---|---|---|---|---|---|
| Ber.:<br>Gef.: | C | 70,84<br>70,73 | H | 7,59<br>7,59 | N | 8,55<br>8,42 |

Beispiel 255

2-[(N-(3-(Indolyl-3)-propyl)-pyrrolidyl-3)-methyl]-6,7-dimethyl-1,2,3,4-tetrahydro-isochinolin-dihydrochlorid-semihydrat

Hergestellt aus 2-[(N-(3-(Indolyl-3)-propyl)-pyrrolidyl-3)-methyl]-6,7-dimethyl-1-oxo-1,2,3,4-tetrahydro-isochinolin und Lithiumaluminiumhydrid in Ether analog Beispiel 3.
Ausbeute: 86 % der Theorie,
Schmelzpunkt: 143-145 ° C

| | | | | | | | | |
|---|---|---|---|---|---|---|---|---|
| Ber.:<br>Gef.: | C | 67,06<br>66,92 | H | 7,92<br>8,07 | N | 8,69<br>8,48 | Cl | 14,66<br>14,87 |

Beispiel 256

2-[(N-(3-(Indolyl-3)-propyl)-pyrrolidyl-3)-methyl]-6,7-dimethyl-1-oxo-1,2,3,4-tetrahydro-isochinolin-hydrochlorid x 1,5 hydrat

Hergestellt aus 2-[(Pyrrolidyl-3)-methyl]-6,7-dimethyl-1-oxo-1,2,3,4-tetrahydro-isochinolin und 3-(3-Benzolsulfonyloxy-propyl)-indol analog Beispiel 1.
Ausbeute: 67 % der Theorie,
Schmelzpunkt: 117-120 ° C

| | | | | | | | | |
|---|---|---|---|---|---|---|---|---|
| Ber.:<br>Gef.: | C | 67,69<br>67,62 | H | 7,78<br>7,80 | N | 8,77<br>8,72 | Cl | 7,40<br>7,93 |

Beispiel 257

Tabletten zu 7,5 mg 3-[(N-(2-(Naphthyl-2)-ethyl)-piperidyl-3)-methyl]-7,8-dimethoxy-2-oxo-1,3,4,5-tetrahydro-2H-3-benzazepin-hydrochlorid

Zusammensetzung:

| 1 Tablette enthält: | |
|---|---|
| Wirksubstanz | 7,5 mg |
| Maisstärke | 59,5 mg |
| Milchzucker | 48,0 mg |
| Polyvinylpyrrolidon | 4,0 mg |
| Magnesiumstearat | 1.0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45 °C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht: 120 mg

Beispiel 258

Dragées zu 5 mg 3-[(N-(2-(Naphthyl-2)-ethyl)-piperidyl-3)-methyl]-7,8-dimethoxy-2-oxo-1,3,4,5-tetrahydro-2H-3-benzazepin-hydrochlorid

| 1 Dragéekern enthält: | |
|---|---|
| Wirksubstanz | 5,0 mg |
| Maisstärke | 41,5 mg |
| Milchzucker | 30,0 mg |
| Polyvinylpyrrolidon | 3,0 mg |
| Magnesiumstearat | 0.5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45 °C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht: 130 mg

Beispiel 259

Ampullen zu 5 mg 3-[(N-(2-(Naphthyl-2)-ethyl)-piperidyl-3)-methyl]-7,8-dimethoxy-2-oxo-1,3,4,5-tetrahydro-2H-3-benzazepin-hydrochlorid

| 1 Ampulle enthält: | |
|---|---|
| Wirksubstanz | 5,0 mg |
| Sorbit | 50,0 mg |
| Wasser für Injektionszwecke ad | 2,0 ml |

Herstellungsverfahren

In einem geeigneten Ansatzgefäß wird der Wirkstoff in Wasser für Injektionszwecke gelöst und die Lösung mit Sorbit isotonisch gestellt.

Nach Filtration über einem Membranfilter wird die Lösung unter $N_2$-Begasung in gereinigte und sterilisierte Ampullen abgefüllt und 20 Minuten im strömenden Wasserdampf autoklaviert.

Beispiel 260

Suppositorien zu 10 mg 3-[(N-(2-(Naphthyl-2)-ethyl)-piperidyl-3)-methyl]-7,8-dimethoxy-2-oxo-1,3,4,5-tetrahydro-2H-3-benzazepin-hydrochlorid

| 1 Zäpfchen enthält: | |
|---|---|
| Wirksubstanz | 0,010 g |
| Hartfett (z.B. Witepsol H 19 und W 45) | 1,690 g |
| | 1,700 g |

Herstellungsverfahren:

Das Hartfett wird geschmolzen. Bei 38 °C wird die gemahlene Wirksubstanz in der Schmelze homogen dispergiert. Es wird auf 35 °C abgekühlt und in schwach vorgekühlte Suppositorienformen ausgegossen.

Beispiel 261

Tropfenlösung mit 10 mg 3-[(N-(2-(Naphthyl-2)-ethyl)-piperidyl-3)-methyl]-7,8-dimethoxy-2-oxo-1,3,4,5-tetrahydro-2H-3-benzazepin-hydrochlorid

| 100 ml Lösungen enthalten: | |
|---|---|
| Wirksubstanz | 0,2 g |
| Hydroxyethylcellulose | 0,15 g |
| Weinsäure | 0,1 g |
| Sorbitlösung 70 % Trockensubstanz | 30,0 g |
| Glycerin | 10,0 g |
| Benzoesäure | 0,15 g |
| Dest.Wasser | ad 100 ml |

Herstellungsverfahren:

Dest. Wasser wird auf 70 °C erhitzt. Hierin wird unter Rühren Hydroxyethylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

**Patentansprüche**

**1.** Cyclische Aminderivate der allgemeinen Formel (I)

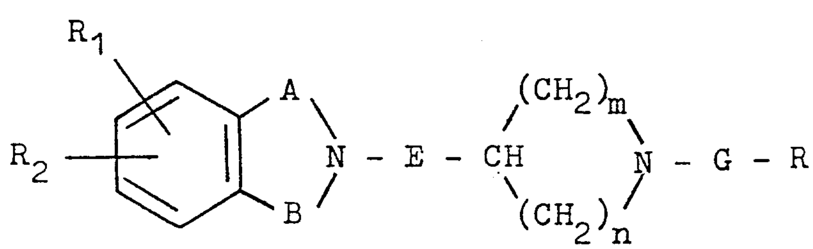

(I)

in der

A eine $-CH_2-$, $-CH_2-CH_2-$ oder $-CH=CH-$Gruppe,
B eine $-CH_2-$, $-CH_2-CH_2-$, -CO- oder $-\overset{x}{C}H_2CO-$Gruppe, wobei das mit x gekennzeichnete Kohlenstoffatom mit dem Phenylkern verknüpft ist,
E eine geradkettige Alkylengruppe mit 1 bis 3 Kohlenstoffatomen,
G eine geradkettige Alkylengruppe mit 1 bis 6 Kohlenstoffatomen, wobei eine mit einem aromatischen oder heteroaromatischen Rest R verbundene Methylengruppe einer geradkettigen Alkylengruppe mit 3 bis 6 Kohlenstoffatomen durch ein Sauerstoffatom, eine Methylimino- oder Ethyliminogruppe ersetzt sein kann,
m die Zahl 1, 2, 3, 4, 5 oder 6 und
n die Zahl 0, 1, 2 oder 3, wobei jedoch n + m die Zahl 3, 4, 5 oder 6 darstellen muß,
$R_1$ eine Methyl- oder Methoxygruppe,
$R_2$ eine Methyl- oder Methoxygruppe oder $R_1$ und $R_2$ zusammen eine Methylendioxygruppe und
R eine gegebenenfalls durch eine Methylgruppe substituierte Furyl-, Thienyl-, Pyridyl-, Benzo[b]furyl- oder Benzo[b]thienylgruppe, eine durch ein Halogenatom, eine Methoxy- oder Methansulfonyloxygruppe substituierte Benzo[b]thienylgruppe, eine gegebenenfalls durch eine Hydroxy-, Methoxy- oder Benzyloxygruppe substituierte Indolyl- oder N-Methylindolylgruppe, eine Dimethyl-thienyl- oder Dimethoxy-isochinolylgruppe, eine gegebenenfalls durch Methyl- oder Methoxygruppen mono- oder disubstituierte Naphthylgruppe, wobei die Substituenten gleich oder verschieden sein können, oder auch, wenn B eine $-CH_2-$ oder CO-Gruppe darstellt, eine gegebenenfalls durch eine Methylendioxygruppe substituierte Phenylgruppe, eine durch Chlor- oder Bromatome, Methyl- oder Methoxygruppen mono- oder disubstituierte Phenylgruppe, wobei die Substituenten gleich oder verschieden sein können, eine durch eine Hydroxy-, Benzyloxy-, Methansulfonyloxy-, Trifluormethansulfonyloxy-, Trifluormethyl-, Trifluormethoxy-, Nitro-, Amino-, Acetamido-, Methansulfonylamino- oder Bis(methansulfonyl)aminogruppe substituierte Phenylgruppe, eine Trimethoxyphenyl-, Tetramethylphenyl- oder Dihalogenaminophenylgruppe bedeuten, deren Enantiomeren, deren Diastereomeren, deren N-Oxide und deren Säureadditionssalze.

**2.** Cyclische Aminderivate der allgemeinen Formel (Ia)

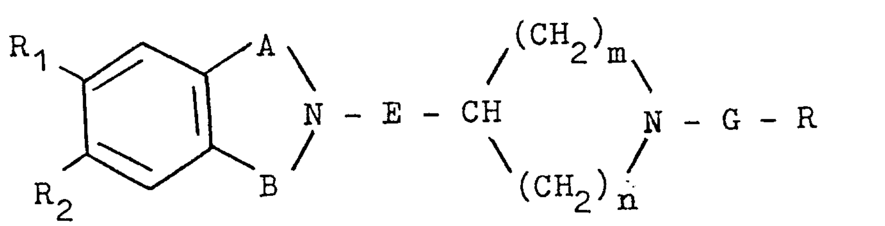
(Ia)

in der
R, $R_1$, $R_2$, A, B, E, G, m und n wie im Anspruch 1 definiert sind,
deren Enantiomeren, deren Diastereomeren, deren N-Oxide und deren Säureadditionssalze.

**3.** Cyclische Aminderivate der allgemeinen Formel (Ia) gemäß Anspruch 2, in der
A eine $-CH_2CH_2-$Gruppe,
B eine $-CH_2-CH_2-$, -CO- oder $-\overset{x}{C}H_2CO-$Gruppe, wobei das mit x gekennzeichnete Kohlenstoffatom mit dem Phenylkern verknüpft ist,
E eine Methylen- oder Ethylengruppe,
G eine geradkettige Alkylengruppe mit 2 bis 4 Kohlenstoffatomen, wobei die mit einem aromatischen oder heteroaromatischen Rest R verbundene Methylengruppe einer geradkettigen Alkylengruppe mit 3 oder 4 Kohlenstoffatomen durch ein Sauerstoff ersetzt sein kann,
$R_1$ eine Methoxygruppe,
$R_2$ eine Methoxygruppe oder $R_1$ und $R_2$ zusammen eine Methylendioxygruppe,
m die Zahl 2, 3 oder 4,
n die Zahl 1 und
R eine Naphthyl-2-, 6-Methoxy-naphthyl-2-, 5-Methyl-6-methoxy-naphthyl-2-, Thienyl-2-, 6-Methyl-pyridyl-2-, Benzo[b]furyl-2- oder Benzo[b]thienyl-3-gruppe oder auch, wenn B die -CO-Gruppe darstellt, eine 4-Methoxyphenyl- oder 3,4-Dimethoxyphenylgruppe bedeuten,
deren Enantiomeren, deren Diastereomeren und deren Säureadditionssalze.

**4.** Cyclische Aminderivate der allgemeinen Formel (Ia) gemäß Anspruch 2
a) 3-[(N-(2-(Naphthyl-2)-ethyl)-piperidyl-3)-methyl]-7,8-dimethoxy-2-oxo-1,3,4,5-tetrahydro-2H-3-benzazepin,
b) 3-[(N-(2-(5-Methyl-6-methoxy-naphthyl-2)-ethyl)-piperidyl-3)-methyl]-7,8-dimethoxy-2-oxo-1,3,4,5-tetrahydro-2H-3-benzazepin,
c) 3-[2-(N-(2-(6-Methoxy-naphthyl-2)-ethyl)-piperidyl-2)-ethyl]-7,8-dimethoxy-2-oxo-1,3,4,5-tetrahydro-2H-3-benzazepin,
d) 2-[(N-(2-(6-Methoxy-naphthyl-2)-ethyl)-hexahydro-azepinyl-3)-methyl]-6,7-methylendioxy-1-oxo-1,2,3,4-tetrahydro-isochinolin,
e) 2-[(N-(2-(Naphthyl-2)-ethyl)-hexahydro-azepinyl-3)-methyl]-6,7-methylendioxy-1-oxo-1,2,3,4-tetrahydro-isochinolin,
f) 2-[(N-(2-(3,4-Dimethoxy-phenyl)-ethyl)-piperidyl-3)-methyl]-6,7-dimethyl-1,2,3,4-tetrahydro-isochinolin,
g) 2-[(N-(2-(3,4-Dimethoxy-phenyl)-ethyl)-piperidyl-3)-methyl]-6,7-dimethyl-1-oxo-1,2,3,4-tetrahydro-isochinolin,
h) 2-[(N-(3-(4-Methoxy-phenoxy)-propyl)-piperidyl-3)-methyl]-6,7-dimethyl-1-oxo-1,2,3,4-tetrahydro-isochinolin,
i) 3-[(N-(4-(Thienyl-2)-butyl)-piperidyl-3)-methyl]-7,8-dimethoxy-2-oxo-1,3,4,5-tetrahydro-2H-3-benzazepin,
k) 3-[(N-(2-(Benzo[b]furyl-2)-ethyl)-piperidyl-3)-methyl]-7,8-dimethoxy-2-oxo-1,3,4,5-tetrahydro-2H-3-benzazepin,
l) 3-[(N-(2-(Benzo[b]thienyl-3)-ethyl)-piperidyl-3)-methyl]-7,8-dimethoxy-2-oxo-1,3,4,5-tetrahydro-2H-3-benzazepin,
m) 2-[(N-(3-(6-Methoxy-naphthyl-2-oxy)-propyl)-pyrrolidyl-3)-methyl]-6,7-dimethoxy-1-oxo-1,2,3,4-tetrahydro-isochinolin und
n) 2-[(N-(2-(6-Methoxy-naphthyl-2)-ethyl)-hexahydro-azepinyl-3)-methyl]-6,7-methylendioxy-1-oxo-1,2,3,4-tetrahydro-isochinolin,
deren Enantiomeren, deren Diastereomeren und deren Säureadditionssalze.

**5.** Cyclische Aminderivate der allgemeinen Formel (Ia) gemäß Anspruch 2
a) 3-[(N-(2-(Naphthyl-2)-ethyl)-piperidyl-3)-methyl]-7,8-dimethoxy-2-oxo-1,3,4,5-tetrahydro-2H-3-benzazepin,
b) 2-[(N-(3-(4-Methoxy-phenoxy)-propyl)-piperidyl-3)-methyl]-6,7-dimethyl-1-oxo-1,2,3,4-tetrahydro-isochinolin,
c) 3-[(N-(4-(Thienyl-2)-butyl)-piperidyl-3)-methyl]-7,8-dimethoxy-2-oxo-1,3,4,5-tetrahydro-2H-3-benzazepin,
d) 2-[(N-(3-(6-Methoxy-naphthyl-2-oxy)-propyl)-pyrrolidyl-3)-methyl]-6,7-dimethoxy-1-oxo-1,2,3,4-tetrahydro-isochinolin,
e) 2-[(N-(2-(6-Methoxy-naphthyl-2)-ethyl)-hexahydro-azepinyl-3)-methyl]-6,7-methylendioxy-1-oxo-1,2,3,4-tetrahydro-isochinolin,
f) 3-[(N-(2-(4-Methoxy-phenyl)-ethyl)-hexahydro-azepinyl-3)-methyl]-7,8-dimethoxy-2-oxo-1,3,4,5-tetrahydro-2H-3-benzazepin und
g) 3-[(N-(2-(3,4-Dimethoxy-phenyl)-ethyl)-hexahydro-azepinyl-3)-methyl]-7,8-methylendioxy-2-oxo-1,3,4,5-tetrahydro-2H-3-benzazepin,
deren Enantiomeren, deren Diastereomeren und deren Säureadditionssalze.

**6.** 3-[(N-(2-(Naphthyl-2)-ethyl)-piperidyl-3)-methyl]-7,8-dimethoxy-2-oxo-1,3,4,5-tetrahydro-2H-3-benzazepin, dessen Enantiomeren, dessen Diastereomeren und dessen Säureadditionssalze.

**7.** Physiologisch verträgliche Säureadditionssalze der Verbindungen gemäß den Ansprüchen 1 bis 6 mit anorganischen oder organischen Säuren.

**8.** Arzneimittel, enthaltend eine Verbindung der allgemeinen Formel (I) gemäß den Ansprüchen 1 bis 6 oder dessen physiologisch verträgliches Säureadditionssalz gemäß Anspruch 7 neben einem oder mehreren inerten Trägerstoffen und/oder Verdünnungsmitteln.

**9.** Arzneimittel gemäß Anspruch 8 zur Verwendung bei der Behandlung von Sinustachykardien und ischämischen Herzerkrankungen.

**10.** Verfahren zur Herstellung eines Arzneimittels gemäß den Ansprüchen 8 und 9, dadurch gekennzeichnet, daß auf nichtchemischem Wege eine Verbindung gemäß den Ansprüchen 1 bis 6 oder dessen physiologisch verträgliches Säureadditionssalz gemäß Anspruch 7 in einen oder mehreren inerten Trägerstoffen und/oder Verdünnungsmitteln eingearbeitet wird.

**11.** Verfahren zur Herstellung von cyclischen Aminderivaten gemäß den Ansprüchen 1 bis 7, dadurch gekennzeichnet, daß

a) eine Verbindung der allgemeinen Formel (II)

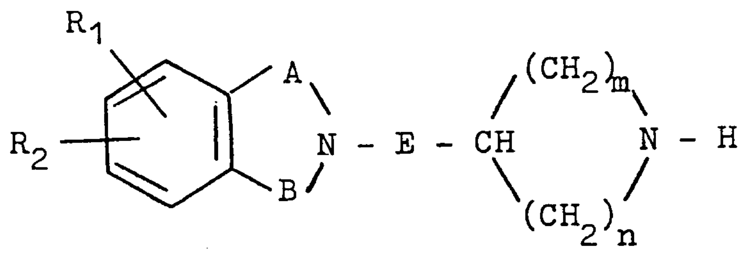

(II)

in der

$R_1$, $R_2$, A, B, E, m und n wie in den Ansprüchen 1 bis 6 definiert sind, mit einer Verbindung der allgemeinen Formel (III)

$Z_1$ - G - R (III)

in der

R und G wie in den Ansprüchen 1 bis 6 definiert sind und

$Z_1$ eine nukleophile Austrittsgruppe, eine Hydroxygruppe oder $Z_1$ zusammen mit einem Wasserstoffatom der benachbarten Methylengruppe ein Sauerstoffatom darstellt, umgesetzt wird oder

b) eine Verbindung der allgemeinen Formel (IV)

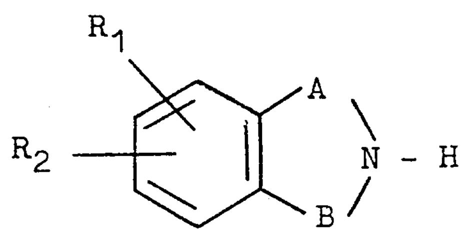

(IV)

in der

$R_1$, $R_2$, A und B wie in den Ansprüchen 1 bis 6 definiert sind, mit einer Verbindung der allgemeinen Formel (V)

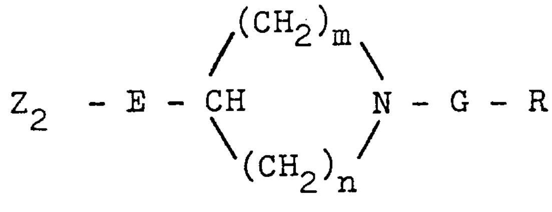

(V)

in der

R, E, G, m und n wie in den Ansprüchen 1 bis 6 definiert sind und

$Z_2$ eine nukleophile Austrittsgruppe darstellt, umgesetzt wird und

erforderlichenfalls anschließend ein während den Umsetzungen zum Schutze von reaktiven Gruppen wie Hydroxy-, Amino-, Alkylamino- oder Iminogruppen verwendeter Schutzrest abgespalten wird

und/oder
gewünschtenfalls anschließend eine so erhaltene Verbindung der allgemeinen Formel (I), der R eine Nitrogruppe enthält, mittels Reduktion in eine entsprechende Aminoverbindung der allgemeinen Formel (I) übergeführt wird und/oder
eine so erhaltene Verbindung der allgemeinen Formel (I), in der R eine Aminogruppe enthält, mittels Acylierung in eine entsprechende Alkanoylaminoverbindung der allgemeinen Formel (I) übergeführt wird und/oder
eine so erhaltene Verbindung der allgemeinen Formel (I), welche mindestens ein chirales Zentrum enthält, in ihre Diastereomeren oder in ihre Enantiomeren aufgetrennt wird und/oder
eine so erhaltene Verbindung der allgemeinen Formel (I) in ihre Säureadditionssalze, insbesondere in ihre physiologisch verträglichen Säureadditionssalze mit anorganischen oder organischen Säuren übergeführt wird.

**12.** Verfahren zur Herstellung von cyclischen Aminderivaten der allgemeinen Formel (I) gemäß den Ansprüchen 1 bis 7, in denen B eine $-CH_2-$ oder $-CH_2CH_2-$Gruppe darstellt, dadurch gekennzeichnet, daß
c) eine Verbindung der allgemeinen Formel (VI)

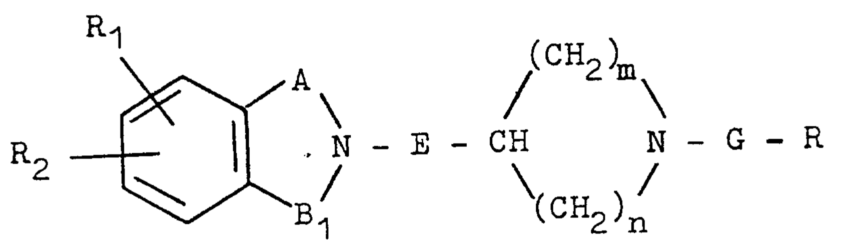

(VI)

in der
R, $R_1$, $R_2$, A, E, G, m und n wie in den Ansprüchen 1 bis 6 definiert sind und
$B_1$ eine -CO- oder $-\overset{x}{C}H_2CO-$Gruppe darstellt, wobei das mit x gekennzeichnete Kohlenstoffatom mit dem Phenylkern verknüpft ist, reduziert wird und
gewünschtenfalls anschließend eine so erhaltene Verbindung der allgemeinen Formel (I), in der R eine Nitrogruppe enthält, mittels Reduktion in eine entsprechende Aminoverbindung der allgemeinen Formel (I) übergeführt wird und/oder
eine so erhaltene Verbindung der allgemeinen Formel (I), in der R eine Aminogruppe enthält, mittels Acylierung in eine entsprechende Alkanoylaminoverbindung der allgemeinen Formel (I) übergeführt wird und/oder
eine so erhaltene Verbindung der allgemeinen Formel (I), welche mindestens ein chirales Zentrum enthält, in ihre Diastereomeren oder in ihre Enantiomeren aufgetrennt wird und/oder
eine so erhaltene Verbindung der allgemeinen Formel (I) in ihre Säureadditionssalze, insbesondere in ihre physiologisch verträglichen Säureadditionssalze mit anorganischen oder organischen Säuren übergeführt wird.

**13.** Verfahren zur Herstellung von cyclischen Aminderivaten der allgemeinen Formel I gemäß den Ansprüchen 1 bis 7, in denen A eine $-CH_2-$Gruppe und B eine -CO- oder $-CH_2CO-$Gruppe darstellen, dadurch gekennzeichnet, daß
d) eine Verbindung der allgemeinen Formel (VII)

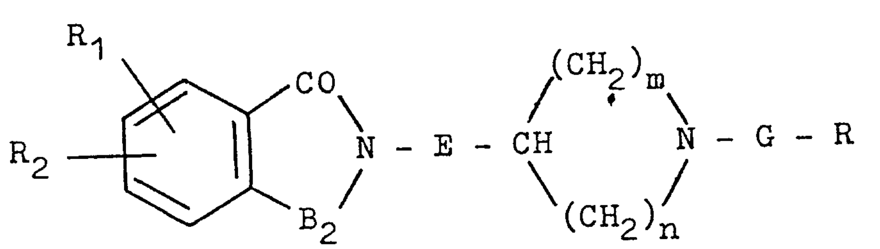

(VII)

in der
R, $R_1$, $R_2$, E, G, m und n wie in den Ansprüchen 1 bis 6 definiert sind und
$B_2$ eine -CO- oder $-\overset{x}{C}H_2CO$-Gruppe darstellt, wobei das mit x gekennzeichnete Kohlenstoffatom mit dem Phenylkern verknüpft ist, mit nascierendem Wasserstoff reduziert wird und
gewünschtenfalls anschließend eine so erhaltene Verbindung der allgemeinen Formel (I), in der R eine Aminogruppe enthält, mittels Acylierung in eine entsprechende Alkanoylaminoverbindung der allgemeinen Formel I übergeführt wird und/oder
eine so erhaltene Verbindung der allgemeinen Formel (I), welche mindestens ein chirales Zentrum enthält, in ihre Diastereomeren oder in ihre Enantiomeren aufgetrennt wird und/oder
eine so erhaltene Verbindung der allgemeinen Formel (I) in ihre Säureadditionssalze, insbesondere in ihre physiologisch verträglichen Säureadditionssalze mit anorganischen oder organischen Säuren übergeführt wird.

**14.** Verfahren zur Herstellung von neuen cyclischen Aminderivaten gemäß den Ansprüchen 1 bis 7, in denen A die $-CH_2-CH_2$-Gruppe und B die Methylen- oder Carbonylgruppe darstellt, dadurch gekennzeichnet, daß
e) eine Verbindung der allgemeinen Formel (VIII)

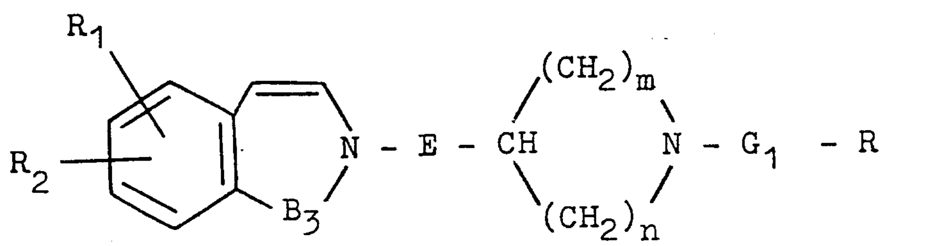
(VIII)

in der
R, $R_1$, $R_2$, B, E, G, m und n wie in den Ansprüchen 1 bis 6 definiert sind, und
$B_3$ eine $-CH_2$- oder -CO-Gruppe darstellt, hydriert wird und
gewünschtenfalls anschließend eine so erhaltene Verbindung der allgemeinen Formel (I), in der R eine Aminogruppe enthält, mittels Acylierung in eine entsprechende Alkanoylaminoverbindung der allgemeinen Formel (I) übergeführt wird und/oder
eine so erhaltene Verbindung der allgemeinen Formel (I), welche mindestens ein chirales Zentrum enthält, in ihre Diastereomeren oder in ihre Enantiomeren aufgetrennt wird und/oder
eine so erhaltene Verbindung der allgemeinen Formel (I) in ihre Säureadditionssalze, insbesondere in ihre physiologisch verträglichen Säureadditionssalze mit anorganischen oder organischen Säuren übergeführt wird.

## Claims

**1.** Cyclic amine derivatives of general formula (I)

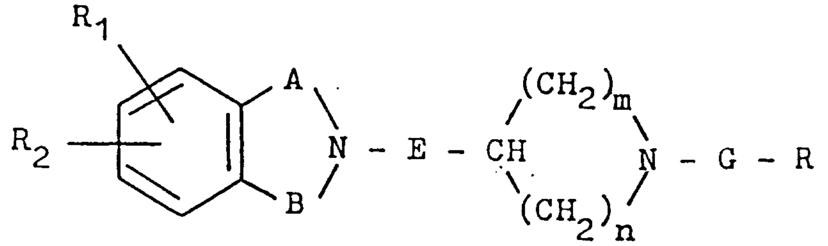
(I)

wherein
A represents a $-CH_2-$, $-CH_2-CH_2-$ or $-CH=CH-$ group,
B represents a $-CH_2-$, $-CH_2-CH_2-$, -CO- or $-\overset{x}{C}H_2CO$ group, whilst the carbon atom marked x is linked to the phenyl nucleus,

E represents a straight-chained $C_{1-3}$-alkylene group,
G represents a straight-chained $C_{1-6}$-alkylene group, wherein a methylene group, linked to an aromatic or heteroaromatic group R, of a straight-chained $C_{3-6}$-alkylene group may be replaced by an oxygen atom, or by a methylimino or ethylimino group,
m represents the number 1, 2, 3, 4, 5 or 6 and
n represents the number 0, 1, 2 or 3, although n + m must represent the number 3, 4, 5 or 6,
$R_1$ represents a methyl or methoxy group,
$R_2$ represents a methyl or methoxy group or $R_1$ and $R_2$ together represent a methylenedioxy group and
R represents an optionally methyl-substituted furyl, thienyl, pyridyl, benzo[b]furyl or benzo[b]thienyl group, a benzo[b]thienyl group substituted by a halogen atom or by a methoxy or methanesulphonyloxy group, an indolyl or N-methyl-indolyl group optionally substituted by a hydroxy, methoxy or benzyloxy group, a dimethyl-thienyl or dimethoxy-isoquinolyl group, a naphthyl group optionally mono- or disubstituted by methyl or methoxy groups, whilst the substituents may be identical or different, or, if B represents a $-CH_2-$ or -CO- group, a phenyl group optionally substituted by a methylenedioxy group, a phenyl group mono- or disubstituted by a chlorine or bromine atom or methyl or methoxy groups, whilst the substituents may be identical or different, a phenyl group substituted by a hydroxy, benzyloxy, methanesulphonyloxy, trifluoromethanesulphonyloxy, trifluoromethyl, trifluoromethoxy, nitro, amino, acetamido, methanesulphonylamino or bis(methanesulphonyl)amino group, or a trimethoxyphenyl, tetramethylphenyl or dihaloaminophenyl group,
the enantiomers, diastereomers, N-oxides and acid addition salts thereof.

**2.** Cyclic amine derivatives of general formula (Ia)

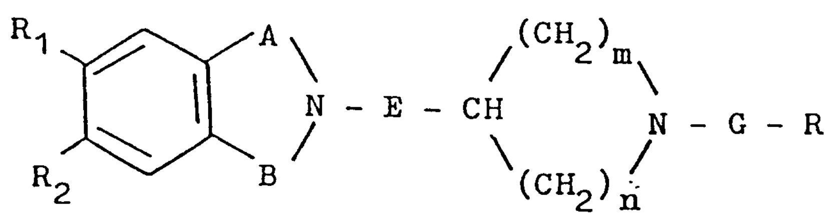

(Ia)

wherein
R, $R_1$, $R_2$, A, B, E, G, m and n are defined as in claim 1, the enantiomers, diastereomers, N-oxides and acid addition salts thereof.

**3.** Cyclic amine derivatives of general formula Ia according to claim 2, wherein
A represents a $-CH_2CH_2-$ group,
B represents a $-CH_2-CH_2$, -CO- or $-\overset{x}{C}H_2CO-$ group, wherein the carbon atom designated x is linked to the phenyl nucleus,
E represents a methylene or ethylene group,
G represents a straight-chained $C_{2-4}$-alkylene group, wherein a methylene group of a straight-chained $C_{3-4}$-alkylene group, linked to an aromatic or heteroaromatic group R, may be replaced by an oxygen,
$R_1$ represents a methoxy group,
$R_2$ represents a methoxy group or $R_1$ and $R_2$ together represent a methylenedioxy group,
m represents the number 2, 3 or 4,
n represents the number 1 and
R represents a naphth-2-yl, 6-methoxy-naphth-2-yl, 5-methyl-6-methoxy-naphth-2-yl, thien-2-yl, 6-methyl-pyrid-2-yl, benzo[b]fur-2-yl or benzo[b]thien-3-yl group or, if B represents a -CO- group, a 4-methoxyphenyl or 3,4-dimethoxyphenyl group,
the enantiomers, diastereomers and acid addition salts thereof.

**4.** Cyclic amine derivatives of general formula (Ia) according to claim 2
a) 3-[(N-(2-(naphth-2-yl)-ethyl)-piperid-3-yl)-methyl]-7,8-dimethoxy-2-oxo-1,3,4,5-tetrahydro-2H-3-benzazepine,

b) 3-[(N-(2-(5-methyl-6-methoxy-naphth-2-yl)-ethyl)-piperid-3-yl)-methyl]-7,8-dimethoxy-2-oxo-1,3,4,5-tetrahydro-2H-3-benzazepine,
c) 3-[2-(N-(2-(6-methoxy-naphth-2-yl)-ethyl)-piperid-2-yl)-ethyl]-7,8-dimethoxy-2-oxo-1,3,4,5-tetrahydro-2H-3-benzazepine,
d) 2-[(N-(2-(6-methoxy-naphth-2-yl)-ethyl)-hexahydro-azepin-3-yl)-methyl]-6,7-methylenedioxy-1-oxo-1,2,3,4-tetrahydro-isoquinoline,
e) 2-[(N-(2-(naphth-2-yl)-ethyl)-hexahydro-azepin-3-yl)-methyl]-6,7-methylenedioxy-1-oxo-1,2,3,4-tetrahydro-isoquinoline,
f) 2-[(N-(2-(3,4-dimethoxy-phenyl)-ethyl)-piperid-3-yl)-methyl]-6,7-dimethyl-1,2,3,4-tetrahydro-isoquinoline,
g) 2-[(N-(2-(3,4-dimethoxy-phenyl)-ethyl)-piperid-3-yl)-methyl]-6,7-dimethyl-1-oxo-1,2,3,4-tetrahydro-isoquinoline,
h) 2-[(N-(3-(4-methoxy-phenoxy)-propyl)-piperid-3-yl)-methyl]-6,7-dimethyl-1-oxo-1,2,3,4-tetrahydro-isoquinoline,
i) 3-[(N-(4-(thien-2-yl)-butyl)-piperid-3-yl)-methyl]-7,8-dimethoxy-2-oxo-1,3,4,5-tetrahydro-2H-3-benzazepine,
k) 3-[(N-(2-(benzo[b]fur-2-yl)-ethyl)-piperid-3-yl)-methyl]-7,8-dimethoxy-2-oxo-1,3,4,5-tetrahydro-2H-3-benzazepine,
l) 3-[(N-(2-(benzo[b]thien-3-yl)-ethyl)-piperid-3-yl)-methyl]-7,8-dimethoxy-2-oxo-1,3,4,5-tetrahydro-2H-3-benzazepine,
m) 2-[(N-(3-(6-methoxy-naphth-2-yl-oxy)-propyl)-pyrrolid-3-yl)-methyl]-6,7-dimethoxy-1-oxo-1,2,3,4-tetrahydro-isoquinoline and
n) 2-[(N-(2-(6-methoxy-naphth-2-yl)-ethyl)-hexahydro-azepin-3-yl)-methyl]-6,7-methylenedioxy-1-oxo-1,2,3,4-tetrahydro-isoquinoline,

the enantiomers, diastereomers and acid addition salts thereof.

**5.** Cyclic amine derivatives of general formula (Ia) according to claim 2
a) 3-[(N-(2-(naphth-2-yl)-ethyl)-piperid-3-yl)-methyl]-7,8-dimethoxy-2-oxo-1,3,4,5-tetrahydro-2H-3-benzazepine,
b) 2-[(N-(3-(4-methoxy-phenoxy)-propyl)-piperid-3-yl)-methyl]-6,7-dimethyl-1-oxo-1,2,3,4-tetrahydro-isoquinoline,
c) 3-[(N-(4-(thien-2-yl)-butyl)-piperid-3-yl)-methyl]-7,8-dimethoxy-2-oxo-1,3,4,5-tetrahydro-2H-3-benzazepine,
d) 2-[(N-(3-(6-methoxy-naphthyl-2-oxy)-propyl)-pyrrolid-3-yl)-methyl]-6,7-dimethoxy-1-oxo-1,2,3,4-tetrahydro-isoquinoline,
e) 2-[(N-(2-(6-methoxy-naphth-2-yl)-ethyl)-hexahydro-azepin-3-yl)-methyl]-6,7-methylenedioxy-1-oxo-1,2,3,4-tetrahydro-isoquinoline,
f) 3-[(N-(2-(4-methoxy-phenyl)-ethyl)-hexahydro-azepin-3-yl)-methyl]-7,8-dimethoxy-2-oxo-1,3,4,5-tetrahydro-2H-3-benzazepine and
g) 3-[(N-(2-(3,4-dimethoxy-phenyl)-ethyl)-hexahydro-azepin-3-yl)-methyl]-7,8-methylenedioxy-2-oxo-1,3,4,5-tetrahydro-2H-3-benzazepine

the enantiomers, diastereomers and acid addition salts thereof.

**6.** 3-[(N-(2-(Naphth-2-yl)-ethyl)-piperid-3-yl)-methyl]-7,8-dimethoxy-2-oxo-1,3,4,5-tetrahydro-2H-3-benzazepine, the enantiomers, diastereomers and acid addition salts thereof.

**7.** Physiologically acceptable acid addition salts of the compounds according to claims 1 to 6 with inorganic or organic acids.

**8.** Pharmaceutical compositions containing a compound of general formula I according to claims 1 to 6 or a physiologically acceptable acid addition salt thereof according to claim 7 together with one or more inert carriers and/or diluents.

**9.** Pharmaceutical compositions according to claim 8 suitable for the treatment of sinus tachycardia and ischaemic heart disease.

**10.** Process for the preparation of a pharmaceutical preparation according to claims 8 and 9, characterised in that a compound according to claims 1 to 6 or a physiologically acceptable acid addition salt thereof

according to claim 7 is incorporated in one or more inert carriers and/or diluents by a non-chemical method.

**11.** Process for the preparation of cyclic amine derivatives according to claims 1 to 7, characterised in that a) a compound of general formula (II)

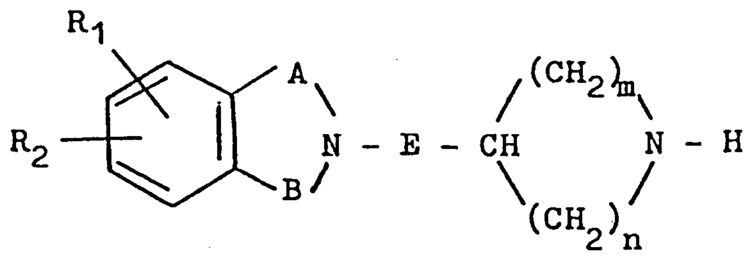

(II)

wherein
$R_1$, $R_2$, A, B, E, m and n are defined as in claims 1 to 6, is reacted with a compound of general formula

$Z_1$ - G - R (III)

wherein
R and G are defined as in claims 1 to 6 and
$Z_1$ represents a nucleophilic leaving group, a hydroxy group or $Z_1$ together with a hydrogen atom of the adjacent methylene group represents an oxygen atom, or
b) a compound of general formula (IV)

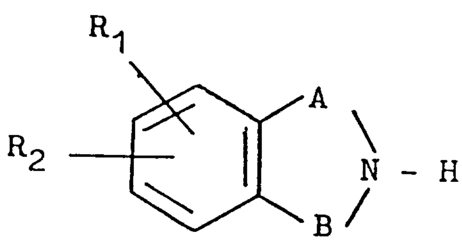

(IV)

wherein
$R_1$, $R_2$, A and B are defined as in claims 1 to 6, is reacted with a compound of general formula (V)

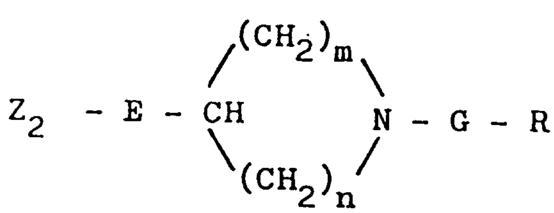

(V)

wherein
R, E, G, m and n are defined as in claims 1 to 6 and
$Z_2$ represents a nucleophilic leaving group, and
subsequently, if desired, any protecting group used during the reactions a) to e) to protect reactive groups such as hydroxy, amino, alkylamino or imino groups, is cleaved and/or
subsequently, if desired, a compound of general formula I thus obtained wherein R contains a nitro group, is converted by reduction into a corresponding amino compound of general formula I and/or
a compound of general formula I thus obtained wherein R contains an amino group is converted by

acylation into a corresponding alkanoylamino compound of general formula I and/or
a compound of general formula I thus obtained which contains at least one chiral centre is resolved into its diastereomers or its enantiomers and/or
a compound of general formula I thus obtained is converted into the acid addition salts thereof, more particularly the physiologically acceptable acid addition salts thereof with inorganic or organic acids.

**12.** Process for preparing cyclic amine derivatives of general formula (I) according to claims 1 to 7 wherein B denotes a $-CH_2-$ or $-CH_2CH_2$ group, characterised in that
c) a compound of general formula (VI)

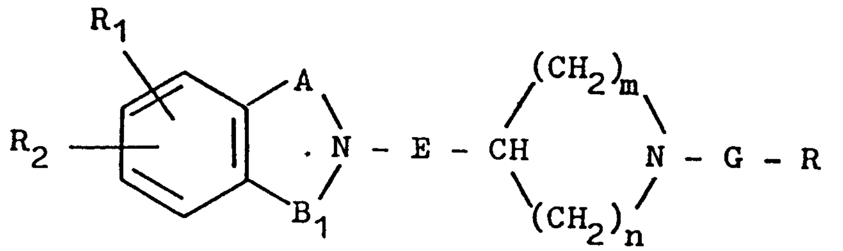

(VI)

wherein
R, $R_1$, $R_2$, A, E, G, m and n are defined as in claims 1 to 6 and
$B_1$ represents a -CO- or $-\overset{x}{C}H_2CO-$ group, whilst the carbon atom marked x is linked to the phenyl nucleus, is reduced, and
subsequently, if desired, a compound of general formula I thus obtained wherein R contains a nitro group, is converted by reduction into a corresponding amino compound of general formula I and/or
a compound of general formula I thus obtained wherein R contains an amino group is converted by acylation into a corresponding alkanoylamino compound of general formula I and/or
a compound of general formula I thus obtained which contains at least one chiral centre is resolved into its diastereomers or its enantiomers and/or
a compound of general formula I thus obtained is converted into the acid addition salts thereof, more particularly the physiologically acceptable acid addition salts thereof with inorganic or organic acids.

**13.** Process for preparing cyclic amine derivatives of general formula I according to claims 1 to 7, wherein A represents a $-CH_2-$ group and B represents a -CO- or $-CH_2CO-$ group, characterised in that a compound of general formula (VII)

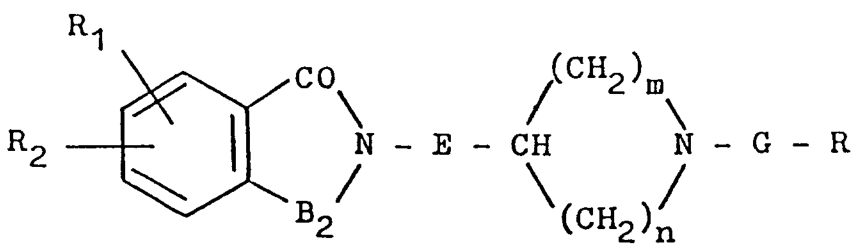

(VII)

wherein
R, $R_1$, $R_2$, E, G, m and n are defined as in claims 1 to 6 and
$B_2$ represents a -CO- or $-\overset{x}{C}H_2CO-$ group, the carbon atom marked x being linked to the phenyl nucleus, is reduced with nascent hydrogen, and
a compound of general formula I thus obtained wherein R contains an amino group is converted by acylation into a corresponding alkanoylamino compound of general formula I and/or
a compound of general formula I thus obtained which contains at least one chiral centre is resolved into its diastereomers or its enantiomers and/or
a compound of general formula I thus obtained is converted into the acid addition salts thereof, more particularly the physiologically acceptable acid addition salts thereof with inorganic or organic acids.

14. Process for preparing new cyclic amine derivatives according to claims 1 to 7, wherein A represents a $-CH_2CH_2-$ group and B represents a methylene or carbonyl group, characterised in that
e) a compound of general formula (VIII)

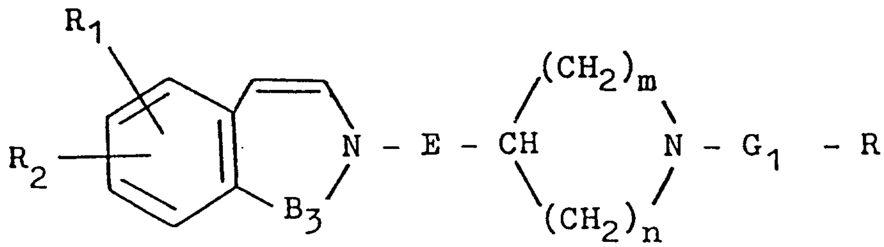

(VIII)

wherein
R, $R_1$, $R_2$, B, E, G, m and n are defined as in claims 1 to 6, and
$B_3$ represents a $-CH_2-$ or -CO- group,
is hydrogenated, and
subsequently, if desired, a compound of general formula I thus obtained wherein R contains an amino group is converted by acylation into a corresponding alkanoylamino compound of general formula I and/or
a compound of general formula I thus obtained which contains at least one chiral centre is resolved into its diastereomers or its enantiomers and/or
a compound of general formula I thus obtained is converted into the acid addition salts thereof, more particularly the physiologically acceptable acid addition salts thereof with inorganic or organic acids.

**Revendications**

1. Dérivés d'amines cycliques de formule générale (1)

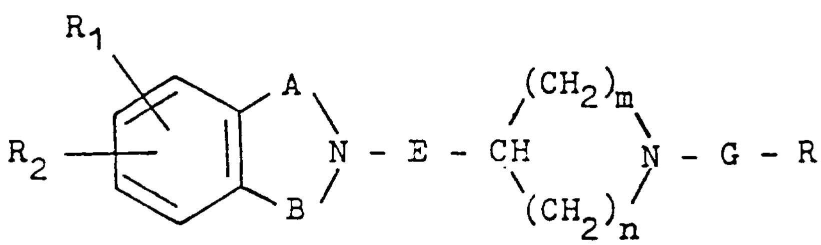

(I)

dans laquelle
A représente un groupe $-CH_2-$, $-CH_2-CH_2-$ ou $-CH=CH-$,
B représente un groupe $-CH_2-$, $-CH_2-CH_2-$, -CO- ou
$-\overset{x}{C}H_2CO-$ dans lequel l'atome de carbone désigné par x est lié au noyau phényle,
E représente un groupe alkylène linéaire de 1 à 3 atomes de carbone,
G représente un groupe alkylène linéaire de 1 à 6 atomes de carbone, un groupe méthylène d'un groupe alkylène linéaire de 3 à 6 atomes de carbone qui est lié à un reste aromatique ou hétéroaromatique R pouvant être remplacé par un atome d'oxygène ou par un groupe méthylimino ou éthylimino,
m représente le nombre 1, 2, 3, 4, 5 ou 6 et
n représente le nombre 0, 1, 2 ou 3, n + m devant cependant représenter le nombre 3, 4, 5 ou 6,
$R_1$ représente un groupe méthyle ou méthoxy,
$R_2$ représente un groupe méthyle ou méthoxy ou bien $R_1$ et $R_2$ représentent ensemble un groupe méthylènedioxy et
R représente un groupe furyle, thiényle, pyridyle, benzo[b]furyle ou benzo[b]thiényle éventuellement substitué par un groupe méthyle, un groupe benzo[b]thiényle substitué par un atome d'halogène ou par un groupe méthoxy ou méthanesulfonyloxy, un groupe indolyle ou N-méthylindolyle éventuellement substitué par un groupe hydroxyle, méthoxy ou benzyloxy, un groupe diméthylthiényle ou diméthoxyi-

soquinolyle, un groupe naphtyle éventuellement mono- ou disubstitué par des groupes méthyle ou méthoxy, les substituants pouvant être identiques ou différents, ou encore, lorsque B représente un groupe $-CH_2-$ ou CO, un groupe phényle éventuellement substitué par un groupe méthylènedioxy, un groupe phényle mono- ou disubstitué par des atomes de chlore ou de brome ou par des groupes méthyle ou méthoxy, les substituants pouvant être identiques ou différents, un groupe phényle substitué par un groupe hydroxyle, benzyloxy, méthanesulfonyloxy, trifluorométhanesulfonyloxy, trifluorométhyle, trifluorométhoxy, nitro, amino, acétamido, méthanesulfonylamino ou bis(méthanesulfonyl)-amino, un groupe triméthoxyphényle, tétraméthylphényle ou dihalogénoaminophényle, leurs énantiomères, leurs diastéréoisomères, leurs N-oxydes et leurs sels d'addition d'acide.

**2.** Dérivés d'amines cycliques de formule générale (Ia)

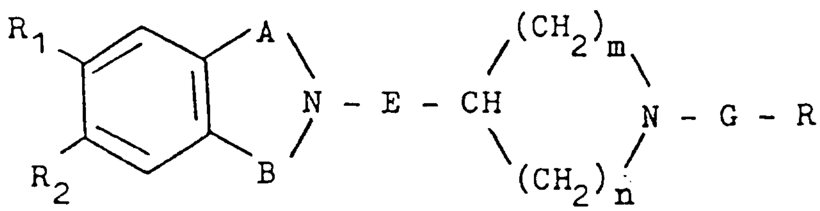

(Ia)

dans laquelle
R, $R_1$, $R_2$, A, B, E, G, m et n sont définis comme dans la revendication 1,
leurs énantiomères, leurs diastéréoisomères, leurs N-oxydes et leurs sels d'addition d'acide.

**3.** Dérivés d'amines cycliques de formule générale (Ia) selon la revendication 2, dans laquelle
A représente un groupe $-CH_2-CH_2-$,
B représente un groupe $-CH_2-CH_2-$, -CO- ou $-\overset{x}{C}H_2CO-$ dans lequel l'atome de carbone désigné par x est lié au noyau phényle,
E représente un groupe méthylène ou éthylène,
G représente un groupe alkylène linéaire de 2 à 4 atomes de carbone, le groupe méthylène d'un groupe alkylène linéaire de 3 ou 4 atomes de carbone qui est lié à un reste aromatique ou hétéroaromatique R pouvant être remplacé par un atome d'oxygène,
$R_1$ représente un groupe méthoxy,
$R_2$ représente un groupe méthoxy ou $R_1$ et $R_2$ représentent ensemble un groupe méthylènedioxy,
m représente le nombre 2, 3 ou 4,
n représente le nombre 1 et
R représente un groupe naphtyl-2, 6-méthoxy-naphtyl-2, 5-méthyl-6-méthoxy-naphtyl-2, thiényl-2, 6-méthyl-pyridyl-2, benzo[b]furyl-2 ou benzo[b]thiényl-3, ou encore, lorsque B représente le groupe -CO-, un groupe 4-méthoxyphényle ou 3,4-diméthoxyphényle,
leurs énantiomères, leurs diastéréoisomères et leurs sels d'addition d'acide.

**4.** Dérivés d'amines cycliques de formule générale (Ia) selon la revendication 2

a) 3-[(N-(2-(naphtyl-2)-éthyl)-pipéridyl-3)-méthyl]-7,8-diméthoxy-2-oxo-1,3,4,5-tétrahydro-2H-3-benzazépine,

b) 3-[(N-(2-(5-méthyl-6-méthoxy-naphtyl-2)-éthyl)-pipéridyl-3)-méthyl]-7,8-diméthoxy-2-oxo-1,3,4,5-tétrahydro-2H-3-benzazépine,

c) 3-[2-(N-(2-(6-méthoxy-naphtyl-2)-éthyl)-pipéridyl-2)-éthyl]-7,8-diméthoxy-2-oxo-1,3,4,5-tétrahydro-2H-3-benzazépine,

d) 2-[(N-(2-(6-méthoxy-naphtyl-2)-éthyl)-hexahydro-azépinyl-3)-méthyl]-6,7-méthylènedioxy-1-oxo-1,2,3,4-tétrahydro-isoquinoléine,

e) 2-[(N-(2-(naphtyl-2)-éthyl)-hexahydro-azépinyl-3)-méthyl]-6,7-méthylènedioxy-1-oxo-1,2,3,4-tétrahydro-isoquinoléine,

f) 2-[(N-(2-(3,4-diméthoxy-phényl)-éthyl)-pipéridyl-3)-méthyl]-6,7-diméthyl-1,2,3,4-tétrahydro-isoquinoléine,

g) 2-[(N-(2-(3,4-diméthoxy-phényl)-éthyl)-pipéridyl-3)-méthyl]-6,7-diméthyl-1-oxo-1,2,3,4-tétrahydro-isoquinoléine,

h) 2-[(N-(3-(4-méthoxy-phénoxy)-propyl)-pipéridyl-3)-méthyl]-6,7-diméthyl-1-oxo-1,2,3,4-tétrahydro-isoquinoléine,
i) 3-[(N-(4-(thiényl-2)-butyl)-pipéridyl-3)-méthyl]-7,8-diméthoxy-2-oxo-1,3,4,5-tétrahydro-2H-3-benzazépine,
k) 3-[(N-(2-(benzo[b]furyl-2)-éthyl)-pipéridyl-3)-méthyl]-7,8-diméthoxy-2-oxo-1,3,4,5-tétrahydro-2H-3-enzazépine,
l) 3-[(N-(2-(benzo[b]thiényl-3)-éthyl)-pipéridyl-3)-méthyl]-7,8-diméthoxy-2-oxo-1,3,4,5-tétrahydro-2H-3-benzazépine,
m) 2-[(N-(3-(6-méthoxy-naphtyl-2-oxy)-propyl)-pyrrolidyl-3)-méthyl]-6,7-diméthoxy-1-oxo-1,2,3,4-tétrahydro-isoquinoléine et
n) 2-[(N-(2-(6-méthoxy-naphtyl-2)-éthyl)-hexahydro-azépinyl-3)-méthyl]-6,7-méthylènedioxy-1-oxo-1,2,3,4-tétrahydro-isoquinoléine,

leurs énantiomères, leurs diastéréoisomères et leurs sels d'addition d'acide.

**5.** Dérivés d'amines cycliques de formule générale (Ia) selon la revendication 2
a) 3-[(N-(2-(naphtyl-2)-éthyl)-pipéridyl-3)-méthyl]-7,8-diméthoxy-2-oxo-1,3,4,5-tétrahydro-2H-3-benzazépine,
b) 2-[(N-(3-(4-méthoxy-phénoxy)-propyl)-pipéridyl-3)-méthyl]-6,7-diméthyl-1-oxo-1,2,3,4-tétrahydro-isoquinoléine,
c) 3-[2-(N-(6-méthoxy-naphtyl-2)-éthyl)-pipéridyl-2)-éthyl]-7,8-diméthoxy-2-oxo-1,3,4,5-tétrahydro-2H-3-benzazépine,
d) 2-[(N-(3-(6-méthoxy-naphtyl-2-oxy)-propyl)-pyrrolidyl-3)-méthyl]-6,7-diméthoxy-1-oxo-1,2,3,4-tétrahydro-isoquinoléine,
e) 2-[(N-(2-(6-méthoxy-naphtyl-2)-éthyl)-hexahydro-azépinyl-3)-méthyl]-6,7-méthylènedioxy-1-oxo-1,2,3,4-tétrahydro-isoquinoléine,
f) 3-[(N-(2-(4-méthoxy-phényl)-éthyl)-hexahydro-azépinyl-3)-méthyl]-7,8-diméthoxy-2-oxo-1,3,4,5-tétrahydro-2H-3-benzazépine et
g) 3-[(N-(2-(3,4-diméthoxy-phényl)-éthyl)-hexahydroazépinyl-3)-méthyl]-7,8-méthylènedioxy-2-oxo-1,3,4,5-tétrahydro-2H-3-benzazépine,

leurs énantiomères, leurs diastéréoisomères et leurs sels d'addition d'acide.

**6.** 3-[(N-(2-(naphtyl-2)-éthyl)-pipéridyl-3)-méthyl]-7,8-diméthoxy-2-oxo-1,3,4,5-tétrahydro-2H-3-benzazépine, ses énantiomères, ses diastéréoisomères et ses sels d'addition d'acide.

**7.** Sels d'addition d'acide physiologiquement acceptables des composés selon les revendications 1 à 6 avec des acides inorganiques ou organiques.

**8.** Médicament contenant un composé de formule générale (I) selon les revendications 1 à 6 ou son sel d'addition d'acide physiologiquement acceptable selon la revendication 7 ainsi qu'un ou plusieurs supports et/ou diluants inertes.

**9.** Médicament selon la revendication 8 destiné à être utilisé pour le traitement des tachycardies sinusales et des maladies cardiaques ischémiques.

**10.** Procédé de préparation d'un médicament selon les revendications 8 et 9, caractérisé en ce que, par voie non chimique, un composé selon les revendications 1 à 6 ou son sel d'addition d'acide physiologiquement acceptablé selon la revendication 7 est incorporé dans un ou plusisurs supports et/ou diluants inertes.

**11.** Procédé de préparation de dérivés d'amines cycliques selon les revendications 1 à 7, caractérisé en ce que

a) un composé de formule générale (II)

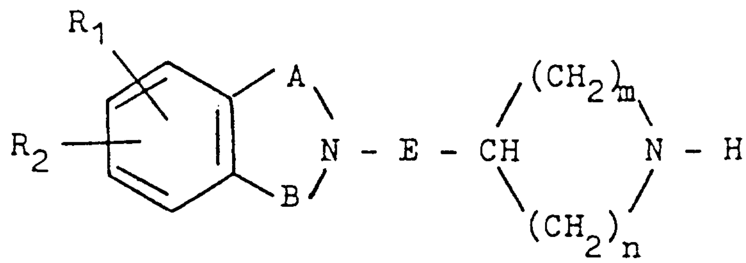

(II)

dans laquelle
$R_1$, $R_2$, A, B, E, m et n sont définis comme dans les revendications 1 à 6, est mis à réagir avec un composé de formule générale (III)

$Z_1$ - G - R (III)

dans laquelle
R et G sont définis comme dans les revendications 1 à 6 et $Z_1$ est un groupe partant nucléophile, un groupe hydroxyle ou $Z_1$ représente avec un atome d'hydrogène du groupe méthylène voisin un atome d'oxygène, ou bien
b) un composé de formule générale (IV)

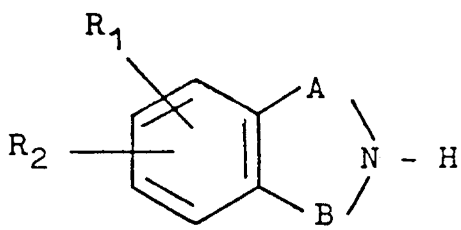

(IV)

dans laquelle
$R_1$, $R_2$, A et B sont définis comme dans les revendications 1 à 6 est mis à réagir avec un composé de formule générale (V)

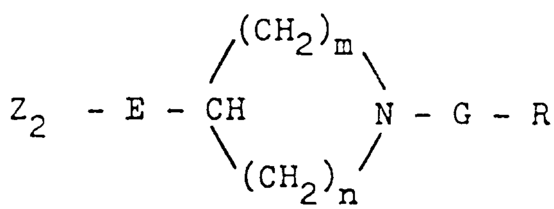

(V)

dans laquelle
R, E, G, m et n sont définis comme dans les revendications 1 à 6 et
$Z_2$ représente un groupe partant nucléophile, puis,
si nécessaire, un reste protecteur utilisé pendant les réactions pour la protection de groupes réactifs tels que des groupes hydroxyle, amino, alkylamino ou imino est clivé et/ou
si on le souhaite, un composé ainsi obtenu de formule générale (I) dans laquelle R contient un groupe nitro, est converti par réduction en un composé amino correspondant de formule générale (I) et/ou
un composé ainsi obtenu de formule générale (I) dans laquelle R contient un groupe amino, est converti par acylation en un composé alcanoylamino de formule générale (I) correspondant et/ou

un composé de formule générale (I) ainsi obtenu, qui contient au moins un centre chiral, est dédoublé en ses diastéréoisomères ou en ses énantiomères et/ou
un composé de formule générale (I) ainsi obtenu est converti en ses sels d'addition d'acide, en particulier en ses sels d'addition d'acide physiologiquement acceptables avec des acides inorganiques ou organiques.

**12.** Procédé de préparation de dérivés d'amines cycliques de formule générale (I) selon les revendications 1 à 7, dans lesquels B représente un groupe $-CH_2-$ ou $-CH_2CH_2-$, caractérisé en ce que
c) un composé de formule générale (VI)

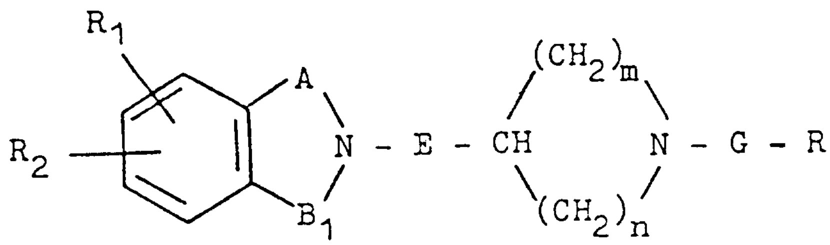

(VI)

dans laquelle
R, $R_1$, $R_2$, A, E, G, m et n sont définis comme dans les revendications 1 à 6, et
$B_1$ représente un groupe -CO- ou $-\overset{x}{C}H_2CO-$ dans lequel l'atome de carbone désigné par x est lié au noyau phényle, est réduit puis,
si on le souhaite, un composé ainsi obtenu, de formule générale (I) dans laquelle R contient un groupe nitro, est converti par réduction en un composé amino de formule générale (I) correspondant et/ou
un composé ainsi obtenu, de formule générale (I) dans laquelle R contient un groupe amino, est converti par acylation en un composé alcanoylamino de formule générale (I) correspondant et/ou
un composé de formule générale (I) ainsi obtenu, qui contient au moins un centre chiral, est dédoublé en ses diastéréoisomères ou en ses énantiomères et/ou
un composé de formule générale (I) ainsi obtenu est converti en ses sels d'addition d'acide, en particulier en ses sels d'addition d'acide physiologiquement acceptables avec des acides inorganiques ou organiques.

**13.** Procédé de préparation de dérivés d'amines cycliques de formule générale (I) selon les revendications 1 à 7, dans lesquels A représente un groupe $-CH_2-$ et B représente un groupe -CO- ou $-CH_2CO-$, caractérisé en ce que
d) un composé de formule générale (VII)

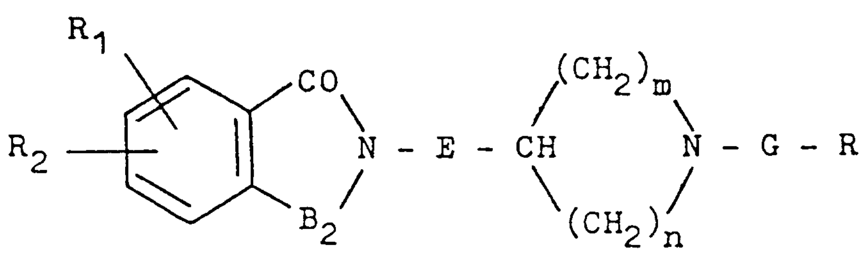

(VII)

dans laquelle
R, $R_1$, $R_2$, E, G, m et n sont définis comme dans les revendications 1 à 6, et
$B_2$ représente un groupe -CO- ou $-\overset{x}{C}H_2CO-$ dans lequel l'atome de carbone désigné par x est lié au noyau phényle, est réduit avec de l'hydrogène naissant puis,
si on le souhaite, un composé ainsi obtenu, de formule générale (I) dans laquelle R contient un groupe amino, est converti par acylation en un composé alcanoylamino de formule générale (I) correspondant et/ou

un composé de formule générale (I) ainsi obtenu, qui contient au moins un centre chiral, est dédoublé en ses diastéréoisomères ou en ses énantiomères et/ou
un composé de formule générale (I) ainsi obtenu est converti en ses sels d'addition d'acide, en particulier en ses sels d'addition d'acide physiologiquement acceptables avec des acides inorganiques ou organiques.

**14.** Procédé de préparation de nouveaux dérivés d'amines cycliques selon les revendications 1 à 7, dans lesquels A représente le groupe $-CH_2-CH_2-$ et B représente le groupe méthylène ou carbonyle, caractérisé en ce que
e) un composé de formule générale (VIII)

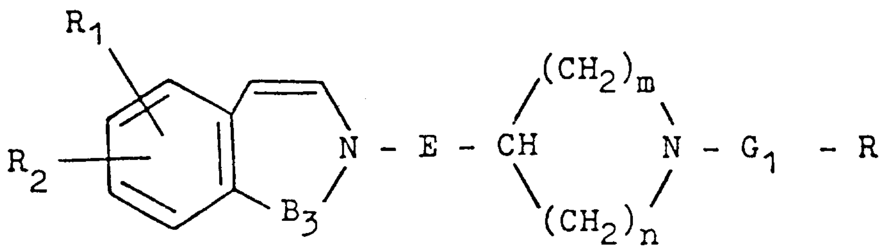

(VIII)

dans laquelle
R, $R_1$, $R_2$, B, E G, m et n sont définis comme dans les revendications 1 à 6, et
$B_3$ représente un groupe $-CH_2-$ ou -CO-, est hydrogéné puis, si on le souhaite, un composé ainsi obtenu, de formule générale (I) dans laquelle R contient un groupe amino, est converti par acylation en un composé alcanoylamino de formule générale (I) correspondant et/ou
un composé de formule générale (I) ainsi obtenu, qui contient au moins un centre chiral, est dédoublé en ses diastéréoisomères ou en ses énantiomères et/ou
un composé de formule générale (I) ainsi obtenu est converti en ses sels d'addition d'acide, en particulier en ses sels d'addition d'acide physiologiquement acceptables avec des acides inorganiques ou organiques.